# EUROPEAN PATENT SPECIFICATION

(11) **EP 2 132 343 B1**
(45) Date of publication and mention of the grant of the patent: **29.08.2012**
(21) Application number: 08717304.3
(22) Date of filing: 29.02.2008
(51) Int. Cl.: C12Q 1/68

(54) **METHOD FOR THE DETERMINATION AND THE CLASSIFICATION OF RHEUMATIC CONDITIONS**
VERFAHREN ZUR BESTIMMUNG UND KLASSIFIZIERUNG RHEUMATISCHER LEIDEN
PROCÉDÉ POUR LA DÉTERMINATION ET LA CLASSIFICATION DE CONDITIONS RHUMATISMALES

(30) Priority: 01.03.2007 EP 07447014
(43) Date of publication of application: 16.12.2009
(73) Proprietor: Université Catholique de Louvain, 1348 Louvain-la-Neuve (BE); Cliniques Universitaires Saint-Luc, 1200 Bruxelles (BE)
(72) Inventor: LAUWERYS, Bernard, B-3080 Tervuren (BE); VAN DEN EYNDE, Benoit, B-1332 Genval (BE); HOUSSIAU, Frédéric, B-1950 Kraainem (BE); GUTIERREZ-ROELENS, Ilse, B-1473 Glabais (BE)
(74) Representative: Vanhalst, Koen
(86) International application number: PCT/EP2008/052532
(87) International publication number: WO 2008/104608

(56) References cited:
- WO-A-2004/035827
- WO-A-2006/020899
- WO-A-2007/035651
- WO-A1-03/072827
- DE-A1- 10 328 033
- US-A1- 2003 148 298
- DEVAUCHELLE V ET AL: "DNA microarray allows molecular profiling of rheumatoid arthritis and identification of pathophysiological targets" GENES AND IMMUNITY, vol. 5, no. 8, December 2004 (2004-12), pages 597-608, XP008063572 ISSN: 1466-4879
- AKILESH SHREERAM ET AL: "Customized molecular phenotyping by quantitative gene expression and pattern recognition analysis." GENOME RESEARCH, vol. 13, no. 7, July 2003 (2003-07), pages 1719-1727, XP001167928 ISSN: 1088-9051
- DATABASE AFFYMETRIX [Online] 2003, XP002449668 retrieved from HTTP://WWW.AFFYMETRIX.COM/SUPPORT/TECHNICA L/DATASHEETS/HUMAN_DATASHEET.PDF

## Description

### Field of the invention

The present invention relates to methods for determining and differentiating between several rheumatic conditions.

### Background of the invention

Arthritis is a symptom of many rheumatic conditions including rheumatoid arthritis (RA), osteoarthritis (OA), seronegative arthritis (SA), infectious arthritis (INF), microcrystalline arthritis (MIC), systemic lupus erythematosus (SLE) and other systemic disorders. In many cases, the diagnosis can be established based on the clinical presentation and additional laboratory or radiological tests. However, in some cases, in particular in early disease, it can be very hard to discriminate between these disorders and the correct identification can only be made after several weeks or months of evolution.

Expression profiling has already shown its usefulness in identifying genes in specific cell types under defined conditions and in establishing characteristic patterns of gene expression in a variety of diseases. WO 2004/110244 describes a method for detecting a predisposition to developing established rheumatoid arthritis (RA) in a subject by obtaining a biological sample from the subject, determining expression levels of at least two genes in the biological sample, and comparing the expression level of each gene with a standard, wherein the comparing detects a predisposition to developing established RA in the subject. WO 2004/035827 describes libraries of polynucleotide sequences and polynucleotide array useful for prognosticating or diagnosing rheumatoid arthritis or osteoarthritis.

WO2006/020899 discloses a group of 1645 genes together with a method, both of which are useful as markers in methods of identifying rheumatic conditions, differential diagnosis and classification of rheumatic conditions, particularly SLE, RA and Wegener's granulomatosus. WO2006/020899 does identify thereby similar marker genes as in the underlying application namely IFI44L, CD79A, GRB2-related, AA789123, GPR43, SRPRB or IFI35 for example.

However, these observations are not useful in daily medical practice, when the differential diagnosis needs to be made not only between RA versus normal but between a larger spectrum of differential diagnosis. Yet, it is very important to provide patients with early identification and classification between different rheumatic conditions and thereby to provide adequate therapy in order to achieve early remission and avoid long-term damage.

### Summary of the invention

The present inventors have designed an arthritis discrimination test, which allows early identification of several rheumatic conditions.

The present inventors have identified a series of genes useful in screening for and differentiating several arthritis diseases. The expression profiles of these genes can be used in identifying whether the individual has a rheumatic condition selected from, but not limited to systemic lupus erythematosus (SLE), osteoarthritis (OA), rheumatoid arthritis (RA), seronegative arthritis (SA) or microcrystalline arthritis (MIC).

The present invention therefore concern a method for the determination and the classification of rheumatic conditions in at least one biological sample of a subject afflicted with said rheumatic condition, comprising the steps of
- determining in said biological sample the level of expression of at least 20 genes, preferably at least 100 genes or fragments provided with Table III or also disclosed as selected from the group comprising CGI-12; PSMC2; AW971248; Al824171; C9orf10; ID4; CCND2; LOC129607; AL133577; ZNF432; AMOTL1; H72914; EWSR1; LHFPL2; RABGEF1; PHYH; HOXA9; FLJ10357; BQ028191; AHI1; MGC3222; GTF2A1; LOC134218; MAP4; HDLBP; IFIT1; HSXIAPAF1; N53479; GYG; HLA-E; AA705933; NOL7; N34842; AGPS; DENR; GSTA4; EFA6R; ARL1; EIF5A; Sep-10; MAK10; SON; BCLAF1; FN1; DKFZp586M; 1819; ANAPC5; DKFZP434C212; TNC; CTMP; CBFA2T1; CMAH; AI816849; APP; ACTR2; C12orf14; EMP2; LOC116064; KIF3B; AA701676; BE674118; FLJ20637; ATPIF1; AKR1B1; EVI2A; MGC40053; UBE4B; C21orf97; FLJ10597; ANGPTL2; TSAP6; XG; FKBP9; TM4SF10; PTPRM; DLC1; GBP1; SDCCAG1; MARK1; AGL; RPN1; IQGAP1; SOX4; CHD1; PRPF3; LOC339047; TIA1; ANK3; AW265065; LOC221091; METAP1; SNX10; BCAP29; SLC26A2; ENSA; EBF; OSBPL9; COL14A1; RP42; TYMS; BG284827; MAN1A2; GNA13; MLL; SPEC1; TTC3; W61005; PPP3CB; SAT2; CD2; SMILE; HSXIAPAF1; PSCD3; DCN; SMS; C9orf3; MYO6; TNFSF13B; RAB45; IBRDC2; ZIC1; FTO; MX1; EVI1; GPR125; BC014318; MAP3K2; WDR7; PRO1853; RECK; LOC286044; NCKAP1; NPIP; AI767751; SET; BRP44L; APOBEC3G; LEPROTL1; RTN3; FLJ30656; NOL7; ZNF133; DDHD2; ALS2CR4; MGC45871; EXTL2; SCRG1; USP36; AI972451; C21orf66; THOC1; RPL10; FLJ23018; KRT10; GOLGA3; CALD1; MAP4K5; ITGB5; AK000106; C2orf8; RNF159; C7orf11; FOXP1; DYRK2; HS2ST1; KIAA0157; MEIS2; SATB1; ICAM3; FLJ20202; SUCLA2; C1QDC1; PNPO; SET; TCF4; MRPL21; FLJ11220; DSC96 mRNA; G1P2; LRIG2; UHRF2; RASSF5; NCOA3; KIAA2010; MST4; TAL1; SLC38A1; AI801777; CYBA; NEUGRIN; SRI; GLG1; PTPRC; AI632214; RBBP4; FLJ13386; POLI; PTPNS1; TAF9L; HMGN1; PTPRD; DPP8; FLJ10546; X07868; KIAA1374; UPF3A; LUC7A; FLJ12178; LRBA; GABARAPL1; ING5; DMTF1; HNLF; AV734646; RPL3; VAPB; TCF7; SPAG9; HBS1L; BTBD7; ANAPC5; PPM1F; PEX1; AK024979; EFEMP2; RAPH1; KIAA0934; SSRP1; TNFSF13B; FLJ46365; AK026716; SSBP2; SEC24D; AI819043; FZD8; ATP5C1; ZFP106; ITCH; NCBP1; ANXA5; DNCLI2; SCG2; GFPT2; KIAA0121; HLA-C; VPS24; DNMBP; HLA-C; SIPA1L2; FLJ22028; COX7C; IMMT; RPL14; C20orf161; ZNF7; AA806989; CHSY1; BUB3; WWP1; RPL5; ITGB5; SLC25A13; IFIT4; M6PR; HGRG8; PDK1; MGEA5; IFNGR1; AI188518; PTK9; AI393725; ADARB1; JTB; AW173166; AL079909; KIAA0563; PCMT1; CLK4; DKFZp564C2063; NDN; ARHGAP21; PURB; USP8; AU143940; OA48-18; H49382; IKBKAP; ZNF507; AI690169; DHRS6; RECQL; N90870; MGC45594; LAP1B; NDUFC1; PSMB9; PRO0659; EIF4EBP2; AW014345; MGC40214; W88821; DustyPK; SF3B1; STAF65(gamma); SF3B1; BE645480; MID1; CXCR4; GSS; KIAA0460; SLC25A6; SASH1; SP110; KIAA1554; AW268884; NPHP3; PABPN1; GBA; MGC5370; SCARA3; PRKAR1A; SUMO2; PTMA; Ufc1; C10orf86; ZNF410; DUSP14; FLJ11273; AW590862; LOC90624; PLEKHA1; RAE1; CSNK1A1; POLS; SIAT1; SGKL; CRIM1; KIF13B; RPL7; PAFAH1B2; ARMCX1; FLJ20152; BE617483; RHOBTB3; BG251521; FLJ11220; IGSF4; NRIP1; CALU; OAS2; BG170478; RAP2A; LOC127262; KIAA1033; FLJ12892; POLR3E; AI921844; LACTB; PTEN; THRAP1; ZNF131; NAP1L5; RAB23; RBM6; HIPK2; BE464819; TNFAIP8; INPP1; ICAM1; C20orf110; SDCCAG1; LDHB; COG5; MGC33424; THRB; NAGA; SRP46; NFIC; NKIRAS2; ANAPC5; FLJ25222; GPATC2; NFIB; AI970797; YWHAZ; GLUD1; SPC18; CDKN1C; EPSTI1; NEK3; C6orf96; GALNT10; SFPQ; STRBP; RPL5; AI887749; SH3MD2; RBBP6; HYPB; SMPDL3A; ARHGEF10; BACE1; AA569225; LOC153682; AV714268; MGC40107; SFPQ; FLJ10287; AI890133; C7orf30; JARID1A; SLC43A3; ZNF36; AI435248; LOC56965; MGC45780; NFIB; COPZ2; NR2F2; D2LIC; HOXA5; ARHGEF12; ZAK; HMGB1; FLJ00133; GCH1; C6orf103; ATBF1; AW296081; SLC36A1; FLJ14281; FOXP1; DPP7; NCOA3; ZF; FLJ10159; GBP1; COX7A1; RNU22; MLLT4; STK4; BPAG1; BF115786; UBAP2; FLJ23018; TAP1; BE326214; C1orf29; CDC42BPA; D1S155E; HEY2; CGI-105; LUC7L; BF591996; AD-017; HNRPA2B1; MGC39350; RAB5B; KIAA0372; CTDSPL; AA195485; RGS5; PNN; LOC253827; HARS; ABCA1; KIAA1040; FLJ13089; LOC340371; BSG; TUBD1; KIAA0663; TNPO1; MGC5306; STEAP2; HTLF; MUS81; MGC12760; LRP6; PCCA; NOP5/NOP58; UGCGL1; MGC10871; ACADSB; DD5; GLUD1; ITK; FLJ14001; CPSF2; NUDT3; TTC3; VPS16; ARHE; FLJ20718; AI885294; KIAA0776; CUTL1; PLEKHA1; EPSTI1; G1P3; SUGT1; FLJ22256 fis; EPAS1; MPP1; LOC58486; PTPRK; LOC150759; AI761578; C10orf56; AI457965; IFI44; STK4; ARHGAP1; BLP2; PDE8A; PTPN13; AI188161; ZBTB4; NRD1; NAP1L4; PDE4C; DAF; HSPG2; CCNI; IGF2R; CNNM3; RPL22; PDE8A; PTP4A1; G3BP; CORO1C; CXCL9; LTBP3; RAB8A; RPL4; FNBP4; RNF110; ST5; CCT6A; BRD8; JMJD2C; RPL4; BF724210; SPIN; IFI27; FLJ12649 ; AI739332; PTPRS; ANKRD10; EFS; EEF1G; SUPT16H; UNC84A; RPL4; PCOLCE; RASGRP1; ASH1L; BF221547; AL524467; SIX1; TI-227H; SFRS6; BPAG1; 7d75g01.x1 NCI_CGAP_Lu24; AA868896; RBM5; DLNB14; ARHGEF10; AW612461; RPL22; RBBP4; PAK2; TNKS1BP1; RBBP6; KIAA1128; DLC1; HNRPL; AIP1; MCM3AP; C9orf65; GRB10; BV20S1 BJ1-5 BC1; PMS2L2; STAT1; PBP; MEG3; FLJ10116; URKL1; IQGAP1; STAT1; SET; SACS; AA156754; ERCC1; PTBP1; PTPRD; UNC13B; TERF1; FLJ20232; PPP2R5E; COPA; HSPC047; FLJ20265; RAB2; PON2; AFG3L1; EEF1G; EIF3S6IP; VDP; NUTF2; SSPN; KBTBD9; LOC150678; LOC124512; ETV6; ZFYVE16; NEDL2; TACC1; PDE8A; LOC375295; BTBD3; LDB2; WBSCR20C; HSPA9B; GSTA4; AI823917; DKFZp547I014; COL12A1; LOC286097; BTBD1; CLIC4; LRP11; TERF2IP; PDE1A; GDAP2; RAB3-GAP150; KIAA0220; FANCL; IFIT4; MAP4K4; SBDS; N25986; YY1; KIAA0746; LOC401494; AW576195; MTUS1; ZCWCC2; 3454421; PAM; LRCH4; TTC3; cig5; TEAD1; SEMA4C; BCLAF1; KIAA0962; C20orf140; AI693516; THRAP3; BOC; KIAA0483; SDAD1; PIK3R1; AL833114; BF674064; LOC144871; SUMO2; PGF; TXNDC5; SAMHD1; SUMF2; HMGB1; FLJ12592; KIS; ZNF264; CCL5; DUT; RPL7; KIAA0367; SFRS14; MKKS; CPEB4; YAP1; PRKR; AI379070; ZNF131; PHIP; GOLGIN-67; JMJD1C; LYSAL1; RBBP9; COL1A2; EVI1; THBS4; KIAA0934; SCML1; AFURS1; REV3L; TMOD1; LY75; RAB45; ARPP-19; LPIN1; LOC152485; AK055769; GAS5; RBPSUH; MGC16169; SF1; PLEKHC1; RUFY2; OXCT1; RPS25; PDLIM7; CSNK1E; TM4SF8; FILIP1; AI708256; CRIM1; MAF; EML1; ZNF462; DDX47; HIMAP4; SYNCOILIN; HMGB2; AI357655; FLJ20232; AW450329; NFAT5; FLJ22649; ARAP3; ATBF1; SET7; COX15; SLC35B2; PCYOX1; T2BP; RBBP6; SPARCL1; AK025416; LAMA4; MESDC1; DLG5; ARL6IP; KIAA0763; SBDS; NBL1; RAD17; SND1; ANK3; C9orf97; PMS1; JAK3; FN1; SFRS14; CAPZA2; ERP70; ZNF505; HUMAUANTIG; NS; TCFL5; SP100; IFRD1; HRIHFB2122; SOCS7; AI307760; LOC285636; YY1; FZD8; IAN4L1; LOC162073; BAG2; ODAG; STOM; TREX2; NRIPL; AU146418; CCND1; EFA6R; C20orf121; PCDH16; SULF2; BF516305; SMARCA1; FLJ11806; AHSA2; BST2; SCARA3; BE621524; SEC14L1; AA480392; NEURL; BF057799; SLC1A1; FLJ20378; FLJ35036; AI733330; RIOK3; RANBP6; STAT1; COL3A1; AW590838; RPL3; AI676241; C12orfi22; AI341053; WBSCR20C; EIF5B; SPEC2; SPG7; PBX1; NUFIP1; ANKH; MGC20781; CRTAC1; RPL7A; PAM; FOXC1; STAT3; AA521504; FLJ21228 fis; SON; TPD52; COL18A1; STAT1; KIAA1450; UACA; TRAM1; RAP2B; LPXN; CALR; KIAA1363; PHLDB1; IL7R; AU144961; ANKH; LOC144571; SOX4; MGC15875; DREV1; LIPT1; FLJ20203; TXNIP; RPL3; ANKMY2; TRIB1; MBD4; TNPO1; KIAA1704; TNFAIP8; MGC9726; UQCRC2; AI242023; HSPCA; SEC61A2; DOCK8; 13CDNA73; FAM20A; TTC3; SAT; MYO10; HSPCA; BF724621; CCNL2; BF954306; AA579047; KIAA1450; FLJ32731; AU146390; APOE; ANKRD25; LOC130355; JDP2; AW449230; ENAH; SYNCRIP; KIF1B; MTMR2; METAP2; DKFZp434K1323; PDE4DIP; KIAA1043; FLJ38973; RUNX3; ATP6V1C1; SYNPO; FBXO21; FLJ20758; SLC18A2; ACIN1; MGC34646; INPP5A; KIAA0582; FLJ33814; AW025579; KBTBD2; APOL3; NBS1; OGFRL1; FLJ13220; UBE2H; LOC284454; KPNA3; CTSD; KLHDC1; LOC283241; LOC286167; IRF2BP2; LOC339047; AF086069; UVRAG; COL27A1 ; AI473796; PRKCL2; AI821780; SLC30A5; MRPS22; BECN1; HEG; TXN2; AQP1; PLXDC1; LAMB1; KIAA0863; PLAGL1; CALM1; CRLF1; FLJ20152; PCDHGA4; AU146924; KIAA1196; CREB5; EMP1; FLJ11379 fis; FAP; C20orf111; GPX1; SUI1; EEF2; FLJ39845 fis; KIAA1078; CRAT; B4GALT1; ATP2B4; CMAS; MAPKAP1; KNS2; RBM9; EIF4B; PEG3; PSMB6; SH120; EIF3S3; SFRS12; BC033548; DICER1; Sep-06; PIR; CRKL; CAST; ZNF148; DDEF2; ZRANB1; HLA-DQA1; MGC14376; ALS2CR3; CEB1; FGD5; PANX1; FOXC1; CNTNAP3; ZNF281; BM041211; AU144887; FLJ13855; KIAA0117; MAT2A; SUMF1; AI096634; FLJ33979; ANKH; FLJ22557; PTX1; RASA2; CNOT7; HEG; RAMP3; ALDH3A2; ETFDH; P4HA1; SNAPC3; IFNGR1; SP110; RHOG; W96225; QP-C; FBXO8; NFIX; AW299905; RSN; DDX1; AL565362; NMI; BTN3A3; MGC3794; MKKS; MGP; FRMD4; ALDH1A1; DDX50; BE467916; ENAH; RNASET2; HSA9761; SP110; GRB2; FLJ32642; HEPH; DPM3; BPAG1; JAK1; ARHGAP17; LYN; C13orf10; SRPK2; AKAP2; PRO0149; AI535737; CKAP1; AIM1; FKBP7; SHPRH; DKFZP566B183; IL15; DUT; RING1; C14orf124; AI801902; FBI4; SH2D3C; NET1; CD59; CGI-30; FLJ14888; CCNI; SLC2A14; KIAA1185; DRCTNNB1A; IF; KIAA0924; MGEA5; KIAA1191; RIPX; ELF1; ITGAE; SIPL; HRH1; FAH; LOC92689; BE620513; PARVA; FCMD; RNPEP; STEAP; BNC2; STATIP1; PLCE1; PAPOLA; MGAT4A; AUTS2; PRKACB; SPTLC2; AU157716; MGC39830; PRDX6; ANKRD17; SMARCC2; ZNF275; BCAP31; SPOCK; BCL2L11; SLC25A12; TIP120A; AL038450; ZNF265; USP48; MGC3794; RNASE3L; CSPG2; AA521438; ZNF42; PDGFRL; FNBP1; SRPK1; CYBB; ACAT1; ARPC5L; LUC7L2; KIAA0907; SYTL2; ZAK; TBX15; TOMM20; LGMN; FAM36A; TRAPPC5; HIPK2; Rif1; LRPPRC; RER1; ZRF1; MEF2C; RPL3; C2orf6; C6orf166; CPNE3; C18orf25; DATF1; BG250721; GAF1; GNG12; FLJ22789; C2orf29; UBE2R2; FBXO9; ATP6V0C; MGC3222; IL17R; PRRG1; C13orf11; EIF4G2; USP16; FLJ12666; GPM6B; ZNF187; FLJ00133; IFI35; TXNIP; ANTXR1; FLJ10539; NDUFA2; xs157 mRNA; ERBP; TUBG2; SERP1; TNFRSF1B; HACE1; AW162210; RAB8B; LOC93380; TLOC1; KRT10; TTC17; ZFYVE20; COL5A2; PLSCR4; SGCD; FLJ30851; AU151222; SH2D1A; LDHB; TNFAIP3; SPEC1; AA079839; RNF159; TXNIP; ZFYVE21; KIAA1554; POLR1B; RDX; KIAA0182; AW167727; T16544; C1orf24; APLP2; APH-1A; FOXP1; THBD; DONSON; PSMC2; CMYA5; UPF3B; CUL2; YWHAH; TCP1; ANKH; FTS; TCF4; RPL13A; NAV1; NCAG1; HSBP1; AL832672; SFRS11; C10orf56; MYO1B; CDADC1; WASF2; AA824321; LENG8; 4821863; FKSG17; STOM; USP32; PRDX1; PC4; UBE1DC1; SBLF; TM6SF1; C20orf108; BAZ1B; FLJ20035; N51102; ZNF521; GBP1; KIAA1712; AA778095; HP1-BP74; HSPA12A; FAM11A; CD164; AI684551; EFCBP1; USP31; NTN4; ARL2; AW024741; SERPINA1; ARPC4; COL1A2; RTN3; TTC3; UBE2D3; KIAA1704; KIAA2024; PRO2730; ROD1; ANGPTL2; NEK1; ENAH; ARHGAP9; ISGF3G; C10orf45; EEF1D; KLF4; PR47; NDFIP1; hIAN7; LOC51185; SLC2A5; chromosome 6 open reading frame 187; CTSS; NOTCH4; FLJ23556; FLJ20850; FUCA1; TTC17; LAP1B; RNPC2; SIP; DustyPK; HAN11; MRPL33; TUFT1; SCOC; PRO2275; MGC21881; BRD9; AA703523; COVA1; LOC152719; GABARAPL1; NT5C3; ZNF558; BCCIP; RAB35; XBP1; PIK3R3; C14orf78; RAC2; AK022838; MGC21881; ITSN1; ZNF258; MGC14151; LANCL1; PCDH9; EIF2S3; FBXO11; KIAA1268; RNF24; TMEM18; CNOT2; RNASE4; DCTD; STK25; FLJ14639; PLVAP; EIF4G1; GABRA4; WDR20; PHF17; DHX40; AZ2; KIAA1361; MASP2; TARBP1; LMOD1; FLJ36754; CREB1; AK026869; CAST; C22orf2; THOC2; TRIP12; PECI; GSPT1; HIP1; PTP4A2; MAP4K4; AMSH-LP; T87730; PWP1; CXXC1; AA704163; AW024656; RNASE1; CD164; ZNF605; ZC3HDC1; AKR7A2; FEM1C; POLR2C; ANKRD12; PDPK1; HRIHFB2122; AI092824; LAP3; SPPL3; KIAA0102; C5orf13; ANKRD6; SSR3; COMMD2; RIPX; DOCK8; RASIPL; PSMD10; PNN; ITGB1BP1; MRPL30; GARNL1; N50119; RQCD1; YY1; TCF7L2; DDX18; C21orf80; HNRPDL; BRD7; COCH; PDGFRB; KIAA0368; SLC2A4RG; BF840360; DRE1; GARNL3; ARPC4; LCP1; ASXL1; TTYH3; AU158570; RNF41; LOC155435; RPS24; TAF1B; KIS; STK4; HAVCR2; CXorf9; ZNFN1A1 ; AI825068; LNX; KIAA0265; NOD27; TGOLN2; PSCDBP; DNAJC3; DGKD; PAK2; NIT1; ADCY6; TIMM50; PCSK5; OAS1; AA988769; PABPN1; CMKOR1; NCF4; BF677084; C1QG; FOXO1A; IL18BP; TNFAIP3; ZFP91; CCR5; DSCR1; C15orf17; APOBEC3G; TCRBV13S1-TCRBJ2S1; LST1; ZNF42; KIAA0792; FLJ22313; BLNK; SLC15A3; C6orf18; ERO1L; HOXD4; HCST; XPR1; AA581439; AI090764; FLJ32954; LST1; SNCAIP; SEMA3A; CPEB4; FLJ12700; KIAA1171; AKAP13; ARHGEF2; SLC25A4; NMT2; LST1; GRB10; pp9099; SELPLG; KLF4; MPHOSPH9; RCP; KIAA2028; PDCD1LG1; KIAA1582; FLJ13373; DBT; FLJ39441; PRKCBP1; DKFZP566J2046; CD37; CACNB3; ZAK; HNRPA1; PGAP1; EPB41L2; ARL11; CXCL16; KLF3; COPG; CD209; LOC55831; 125405 mRNA sequence; SR140; ATAD1; FGD2; DIO2; IMAA; KIAA1416; LOC286440; C1orf16; CDC42BPA; PRKACB; CXCL10; LTB; SMARCC2; ANK3; CENTA1; ZNFN1A1; DOCK6; MAP1D; FLJ35681; RABL3; LILRB4; GATA3; PRKAA1; AL049337; SLC16A6; PRPF18; CTSZ; AI638151; BF511763; IFI44; SLA; OAS3; CARD4; IRF1; SAMHD1; EOMES; DKFZp434G0522; CCR2; P2RY8; LST1; FUT8; CTSS; EAF2; MLF1; MGEA6; B3GNT1; NACA; LOC91316; ZNF37A; FLJ11236; AK098337; ABCC6; C2; FLJ20668; 5301781 ; ARHGAP26; ZNF614; FLJ11577; CD14; KIAA0507; LST1; LAMA2; FYB; DVL1; ARHGEF12; CCL5; KPNA4; CECR1; AI640483; RUNX3; CSPG6; SYK; LAX; SNARK; AL137616; MGC50844; SLC35B4; KIAA0090; IL2RB; DAPP1; CEP1; CCL5; SFRS14; BC033250; TXNRD3; BG400570; UBE2C; AV710542; PDE7B; CRTAM; DC-TM4F2; AI703142; F11066; ATP8B2; ADAMDEC1; CCDC3; AMT; GM2A; UCP2; FLJ12178; UPLC1; GZMA; W73730; MYL6; GGA2; PCSK7; EIF2C2; STAT1; ZDHHC5; W80359; C9orf10OS; ADAM28; QKI; RNF159; NFIC; IL7R; FLJ12687; BNIP1; HIPK1; M12959; Human T-cell receptor rearranged beta-chain V-region; LOC157567; MGC21518; C2orf18; AI401105; SLC16A6; AF009316; AA029888; AIM2; RASA2; WDR10; T57946; TPD52; MET; ZNF608; C6orf59; TMG4; CXCR4; SLAMF8; N73742; MGC2803; DZIP1; IL2RG; FPRL2; SLAMF1; ZNF581; SLC35D1; LOC149705; SEC10L1; BE883167; AK027226; AGT; AW903934; AA365670; IGKC; AI983904; IL12RB1; CDKN1C; DBC-1; RHOF; C14orf106; AL833255; BCL11B; TUB; RAPGEF2; PHLDB2; FLJ10520; AA625683; AL037998; PPP4C; AU145365; KIAA0746; CD3D; KIAA1333; SDK1; KIAA0802; ZNF515; RP26; DOCK6; CXCR4; T90703; SYTL4; PRKCL2; F5; COL16A1; TFCP2L2; AW029203; NOD3; CCL8; KIAA1644; BF512500; CLECSF14; AL832806; PRKCB1; BF028225; RA116; AW972881; AA732944; IGKC; CASP10; DKFZP4341216; SERF1A; SEL1L; C6orf119; LOC112476; SOX5; AI589594; AW440490; RALGPS2; IL15; PLAC8; GM2A; ANK2; HSPC065; DKFZp564C142; PPHLN1; AI027296; MPPE1; ZNF607; PRF1; MBNL2; BCL11A; AW190479; BF196060; BF942260; AU146285; RAI3; 4853814; WBSCR20C; ITGAL; PIGL; DBP; TLE2; FLJ39155; GPR4; IGKC; SPINT2; SLAMF6; KCNAB1; IRF4; LOC113730; BAIAP1; GZMK; IFRG28; ANKH; POU2AF1; CCL4; IL17D; GPR18; CD48; ZAP70; ODF2; TGOLN2; AA765841; DEADC1; AI373107; LCK; IGLJ3; PCBP4; IL27RA; NICE-4; ARFD1; FLJ25955; MGC8974; SMAD5; H53689; LRRC1; CA309468; BCL11B; PREP; GNAS; IGKC; FBI4; PDE4D; NME3; IGHG1; DKFZp761A078; UBD; DSPG3; IGL@; SLC15A2; PEG10; PTP4A3; FLJ39885; FLJ10997; GZMB; BE676335; CPSF1; AW504569; C20orf140; KIAA0870; AK024712; SAMD3; BG527339; IGHG1; TNFRSF7; ZNF251; CD38; R39769; SLAMF7; TOMM22; DHX9; ICAM1; FLJ21395 fis; LOC220929; IGHG1; CD3E; TAPBP; DAPP1; TNFSF8; FLJ37562; IGHM; BU683708; BAIAP2; AW205969; IGLJ3; AK000795; PLCB4; STK10; HPSE; AI805006; PRG1; KIAA0676; BHC80; DOK4; OAS2; AI923633; SMOC1; OAZIN; AL137307; AA707125; W93695; SLC8A1; NELL2; TRGV9; AI225238; FLJ13089; IGL@; IGHG1; TOX; NIN; LOC197336; IGHM; MEF2C; SLAMF8; ELOVL4; IGLJ3; BOMB; immunoglobulin lambda joining 3; PBXIP1; B2M; KIAA1458; BAG4; BG482805; KIAA0563; CTNNB1; SFRS15; FLJ13381; GAS7; KBTBD4; H963; STX6; LAIR2; NSMAF; TULP3; AUTS2; FLJ20202; IL4I1; AK026494; MDA5; CKLFSF4; FLJ10300; IRTA2; TRGV9; AA700633; IGHG1; FLJ32949; TRIM; LOC147004; BITE; MGC20410; AW976347; P2-32 anti-oxidized LDL immunoglobulin light chain Fab; TBX2; GALC; TNFRSF17; IGHG1; IGLJ3; IGHG1; FBI4; AI140189; AI042187; ARF6; GBP5; CENTB2; IBRDC3; LAMP3; TOSO; PPP1R3E; C18orf18; LOC145786; BC033124; PTPN7; NRP2; PACAP; AI798822; SIPA1L3; CXCL13; XLHSRF-1; ISG20; TRGV9; FLJ39873; GBP5; FLJ22781 fis; AA809449; SLC2A6; PACAP; EMILIN2; HLA-DOB; immunoglobulin lambda light chain variable and constant region; RASSF1; AI821404; KIAA1332; CXCL11; LOC283849; FLJ21069; KIAA0746; C6orf190; CST7; AA854843; ARHGAP1; FLJ43842; immunoglobulin heavy chain variable region; LAMA1; OSBPL6; PTPRC; CWF19L1; BQ006233; IGHV; KIAA0420; FLJ30046; IRTA2; OASL; IGHG1; OR2I6; MGC40042; AMN; SRPR; NKG7; P2RY10; BC010121; SLAMF7; IL24; PIM2; T81422; CLECSF12; LIG3; IGHG1; MTAC2D1; XP6; RIPX; FKBP7; CD8A; BG491393; X72475; cig5; CHTF18; CD79A; CXCL11; IGHG1; X84340; PKN3; ISG20; CD6; SLAMF7; NK4; IPO9; KIAA1811; MSI2; SLC1A4; CD3Z; GZMH; IL21R; EGFL5; KIF5A; PTPRCAP; MYST4; LOC200772; D87024; IGKV1D-8; AA706701; CUL5; MGC43690; IGHG1; MGC40042; PCDHGA11; DTNB; FNDC4; GOLGA2; BCL11A; HT036; AE000659; AA789123; ATF5; AV729651; FLJ20406; AU147442; LILRA2; TRD@; PB1; AK021989; G3BP; AK025231; PLK3; SLC24A6; GM2A; TCL1A; IGHG1; AI022173; ZBP1; XCL1; MYO5B; UBE2H; BG231773; AI742685; SYNE1; FBXO26; KIAA0484; CYLD; KIS; FLJ14107; HLA-DOA; ZAP70; AW194655; ALCAM; CTLA4; PNOC; AA572675; GNLY; INDO; ERN1; MGC2655; CHST3; MYO7A; IL21R; U2AF2; RUNX2; PLA2G2D; PKHD1L1; AA149736; HRMT1L1; BE503823; BIRC3; GPR43; TGFBRAP1; C14orf131; AJ388663; STRN3; CYP3A5; PRO2605; PVR; LOC146206; ADCY2; ARNT2; NAP1L; GRID1; AK024204; AA912540; TP53I11; GNB4; C7orf10; DNAJC12; CWF19L1; RNASET2; C20orf103; or KIAA1238; and
- identifying whether the level of expression of said at least 100 genes in said sample correlates with the presence of a rheumatic condition.

The present inventors have specifically selected 2059 genes listed above found by ANOVA to be differentially expressed between the five conditions SLE, OA, RA, SA, or MIC.

In a particular embodiment, the identification step comprises the comparison of the level of expression of said at least 100 genes with the level of expression of said genes in a reference sample of the same type obtained from subject afflicted with determined rheumatic conditions, according to clustering analysis.

In particular, the present invention is performed using a synovial sample.

In an embodiment, said method comprises determining in said synovial sample the expression level of at least 100 up to 2059 genes or fragments thereof selected from the group of genes or fragments thereof listed above.

In an embodiment, said at least 100 genes or fragments thereof selected from the 2059 genes listed above, are the following genes which were found to be sufficient to define a specific signature in every disease : AI923633; ARPC4; ASH1L; AW008502; AW296081; AW612461; BAG2; BCL11B; BE676335; BG231773; BSG; BTBD1; C12orf30/FLJ13089; C14orf131; CALU; CCL5; CCND1; CD79A; CDC42BPA; CDC42SE1/SPEC1; CHD1; CHD9/FLJ12178; CPSF1; CST7; CTDSPL; DUT; EIF3EIP/EIF3S6IP; ETV6; EXTL2; FLJ21395 fis; FN1; FRMD4A; G3BP1; GPR4; GRID1; GYG1; HMGB1; IFI27; IFI6/G1P3; IFIT3/IFIT4; IL7R; JAK3; JOSD3/MGC5306; KIAA0090; KIAA1128; KIAA1377; KIAA1450; LCK; LOC129607; MYEOV2/LOC150678; NBL1; NELL2; OAS1; PARP12/ZC3HDC1; PDE5A; PGF; PHF21A/BHC80; PIK3C2A; PTBP1; PTEN; PTPN7; QKI; RAB8A; RALGPS2; RAP2A; RAPGEF2; RASGRP1; RBBP6; RGS5; RPL4; RSAD2/cig5; SFRS2B/SRP46; SFRS6; SIPA1L3; SLC15A2; SPARCL1; SUPT16H; SYNCOILIN; TARP III TRGC2 III TRGV9; TBC1D20/C20orf140; TBC1D24/KIAA1171; THRAP3; TI-227H III TUG1; TLE2; TMEM43/MGC3222; TNFSF8; TOX; TRBC1 III TRBV19; TSPAN3/TM4SF8; UCKL1/URKL1; WDR90/LOC197336; ZFHX3/ATBF1; T87730.

In an embodiment, said method comprises determining in said synovial sample the expression level of at least 264 genes or fragments thereof selected from the group comprising PLAC8, IRF4, SAMD3, HLA-DOB, EOMES, PDK1, BG548679, AK25048, AU146285, P2RY8, AI225238, JAK3, LAX, PTPN7, RP26, TRIM, SLAMF1, PTPRCAP, LCK, PTP4A3, AI825068, BCL11B, CD79A, IL7R, GPR18, STRBP, C20orf103, AA732944, ZAP70, SLC38A1, RUNX3, TOSO, BCL11A, NELL2, ICAM3, LTB, TCF7, TRD@; ANK3, CREB5, FAH, FKBP7, AF009316, KLF4, ANKH, NTN4, THBS4, SCARA3, CYP3A5, MGP, AMN, CMAH, FN1, GABRA4, ABCC6, BE503823, SLC24A6; CXCL11, LOC113730, cig5, STAT1, GM2A, G1P3, IFIT4, EIF5A, EPSTI1, MX1, IFI44L, IFI27, LOC129607, G1P2, IFIT1, FLJ39885, OAS2, FLJ20637, OAS1, MDA5, OAS3, LILRA2, TGFBRAP1, BST2, OASL, CEB1, HLA-DOA, KIS, GNB4, CLECSF12, AW262311, CALR, FPRL2, MAP3K2, FLJ20668, CYBB, SNX10, GRB2, GPR43, FLJ20035, C1QG, ARF6, IFI35, FLJ21069, BE674143; ZNF607, X07868, AU157716, CCDC3, CPSF1, AW029203, AK022838, CCNL2, C7orf10, SEC24D, AFG3L1, TLE2, PCSK5, AA706701, C18orf18, OSBPL6, BC042472, AUTS2, SOX4, PTPRD, AA572675, COL18A1, COL16A1, GPM6B, SCG2, TNC, TP53I11, COL12A1, AL832806, AA912540, MYST4, STEAP, ARNT2, AA854843, KIAA0484, AU147442, PKN3, SYNE1, DSPG3, AW903934, FBXO26, LOC200772, AF116709; SCRG1, DNAJC12, FNDC4, AK024204, NBL1, BF591996, DIO2, SGCD, T90703, SPOCK, CKLFSF4, AW162210, CDC42BPA, KIAA1171, FKSG17, N73742, ZNF515, PTPRS, CTDSPL, NRP2, EXTL2, CCND1, CALU, PVR, ATBF1, AFURS1, SYNPO, MYO7A, KIAA1450, SLC35B4; BCL2L11, MBNL2, TXNIP, RNASET2, DHX9, RUNX2; MSI2, FZD8, AW265065, DBC-1, ANGPTL2, FBI4, C14orf131, BF057799, SRPR, TTC3, COPA, PCDHGA11; CXCL13, AI823917, AA789123, CD209, IAN4L1, NOD3, KIAA1268, HRMT1L1, AI821404, COPG, FLJ33814, H963, TAP1, CUL5, AW504569, AA809449, CCL8, ZC3HDC1, SYNCOILIN, KIAA0090, GGA2, NAP1L, ETV6, AL037998, AK024712, AW612461, BM873997, AK000795, FLJ00133, FOXC1, MGC43690, BF590303, AI090764, BF221547, ID4, AW296081, PGF, RGS5, W80359, AF086069, FLJ32949, RAMP3, T87730, AI742685, GPR4, GRID1, FAM20A, FRMD4, FLJ13089, TBX2, RAPGEF2, BM353142; SLC1A4, LIG3, EMILIN2, ALDH1A1, TNFSF8, PB1, AA365670, YWHAZ, ZNF581, BAG4, CWF19L1, SLC15A2, PTPRC, STRN3, TCL1A, PKHD1L1, and CTLA4.

These 264 genes or fragment thereof are selected from the 2059 genes listed herein above and define a specific gene signature in each disease.

Furthermore provided is a method for the determination and the classification of a rheumatic condition in at least one synovial sample of a subject afflicted with said rheumatic condition, comprising the steps of
- determining in said synovial sample the level of expression of at least 20 genes or fragments thereof selected from the group comprising FLJ21395 fis, TTC3, NELL2, PTPN7, HLA-DOA, GPR171, COPA, KIAA0484, TRAT1, FNDC4, BCL11B, C14orf131, FKBP7, TBC1D24, AL037998, AI225238, LOC113730, AA789123, KIAA1377, AW504569; and
- identifying whether the level of expression of said at least 20 genes in said sample correlates with the presence of a rheumatic condition, wherein the identification step comprises the comparison of the level of expression of said at least 20 genes with the level of expression of said genes in a reference sample of the same type obtained from subject afflicted with a determined rheumatic condition, according to clustering analysis.

Said 20 genes or fragments thereof are selected from the 2059 genes listed above and were found to be sufficient to define a specific gene signature in every disease.

In a particular embodiment, said synovial sample is a synovial tissue. In another particular embodiment, said synovial sample is a synovial fluid. In yet another embodiment said method is performed on cells from the synovial fluid.

The present inventors have found that gene expression profiling in peripheral blood mononuclear cell (PBMC) is not adequate in order to make a correct diagnosis of joint disorders. Sensitivity and specificity of the gene expression profiles are too low. Therefore, all the previous studies about gene profiling in PBMC are not suitable for development of a diagnostic tool. By contrast, the present invention is based on the study of gene expression profiles in synovial tissue of patients with rheumatic conditions. Preferably, the present invention provides a method for the determination and the classification of a rheumatic condition in at least one synovial sample of a subject afflicted with said rheumatic condition, comprising the steps of
- determining in said synovial sample the level of expression of at least 100 genes or fragments thereof selected from the group listed above; and
- identifying whether the level of expression of said at least 100 genes in said sample correlates with the presence of a rheumatic condition, wherein the identification step comprises the comparison of the level of expression of said at least 100 genes with the level of expression of said genes in a reference sample of the same type obtained from subject afflicted with a determined rheumatic condition, according to clustering analysis. The point of the present invention is that rheumatologists are not confronted to a differential diagnosis between only two conditions but to a differential diagnosis between several inflammatory conditions (in particular, RA, SLE, OA, MIC and SA). The present invention addresses the simultaneous differential diagnosis of all these conditions.

In a particular embodiment, provided is a method for the determination and the classification of a rheumatic condition in at least one synovial sample of a subject afflicted with said rheumatic condition, comprising the steps of
- determining in said synovial sample the level of expression of groups of genes (at least 20, preferably at least 100, at least 264 up to 2059 genes or fragments thereof) that are selected from the groups listed above and define a distinctive signature for each disease; and
- identifying whether the level of expression of said genes (at least 20, preferably at least 100 , at least 264 genes up to 2059) in said sample correlates with the presence of a rheumatic condition, wherein the identification step comprises the comparison of the level of expression of said genes (at least 20, preferably at least 100 , at least 264 genes up to 2059) with the level of expression of said genes in a reference sample of the same type obtained from subject afflicted with a determined rheumatic condition, according to clustering analysis.

In an embodiment, said identification step comprises a step of using a supervised hierarchical clustering algorithm to evaluate whether the general profile of expression of these at least 20 genes, preferably at least 100 genes, preferably at least 264 genes, yet more preferably up to 2059 related to each other, fits into one diagnostic category.

The method of the present invention is not based on the comparison level of one gene as compared to standard values; it is based on the pattern of expression of all the genes listed herein, for example at least 20 genes, preferably at least 100 genes, preferably at least 264 genes, yet more preferably up to 2059 genes or fragments thereof.

The method of the invention is therefore based on the identification of gene signatures in the evaluated samples, determined from the analysis of the expression of said at least 100 genes or fragments thereof, furthermore disclosed at least 264 genes, or up to 2059 genes or fragments thereof. The pattern of expression of said genes allows the studied sample to cluster with a group of RA reference samples previously collected earlier. The same is true for OA, SLE, MIC and SA. Levels of gene expression in healthy subjects are no longer needed in order to obtain a result.

In an embodiment, the expression profiles of these genes can be used in identifying whether the individual has a rheumatic condition selected from SLE, OA, RA, SA, or MIC. In particular, the pattern of expression of said genes allows the studied sample to cluster with a group of RA, OA, SLE, MIC or SA reference samples.

The way said at least 20 genes, preferably at least 100 genes preferably at least 264 genes, yet more preferably up to 2059 genes or fragments thereof, are clustering is important, independently of the disease to be determined. The samples are compared with reference samples using, for example, Pearson correlation.

In an embodiment, the present invention provides early identification between at least five rheumatic conditions based on the analysis of gene expression profiles in a biological sample, such as synovial sample, of a subject with arthritis. Preferably said method uses low-density DNA-spotted microarrays.

Those skilled in the art will immediate recognize the many other effects and advantages of the present method and the numerous possibilities for end uses of the present invention from the detailed description and examples provided below.

### Brief description of the figures

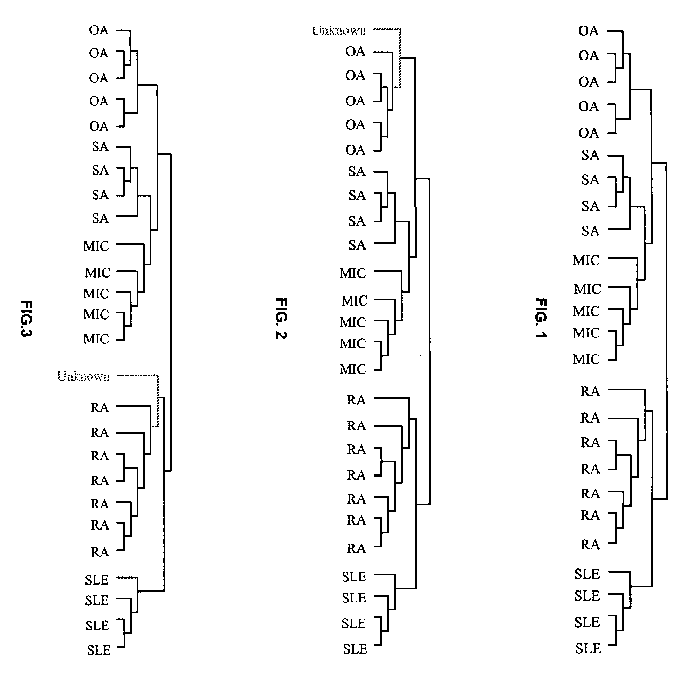

Figure 1: Represents the results of a supervised hierarchical clustering study of OA, RA, SLE, MIC and SA reference synovial samples based on the levels expression of 264, preferably 100 selected genes to be spotted on the low-density microarray slide in an embodiment according to the invention.
Figure 2: Represents the results of a supervised hierarchical clustering study of synovial sample from patient with unknown diagnosis (RA versus OA). The sample clusters with OA synovial reference samples based on analysis of 264, preferably 100 selected genes according to an embodiment of the present invention.
Figure 3: Represents the results of a supervised hierarchical clustering study of synovial sample from patient with unknown diagnosis (RA versus SA). The sample clusters with RA synovial reference samples based on analysis of 264, preferably 100 selected genes according to an embodiment of the present invention.

### Detailed description

Determination of the expression profile of at least 20 genes, preferably at least 100 genes listed herein provides a tool to screen for, diagnose, and also classify these diseases. The present invention allows determining or diagnosing whether subjects are afflicted with a particular form of arthritis.

Preferably, the method comprises determining in said biological sample, in particular in said synovial sample, the expression level of at least 20, at least 50, at least 100, at least 120, at least 150, at least 180, at least 200, at least 220, at least 240, at least 250, at least 260, at least 264, at least 270, or at least 300 genes or fragments thereof, preferably up to 2059 genes or fragments thereof.

In an embodiment, the method comprises determining in said biological sample, in particular in said synovial sample, the expression level of all the genes or fragment thereof listed in Table 1. In an embodiment, the method comprises determining in said biological sample, in particular in said synovial sample, the expression level of all the genes or fragment thereof listed in Table 2. In an embodiment, the method comprises determining in said biological sample, in particular in said synovial sample, the expression level of all the genes or fragment thereof listed in Table 3. In an embodiment, the method comprises determining in said biological sample, in particular in said synovial sample, the expression level of all the genes or fragment thereof listed in Table 4.

In an embodiment, said method comprises:
- providing for said biological sample the gene expression profile of at least 20, at least 50, at least 100, at least 120, at least 150, at least 180, at least 200, at least 220, at least 240, at least 250, at least 260, at least 264, at least 270, or at least 300 genes or fragments thereof as defined herein,
- providing reference profiles by establishing a gene expression profile for reference samples from reference subjects afflicted by OA, RA, SA, SLE and MIC,
- clustering the subject profile together with reference profiles,
- determining the clustered position of said subject profile among the reference profiles, and
- assigning to said rheumatic condition of said subject the class that corresponds to said clustered position in case said subject profile is within any cluster of reference profiles, or assigning to said rheumatic condition of said subject a new rheumatic condition class.

More preferably, said method comprises determining in said biological sample the expression level of at least 20, at least 50 , at least 100, at least 120, at least 150, at least 180, at least 200, at least 220, at least 240, at least 250, at least 260, at least 264, at least 270, or at least 300 genes or fragments thereof (preferably up to 2059 genes of fragment thereof) selected from the 2059 genes listed in Table 1 that were found by ANOVA to be differentially expressed between the five conditions.

The method also comprises determining in said biological sample the expression level of at least 20, at least 50 , at least 100, at least 120, at least 150, at least 180, at least 200, at least 220, at least 240, at least 250, at least 260, at least 270, or at least 300 genes or fragments thereof (preferably up to 2059 genes of fragment thereof) selected from the groups (a), (b), (c), (d), (e), (f), (g), (h), (i), and (j), wherein group (a) comprises IPLAC8, IRF4, SAMD3, HLA-DOB, EOMES, PDK1, BG548679, AK25048, AU146285, P2RY8, Al225238, JAK3, LAX, PTPN7, RP26, TRIM, SLAMF1, PTPRCAP, LCK, PTP4A3, Al825068, BCL11B, CD79A, IL7R, GPR18, STRBP, C20orf103, AA732944, ZAP70, SLC38A1, RUNX3, TOSO, BCL11A, NELL2, ICAM3, LTB, TCF7, TRD@; wherein group (b) comprises ANK3, CREB5, FAH, FKBP7, AF009316, KLF4, ANKH, NTN4, THBS4, SCAR3, CYP3A5, MGP, AMN, CMAH, FN1, GABRA4, ABCC6, BE503823, SLC24A6; wherein group (c) comprises CXCL11, LOC113730, cig5, STAT1, GM2A, G1P3, IFIT4, EIF5A, EPSTI1, MX1, IFI44L, IFI27, LOC129607, G1P2, IFIT1, FLJ39885, OAS2, FLJ20637, OAS1, MDA5, OAS3, LILRA2, TGFBRAP1, BST2, OASL, CEB1, HLA-DOA, KIS, GNB4, GM2A, CLECSF12, AW262311, CALR, FPRL2, MAP3K2, FLJ20668, CYBB, SNX10, GRB2, GPR43, FLJ20035, C1QG, ARF6, IFI35, GM2A, FLJ21069, BE674143, wherein group (d) comprises ZNF607, X07868, AU157716, CCDC3, CPSF1, AW029203, AK022838, CCNL2, C7orf10, SEC24D, AFG3L1, TLE2, PCSK5, AA706701, C18orf18, OSBPL6, BC042472, AUTS2, SOX4, PTPRD, AA572675, COL18A1, COL16A1, GPM6B, SCG2, TNC, TP53111, SCRG1, COL12A1, AL832806, AA912540, MYST4, STEAP, ARNT2, AA854843, KIAA0484, AU147442, PKN3, SYNE1, DSPG3, AW903934, FBXO26, LOC200772, AF116709; wherein group (e) comprises C7orf10, SCRG1, DNAJC12, FNDC4, AK024204, NBL1, BF591996, ANKH, DIO2 SGCD, T90703, SPOCK, CKLFSF4, AW162210, CDC42BPA, KIAA1171, FKSG17, N73742, ZNF515, PTPRS, CTDSPL, NRP2, EXTL2, CCND1, CALU, PVR, ATBF1, AFURS1, SYNPO, MYO7A,

KIAA1450, SLC35B4; wherein group (f) comprises BCL2L11, MBNL2, LOC113730, TXNIP, SRPR, RNASET2, DHX9, RUNX2; wherein group (g) comprises CYP3A5, MSI2, FZD8, AW265065, DBC-1, FN1, ANGPTL2, FB14, C14orf131, BF057799, KLF4, SRPR, TTC3, COPA, PCDHGA11I; wherein group (h) comprises IRF4, CXCL13, Al823917, BCL11 B, AA789123, cig5, PLAC8, CD209, IAN4L1, G1P3, NOD3, KIAA1268, HRMT1L1, AI821404, G1P2, COPG, FLJ33814, H963, TAP1, PTP4A3, CUL5, JAK3, AW504569, AA809449, CCL8, ZC3HDC1, SYNCOILIN, KIAA0090, GGA2, NAP1L, ETV6; wherein group (i) comprises AW903934, AL037998, AK024712, AW612461, BM873997, AK000795, FLJ00133, FOXC1, MGC43690, BF590303, AI090764, BF221547, ID4, AW296081, PGF, RGS5, W80359, AF086069, FLJ32949, RAMP3, T87730, AI742685, GPR4, GRID1, FAM20A, FRMD4, RUNX2, FLJ13089, TBX2, RAPGEF2, BM353142; wherein group (j) comprises SLC1A4, LIG3, EMILIN2, ALDH1A1, TNFSF8, PB1, SNX10, AA365670, YWHAZ, EIF5A, ZNF581, BAG4, ARF6, HLA-DOA, LILRA2, CWF19L1, SLC15A2, PTPRC, GM2A, STRN3, CLECSF12, TCL1A, PKHD1L1 and CTLA4.

Preferably said at least 100 genes are selected from these 2059 genes, based on their ability to define a specific gene signature for each disease. Preferably the method comprises determining the level of expression of 100 genes or fragments thereof selected from the group comprising AI923633; ARPC4; ASH1L; AW008502; AW296081; AW612461; BAG2; BCL11B; BE676335; BG231773; BSG; BTBD1; C12orf30/FLJ13089; C14orf131; CALU; CCL5; CCND1; CD79A; CDC42BPA; CDC42SE1/SPEC1; CHD1; CHD9/FLJ12178; CPSF1; CST7; CTDSPL; DUT; EIF3EIP/EIF3S6IP; ETV6; EXTL2; FLJ21395 fis; FN1; FRMD4A; G3BP1; GPR4; GRID1; GYG1; HMGB1; IFI27; IFI6/G1P3; IFIT3/IFIT4; IL7R; JAK3; JOSD3/MGC5306; KIAA0090; KIAA1128; KIAA1377; KIAA1450; LCK; LOC129607; MYEOV2/LOC150678; NBL1; NELL2; OAS1; PARP12/ZC3HDC1; PDE5A; PGF; PHF21A/BHC80; PIK3C2A; PTBP1; PTEN; PTPN7; QKI; RAB8A; RALGPS2; RAP2A; RAPGEF2; RASGRP1; RBBP6; RGS5; RPL4; RSAD2/cig5; SFRS2B/SRP46; SFRS6; SIPA1L3; SLC15A2; SPARCL1; SUPT16H; SYNCOILIN; TARP /// TRGC2 /// TRGV9; TBC1D20/C20orf140; TBC1D24/KIAA1171; THRAP3; TI-227H /// TUG1; TLE2; TMEM43/MGC3222; TNFSF8; TOX; TRBC1 /// TRBV19; TSPAN3/TM4SF8; UCKL1/URKL1; WDR90/LOC197336; ZFHX3/ATBF1; T87730.

Preferably said at least 20 genes are selected from these 2059 genes, based on their ability to define a specific gene signature for each disease. Preferably the method comprises determining the level of expression of 20 genes or fragments thereof selected from the group comprising FLJ21395 fis, TTC3, NELL2, PTPN7, HLA-DOA, GPR171, COPA, KIAA0484, TRAT1, FNDC4, BCL11B, C14orf131, FKBP7, TBC1D24, AL037998, AI225238, LOC113730, AA789123, KIAA1377, AW504569.

In a particular embodiment, it is provided for a method of determining and classifying a rheumatic condition in at least one biological sample of a subject afflicted with said rheumatic condition, said method comprising:
- determining in said biological sample the level of expression (i.e. providing the gene expression profile) of at least 20, preferably at least 100, preferably at least 260 genes or fragments thereof (preferably up to 2059 genes or fragments thereof) selected from the groups (a), (b), (c), (d), (e), (f), (g), (h), (i), and (j), wherein group (a) comprises IPLAC8, IRF4, SAMD3, HLA-DOB, EOMES, PDK1, BG548679, AK25048, AU146285, P2RY8, AI225238, JAK3, LAX, PTPN7, RP26, TRIM, SLAMF1, PTPRCAP, LCK, PTP4A3, Al825068, BCL11B, CD79A, IL7R, GPR18, STRBP, C20orf103, AA732944, ZAP70, SLC38A1, RUNX3, TOSO, BCL11A, NELL2, ICAM3, LTB, TCF7, TRD@; wherein group (b) comprises ANK3, CREB5, FAH, FKBP7, AF009316, KLF4, ANKH, NTN4, THBS4, SCARA3, CYP3A5, MGP, AMN, CMAH, FN1, GABRA4; ABCC6, BE503823, SLC24A6; wherein group (c) comprises CXCL11, LOC113730, cig5, STAT1, GM2A, G1 P3, IFIT4, EIF5A, EPSTI1, MX1, IFI44L, IFI27, LOC129607, G1P2, IFIT1, FLJ39885, OAS2, FLJ20637, OAS1, MDA5, OAS3, LILRA2, TGFBRAP1, BST2, OASL, CEB1, HLA-DOA, KIS, GNB4, GM2A, CLECSF12, AW262311, CALR, FPRL2, MAP3K2, FLJ20668, CYBB, SNX10, GRB2, GPR43, FLJ20035, C1QG, ARF6, IFI35, GM2A, FLJ21069, BE674143, wherein group (d) comprises ZNF607, X07868, AU157716, CCDC3, CPSF1, AW029203, AK022838, CCNL2, C7orf10, SEC24D, AFG3L1, TLE2, PCSK5, AA706701, C18orf18, OSBPL6, BC042472, AUTS2, SOX4, PTPRD, AA572675, COL18A1, COL16A1, GPM6B, SCG2, TNC, TP53I11, SCRG1, COL12A1, AL832806, AA912540, MYST4, STEAP, ARNT2, AA854843, KIAA0484, AU147442, PKN3, SYNE1, DSPG3, AW903934, FBXO26, LOC200772, AF116709; wherein group (e) comprises C7orf10, SCRG1, DNAJC12, FNDC4, AK024204, NBL1, BF591996, ANKH, DI02, SGCD, T90703, SPOCK, CKLFSF4, AW162210, CDC42BPA, KIAA1171, FKSG17, N73742, ZNF515, PTPRS, CTDSPL, NRP2, EXTL2, CCND1, CALU, PVR, ATBF1, AFURS1, SYNPO, MYO7A, KIAA1450, SLC35B4; wherein group (f) comprises BCL2L11, MBNL2, LOC113730, TXNIP, SRPR, RNASET2, DHX9, RUNX2; wherein group (g) comprises CYP3A5, MSI2, FZD8, AW265065, DBC-1, FN1, ANGPTL2, FBI4, C14orf131, BF057799, KLF4, SRPR, TTC3, COPA, PCDHGA11I; wherein group (h) comprises IRF4, CXCL13, AI823917, BCL11B, AA789123, cig5, PLAC8, CD209, IAN4L1, G1P3, NOD3, KIAA1268, HRMT1L1, AI821404, G1P2, COPG, FLJ33814, H963, TAP1, PTP4A3, CUL5, JAK3, AW504569, AA809449, CCL8, ZC3HDC1, SYNCOILIN, KIAA0090, GGA2, NAP1L, ETV6; wherein group (i) comprises AW903934, AL037998, AK024712, AW612461, BM873997, AK000795, FLJ00133, FOXC1, MGC43690, BF590303, AI090764, BF221547, ID4, AW296081, PGF, RGS5, W80359, AF086069, FLJ32949, RAMP3, T87730, AI742685, GPR4, GRID1, FAM20A, FRMD4, RUNX2, FLJ13089, TBX2, RAPGEF2, BM353142; wherein group (j) comprises SLC1A4, LIG3, EMILIN2, ALDH1A1, TNFSF8, PB1, SNX10, AA365670, YWHAZ, EIF5A, ZNF581, BAG4, ARF6, HLA-DOA, LILRA2, CWF19L1, SLC15A2, PTPRC, GM2A, STRN3, CLECSF12, TCL1A, PKHD1L1, CTLA4,
- providing reference profiles by establishing a gene expression profile for reference samples from reference subjects afflicted by OA, RA, SA, SLE and MIC,
- clustering the subject profile together with reference profiles;
- determining the clustered position of said subject profile among the reference profiles, and
- assigning to said rheumatic condition of said subject the class that corresponds to said clustered position in case said subject profile is within any cluster of reference profiles, or assigning to said rheumatic condition of said subject a new rheumatic condition class.

According to the present invention, the clustering of said gene expression profiles is performed based on the information of differentially- expressed genes listed herein.

In a preferred embodiment, the analyses are based on the levels of expression of all the genes described in the categories (a), (b), (c), (d), (e), (f), (g), (h), (i) and (j) listed herein. According to a further embodiment, group (a) comprises genes more specifically over-expressed in RA: PLAC8, IRF4, SAMD3, HLA-DOB, EOMES, PDK1, BG548679, AK25048, AU146285, P2RY8, AI225238, JAK3, LAX, PTPN7, RP26, TRIM, SLAMF1, PTPRCAP, LCK, PTP4A3, AI825068, BCL11B, CD79A, IL7R, GPR18, STRBP, C20orf103, AA732944, ZAP70, SLC38A1, RUNX3, TOSO, BCL11A, NELL2, ICAM3, LTB, TCF7, TRD@;
group (b) comprises genes more specifically down-regulated in RA: ANK3, CREB5, FAH, FKBP7, AF009316, KLF4, ANKH, NTN4, THBS4, SCARA3, CYP3A5, MGP, AMN, CMAH, FN1, GABRA4, ABCC6, BE503823, SLC24A6;
group (c) comprises genes more specifically over-expressed in SLE : CXCL11, LOC113730, cig5, STAT1, GM2A, G1P3, IFIT4, EIF5A, EPSTI1, MX1, IFI44L, IFI27, LOC129607, G1P2, IFIT1, FLJ39885, OAS2, FLJ20637, OAS1, MDA5, OAS3, LILRA2, TGFBRAP1, BST2, OASL, CEB1, HLA-DOA, KIS, GNB4, GM2A, CLECSF12, AW262311, CALR, FPRL2, MAP3K2, FLJ20668, CYBB, SNX10, GRB2, GPR43, FLJ20035, C1QG, ARF6, IFI35, GM2A, FLJ21069, BE674143;
group (d) comprises genes more specifically down-regulated in SLE : ZNF607, X07868, AU157716, CCDC3, CPSF1, AW029203, AK022838, CCNL2, C7orf10, SEC24D, AFG3L1, TLE2, PCSK5, AA706701, C18orf18, OSBPL6, BC042472, AUTS2, SOX4, PTPRD, AA572675, COL18A1, COL16A1, GPM6B, SCG2, TNC, TP53I11, SCRG1, COL12A1, AL832806, AA912540, MYST4, STEAP, ARNT2, AA854843, KIAA0484, AU147442, PKN3, SYNE1, DSPG3, AW903934, FBXO26, LOC200772, AF116709;
group (e) comprises genes more specifically over-expressed in OA : C7orf10, SCRG1, DNAJC12, FNDC4, AK024204, NBL1, BF591996, ANKH, DIO2 SGCD, T90703, SPOCK, CKLFSF4, AW162210, CDC42BPA, KIAA1171, FKSG17, N73742, ZNF515, PTPRS, CTDSPL, NRP2, EXTL2, CCND1, CALU, PVR, ATBF1, AFURS1, SYNPO, MYO7A, KIAA1450, SLC35B4;
group (f) comprises genes more specifically down-regulated in OA: BCL2L11, MBNL2, LOC113730, TXNIP, SRPR, RNASET2, DHX9, RUNX2;
group (g) comprises genes more specifically over-expressed in MIC: CYP3A5, MSI2, FZD8, AW265065, DBC-1, FN1, ANGPTL2, FBI4, C14orf131, BF057799, KLF4, SRPR, TTC3, COPA, PCDHGA11;
group (h) comprises genes more specifically down-regulated in MIC : IRF4, CXCL13, AI823917, BCL11B, AA789123, cig5, PLAC8, CD209, IAN4L1, G1P3, NOD3, KIAA1268, HRMT1L1, AI821404, G1P2, COPG, FLJ33814, H963, TAP1, PTP4A3, CUL5, JAK3, AW504569, AA809449, CCL8, ZC3HDC1, SYNCOILIN, KIAA0090, GGA2, NAP1L, ETV6;
group (i) comprises genes more specifically over-expressed in SA : AW903934, AL037998, AK024712, AW612461, BM873997, AK000795, FLJ00133, FOXC1, MGC43690, BF590303, AI090764, BF221547, ID4, AW296081, PGF, RGS5, W80359, AF086069, FLJ32949, RAMP3, T87730, AI742685, GPR4, GRID1, FAM20A, FRMD4, RUNX2, FLJ13089, TBX2, RAPGEF2, BM353142; and
group (j) comprises genes more specifically down-regulated in SA : SLC1A4, LIG3, EMILIN2, ALDH1A1, TNFSF8, PB1, SNX10, AA365670, YWHAZ, EIFSA, ZNF581, BAG4, ARF6, HLA-DOA, LILRA2, CWF19L1, SLC15A2, PTPRC, GM2A, STRN3, CLECSF12, TCL1A, PKHD1L1, CTLA4.

As used herein the term "clustering" refers to the activity of collecting, assembling and/or uniting into a cluster or clusters items with the same or similar elements, a "cluster" referring to a group or number of the same or similar items, i.e. gene expression profiles, gathered or occurring closely together based on similarity of characteristics.

The process of clustering used in a method of the present invention may be any mathematical process known to compare items for similarity in characteristics, attributes, properties, qualities, effects, parameters, etc.. Statistical analysis, such as for instance multivariance analysis, or other methods of analysis may be used. Preferably methods of analysis such as self-organizing maps, hierarchical clustering, multidimensional scaling, principle component analysis, supervised learning, k-nearest neighbors, support vector machines and the like.

In an embodiment, the clustering step is performed according to a statistical procedure, comprising: hierarchical clustering selected from complete linkage clustering; average linkage clustering and/or single linkage clustering; using at least one of the following metrics selected from Euclidean distance; Manhattan distance; Average dot product; Pearson correlation; Pearson uncentered; Pearson squared; Cosine correlation; Covariance value; Spearman Rank correlation; Kedall's Tau; or Mutual information. Preferably the present method comprises performing supervised hierarchical clustering analysis. Pearson correlation coefficients are calculated between pairs of samples.

In a preferred embodiment, the method of the invention comprises the steps of determining the level of expression of the following genes (that define a specific signature in each disorder): PLAC8, IRF4, SAMD3, HLA-DOB, EOMES, PDK1, BG548679, AK25048, AU146285, P2RY8, AI225238, JAK3, LAX, PTPN7, RP26, TRIM, SLAMF1, PTPRCAP, LCK, PTP4A3, AI825068, BCL11B, CD79A, IL7R, GPR18, STRBP, C20orf103, AA732944, ZAP70, SLC38A1, RUNX3, TOSO, BCL11A, NELL2, ICAM3, LTB, TCF7, TRD@; ANK3, CREB5, FAH, FKBP7, AF009316, KLF4, ANKH, NTN4, THBS4, SCARA3, CYP3A5, MGP, AMN, CMAH, FN1, GABRA4, ABCC6, BE503823, SLC24A6; CXCL11, LOC113730, cig5, STAT1, GM2A, G1P3, IFIT4, EIF5A, EPSTI1, MX1, IFI44L, IFI27, LOC129607, G1P2, IFIT1, FLJ39885, OAS2, FLJ20637, OAS1, MDA5, OAS3, LILRA2, TGFBRAP1, BST2, OASL, CEB1, HLA-DOA, KIS, GNB4, CLECSF12, AW262311, CALR, FPRL2, MAP3K2, FLJ20668, CYBB, SNX10, GRB2, GPR43, FLJ20035, C1QG, ARF6, IFI35, FLJ21069, BE674143; ZNF607, X07868, AU157716, CCDC3, CPSF1, AW029203, AK022838, CCNL2, C7orf10, SEC24D, AFG3L1, TLE2, PCSK5, AA706701, C18orf18, OSBPL6, BC042472, AUTS2, SOX4, PTPRD, AA572675, COL18A1, COL16A1, GPM6B, SCG2, TNC, TP53I11, COL12A1, AL832806, AA912540, MYST4, STEAP, ARNT2, AA854843, KIAA0484, AU147442, PKN3, SYNE1, DSPG3, AW903934, FBXO26, LOC200772, AF116709; SCRG1, DNAJC12, FNDC4, AK024204, NBL1, BF591996, DIO2 SGCD, T90703, SPOCK, CKLFSF4, AW162210, CDC42BPA, KIAA1171, FKSG17, N73742, ZNF515, PTPRS, CTDSPL, NRP2, EXTL2, CCND1, CALU, PVR, ATBF1, AFURS1, SYNPO, MYO7A, KIAA1450, SLC35B4; BCL2L11, MBNL2, TXNIP, RNASET2, DHX9, RUNX2; MSI2, FZD8, AW265065, DBC-1, ANGPTL2, FBI4, C14orf131, BF057799, SRPR, TTC3, COPA, PCDHGA11; CXCL13, AI823917, AA789123, CD209, IAN4L1, NOD3, KIAA1268, HRMT1L1, AI821404, COPG, FLJ33814, H963, TAP1, CUL5, AW504569, AA809449, CCL8, ZC3HDC1, SYNCOILIN, KIAA0090, GGA2, NAP1L, ETV6, AL037998, AK024712, AW612461, BM873997, AK000795, FLJ00133, FOXC1, MGC43690, BF590303, AI090764, BF221547, ID4, AW296081, PGF, RGS5, W80359, AF086069, FLJ32949, RAMP3, T87730, AI742685, GPR4, GRID1, FAM20A, FRMD4, FLJ13089, TBX2, RAPGEF2, BM353142; SLC1A4, LIG3, EMILIN2, ALDH1A1, TNFSF8, PB1, AA365670, YWHAZ, ZNF581, BAG4, CWF19L1, SLC15A2, PTPRC, STRN3, TCL1A, PKHD1L1, CTLA4; and identifying whether the subject's synovial sample has a pattern or profile or expression of said genes which correlates with the presence of a rheumatic condition such SLE, OA, RA, MIC or SA by clustering analysis compared to reference samples.

In a preferred embodiment, the method comprises the steps of determining the level of expression of the following genes (that define a specific signature in each disorder): AI923633; ARPC4; ASH1L; AW008502; AW296081; AW612461; BAG2; BCL11B; BE676335; BG231773; BSG; BTBD1; C12orf30/FLJ13089; C14orf131; CALU; CCL5; CCND1; CD79A; CDC42BPA; CDC42SE1/SPEC1; CHD1; CHD9/FLJ12178; CPSF1; CST7; CTDSPL; DUT; EIF3EIP/EIF3S6IP; ETV6; EXTL2; FLJ21395 fis; FN1; FRMD4A; G3BP1; GPR4; GRID1; GYG1; HMGB1;IFI27; IFI6/G1P3; IFIT3/IFIT4; IL7R; JAK3; JOSD3/MGC5306; KIAA0090; KIAA1128; KIAA1377; KIAA1450; LCK; LOC129607; MYEOV2/LOC150678; NBL1; NELL2; OAS1; PARP12/ZC3HDC1; PDE5A; PGF; PHF21A/BHC80; PIK3C2A; PTBP1; PTEN; PTPN7; QKI; RAB8A; RALGPS2; RAP2A;. RAPGEF2; RASGRP1; RBBP6; RGS5; RPL4; RSAD2/cig5; SFRS2B/SRP46; SFRS6; SIPA1L3; SLC15A2; SPARCL1; SUPT16H; SYNCOILIN; TARP /// TRGC2 /// TRGV9; TBC1D20/C20orf140; TBC1D24/KIAA1171; THRAP3; TI-227H /// TUG1; TLE2; TMEM43/MGC3222; TNFSF8; TOX; TRBC1 /// TRBV19; TSPAN3/TM4SF8; UCKL1/URKL1; WDR90/LOC197336; ZFHX3/ATBF1; T87730, and identifying whether the subject's synovial sample has a pattern or profile or expression of said genes which correlates with the presence of a rheumatic condition such SLE, OA, RA, MIC or SA by clustering analysis compared to reference samples.

In a preferred embodiment, the method comprises the steps of determining the level of expression of the following genes (that define a specific signature in each disorder): FLJ21395 fis, TTC3, NELL2, PTPN7, HLA-DOA, GPR171, COPA, KIAA0484, TRAT1, FNDC4, BCL11B, C14orf131, FKBP7, TBC1D24, AL037998, AI225238, LOC113730, AA789123, KIAA1377, AW504569, and identifying whether the subject's synovial sample has a pattern or profile or expression of said genes which correlates with the presence of a rheumatic condition such SLE, OA, RA, MIC or SA by clustering analysis compared to reference samples.

In an embodiment, the step of providing reference profiles for OA, RA, SA, SLE and MIC, comprises the steps of : providing a plurality of reference samples from a plurality of reference subjects afflicted by OA, RA, SA, SLE and MIC; providing reference profiles by establishing a gene expression profile for each of said reference samples individually; clustering said individual reference profiles according to a statistical procedure, comprising hierarchical clustering; and Pearson correlation coefficient analysis; and assigning an OA, RA, SA, SLE or MIC class to each cluster.

In an embodiment, the method comprises determining the level of expression of the above listed genes, performing supervised hierarchical clustering analysis, measuring correlation coefficient and identifying whether the subject's sample has a pattern or profile or expression of said genes which correlates with the presence of a rheumatic condition.

The present invention provides a method for diagnosing SLE, OA, RA, SA, or MIC in a subject afflicted by a undefined rheumatic conditions comprising: producing a classification for several SLE, OA, RA, SA, and MIC references samples using the genes listed herein; defining cluster-specific genes for each cluster by selecting those genes of which the expression level characterizes the clustered position of the corresponding SLE, OA, RA, SA, or MIC class, determining the level of expression of at least 20, preferably at least 100 number of said cluster-specific genes in subject afflicted with a rheumatic condition; establishing whether the level of expression of said cluster-specific genes in said subject shares sufficient similarity to the level of expression that characterizes an SLE, OA, RA, SA, or MIC class to thereby determine the presence of a specific rheumatic condition corresponding to said class in said subject.

As used herein the term "biological sample" refers to a sample that comprises a biomolecule that permits the expression level of a gene to be determined. Representative biomolecules include, but are not limited to total RNA, mRNA, and polypeptides, and derivatives of these molecules such as cDNAs and ESTs. As such, a biological sample can comprise a cell or a group of cells. Preferably, said biological sample is a synovial sample, more preferably a knee synovial sample.

As used herein the term "subject" refers to any vertebrate species. Preferably, the term subject encompasses warm-blooded vertebrates, more preferably mammals. More particularly contemplated are mammals such as humans, as well as animals such as carnivores other than humans (such as cats and dogs), swine (pigs, hogs, and wild boars), poultry, ruminants (such as cattle, oxen, sheep, giraffes, deer, goats, bison, and camels), and horses.

For example, said rheumatic condition is determined as SLE when the gene expression profile of the at least 20, at least 50, at least 100, at least 120, at least 150, at least 180, at least 200, at least 220, at least 240, at least 250, at least 260, at least 264, at least 270, or at least 300 genes preferably up to 2059 genes is similar to known SLE samples. That includes but is not limited to up-regulation of interferon-induced genes, down-regulation of genes involved in ECM homeostasis and distinct other patterns of expression of all the genes present on a slide. The same is true for RA (up- and down-regulation of specific groups of genes compared to each other), OA, SA and MIC

When describing the invention, the terms used are to be construed in accordance with the following definitions, unless a context dictates otherwise:
As used in the specification and the appended claims, the singular forms "a", "an," and "the" include plural referents unless the context clearly dictates otherwise. By way of example, "a method" means one method or more than one method.

The term "and/or" as used in the present specification and in the claims implies that the phrases before and after this term are to be considered either as alternatives or in combination.

As used herein, the term "profile" refers to a repository of the expression level data that can be used to compare the expression levels of different genes among various subjects. As used herein the term "gene" encompasses sequences including, but not limited to a coding sequence, a promoter region, a transcriptional regulatory sequence, a non-expressed DNA segment that is a specific recognition sequence for regulatory proteins, a non-expressed DNA segment that contributes to gene expression, a DNA segment designed to have desired parameters, sense and anti-sense strands of genomic DNA (i.e. including any introns occurring therein), EST, RNA generated by transcription of genomic DNA (i.e. prior to splicing), RNA generated by splicing of RNA transcribed from genomic DNA, and proteins generated by translation of spliced RNA (e. g. including proteins both before and after cleavage of normally cleaved regions such as transmembrane signal sequences), cDNA made by reverse transcription of an RNA generated by transcription of genomic DNA (including spliced RNA) and fragments thereof, or combinations thereof. As used herein the term "fragment" shall be understood to mean a nucleic acid that is the same as part of, but not all of a nucleic acid that forms a gene. The term "fragment" also encompasses a part, but not all of an intergenic region.

The term "increased expression" and "decreased expression" refers to expression of the gene in a sample, at a greater or lesser level, respectively, than the level of expression of said gene (e. g. at least two-fold greater or lesser level) in a diseased control (reference sample). The gene is said to be up-regulated or over-expressed or down-regulated or under-expressed if either the gene is present at a greater or lesser level, respectively, than the level in a diseased control. Expression of a gene in a sample is "significantly" higher or lower than the level of expression of a gene in a diseased control if the level of expression of the gene is greater or less, respectively, than the level by an amount greater than the standard error of the assay employed to assess expression, and preferably at least twice, and more preferably three, four, five or ten times that amount. Alternately, expression of the gene in the sample can be considered "significantly" higher or lower than the level of expression in a diseased control if the level of expression is at least about two, and preferably at least about three, four, or five times, higher or lower, respectively, than the level of expression of the gene in said diseased control.

The present invention provides arrays comprising probes for detection of polynucleotides (transcriptional state) or for detection of proteins (translational state) in order to detect differentially-expressed genes of the invention. By "array" is intended a solid support or substrate with peptide or nucleic acid probes attached to said support or substrate. Arrays typically comprise a plurality of different nucleic acid or peptide capture probes that are coupled to a surface of a substrate in different, known locations. These arrays, also described as "microarrays" or colloquially "chips" have been generally described in the art. These arrays may generally be produced using mechanical synthesis methods or light directed synthesis methods which incorporate a combination of photolithographic methods and solid phase synthesis methods.

also provided are microarrays which are used to measure the values to be included in the expression profiles. Microarrays are particularly well suited for this purpose because of the reproducibility between different experiments. In an embodiment, the step of determination of the level of expression is performed using DNA-microarray (also referred as gene chip array), preferably low-density DNA-spotted microarray. As used herein low-density DNA-spotted microarray comprises spotting probes suitable for hybridizing from at least 20, or at least 100 to 5000 genes or fragments thereof, preferably from at least 20 or at least 100 to 3000 genes or fragments thereof, more preferably from at least 20 or at least 100 to 2050 genes or fragment thereof, even more preferably from at least 100 to 500 genes, even more preferably from at least 20 to 500 genes.

Preferably, said method involves clustering of gene expression profiles based on, for instance, DNA-microarray-acquired values for hybridization intensities for each gene. The skilled person is capable of designing oligonucleotide probes that can be used in methods of the present invention. Preferably, such probes are immobilized on a solid surface as to form an oligonucleotide microarray of the invention. The oligonucleotide probes useful in methods of the present invention are capable of hybridizing under stringent conditions to the at least 20 at least 50, at least 100, at least 120, at least 150, at least 180, at least 200, at least 220, at least 240, at least 250, at least 260, at least 264, at least 270, or at least 300 (preferably to up to 2059) rheumatic conditions-associated nucleic acids as described herein.

In some embodiments, each probe in the array detects a nucleic acid molecule selected from the nucleic acid molecules listed in Tables 1, 2, 3 or 4.

Although a planar array surface is preferred, the array may be fabricated on a surface of virtually any shape or even a multiplicity of surfaces. Arrays may be peptides or nucleic acids on beads, gels, polymeric surfaces, and fibers such as fiber optics, glass or any other appropriate substrate. Arrays may be packaged in such a manner as to allow for diagnostics or other manipulation of an all-inclusive device.

Suitable probes for said microarray comprise probes for genes or fragments thereof as listed in Tables 1, 2, 3 and 4. Preferably suitable probes for said microarray comprise probes for PLAC8, IRF4, SAMD3, HLA-DOB, EOMES, PDK1, BG548679, AK025048, AU146285, P2RY8, AI225238, JAK3, LAX, PTPN7, RP26, TRIM, SLAMF1, PTPRCAP, LCK, PTP4A3, AI825068, BCL11B; CD79A, IL7R, GPR18, STRBP, C20orf103, AA732944, ZAP70, SLC38A1, RUNX3, TOSO, BCL11A, NELL2, ICAM3, LTB, TCF7, TRD@; ANK3, CREB5, FAH, FKBP7, AF009316, KLF4, ANKH, NTN4, THBS4, SCARA3, CYP3A5, MGP, AMN, CMAH, FN1, GABRA4, ABCC6, BE503823, SLC24A6; CXCL11, LOC113730, cig5, STAT1, GM2A, G1P3, IFIT4, EIF5A, EPSTI1, MX1, IFI44L, IFI27, LOC129607, G1P2, IFIT1, FLJ39885, OAS2, FLJ20637, OAS1, MDA5, OAS3, LILRA2, TGFBRAP1, BST2, OASL, CEB1, HLA-DOA, KIS, GNB4, CLECSF12, AW262311, CALR, FPRL2, MAP3K2, FLJ20668, CYBB, SNX10, GRB2, GPR43, FLJ20035, C1 QG, ARF6, IFI35, FLJ21069, BE674143; ZNF607, X07868, AU157716, CCDC3, CPSF1, AW029203, AK022838, CCNL2, C7orf10, SEC24D, AFG3L1, TLE2, PCSK5, AA706701, C18orf18, OSBPL6, BC042472, AUTS2, SOX4, PTPRD, AA572675, COL18A1, COL16A1, GPM6B, SCG2, TNC, TP53I11, COL12A1, AL832806, AA912540, MYST4, STEAP, ARNT2, AA854843, KIAA0484, AU147442, PKN3, SYNE1, DSPG3, AW903934, FBX026, LOC200772, AF116709; SCRG1, DNAJC12, FNDC4, AK024204, NBL1, BF591996, DIO2, SGCD, T90703, SPOCK, CKLFSF4, AW162210, CDC42BPA, KIAA1171, FKSG17, N73742, ZNF515, PTPRS, CTDSPL, NRP2, EXTL2, CCND1, CALU, PVR, ATBF1, AFURS1, SYNPO, MYO7A, KIAA1450, SLC35B4; BCL2L11, MBNL2, TXNIP, RNASET2, DHX9, RUNX2; MSI2, FZD8, AW265065, DBC-1, ANGPTL2, FBI4, C14orf131, BF057799, SRPR, TTC3, COPA, PCDHGA11; CXCL13, AI823917, AA789123, CD209, IAN4L1, NOD3, KIAA1268, HRMT1L1, AI821404, COPG, FLJ33814, H963, TAP1, CUL5, AW504569, AA809449, CCLB, ZC3HDC1, SYNCOILIN, KIAA0090, GGA2, NAP1L, ETV6, AL037998, AK024712, AW612461, BM873997, AK000795, FLJ00133, FOXC1, MGC43690, BF590303, AI090764, BF221547, ID4, AW296081, PGF, RGS5, W80359, AF086069, FLJ32949, RAMP3, T87730, AI742685, GPR4, GRID1, FAM20A, FRMD4, FLJ13089, TBX2, RAPGEF2, BM353142; SLC1A4, LIG3, EMILIN2, ALDH1A1, TNFSF8, PB1, AA365670, YWHAZ, ZNF581, BAG4, CWF19L1, SLC15A2, PTPRC, STRN3, TCL1A, PKHD1L1, CTLA4. Preferably suitable probes for said microarray comprise probes for AI923633; ARPC4; ASH1L; AW008502; AW296081; AW612461; BAG2; BCL11B; BE676335; BG231773; BSG; BTBD1; C12orf30/FLJ13089; C14orf131; CALU; CCL5; CCND1; CD79A; CDC42BPA; CDC42SE1/SPEC1; CHD1; CHD9/FLJ12178; CPSF1; CST7; CTDSPL; DUT; EIF3EIP/EIF3S6IP; ETV6; EXTL2; FLJ21395 fis; FN1; FRMD4A; G3BP1; GPR4; GRID1; GYG1; HMGB1; IFI27; IFI6/G1P3; IFIT3/IFIT4; IL7R; JAK3; JOSD3/MGC5306; KIAA0090; KIAA1128; KIAA1377; KIAA1450; LCK; LOC129607; MYEOV2/LOC150678; NBL1; NELL2; OAS1; PARP12/ZC3HDC1; PDE5A; PGF; PHF21A/BHC80; PIK3C2A; PTBP1; PTEN; PTPN7; QKI; RAB8A; RALGPS2; RAP2A; RAPGEF2; RASGRP1; RBBP6; RGS5; RPL4; RSAD2/cig5; SFRS2B/SRP46; SFRS6; SIPA1L3; SLC15A2; SPARCL1; SUPT16H; SYNCOILIN; TARP /// TRGC2 /// TRGV9; TBC1D20/C20orf140; TBC1D24/KIAA1171; THRAP3; TI-227H /// TUG1; TLE2; TMEM43/MGC3222; TNFSF8; TOX; TRBC1 /// TRBV19; TSPAN3/TM4SF8; UCKL1/URKL1; WDR90/LOC197336; ZFHX3/ATBF1; T87730.

Analysis can be conducted using for example average or complete linkage clustering and Pearson's correlation. Expression files can be analyzed using for example the open-source softwares: TMEV (Tigr Multiarray Experiment Viewer) (www.tigr.org/software), J-Express: http://www.ii.uib.no/∼bjarted/jexpress, or Genesis®, genome.tugraz.at/Software/Genesis/ or using Support vector machine (SVM) or Leave-one-out analyses in GeneSpring.

According to the underlying embodiments, the present method is performed using a plurality (e.g. from 20 to 2059 genes, for e.g. at least 20, at least 100, at least 264 genes) of genes. In such methods, the level of expression in the sample of said genes as described above can be compared with the level of expression of the plurality of genes in reference samples of the same type obtained from diseased control afflicted with rheumatic conditions for example RA, OA, SLE, MIC, or SA.

The methods of the present invention are particularly useful for subjects with identified inflammatory synovitis or other symptoms associated with rheumatic conditions.

The sample can, of course, be subjected to a variety of well-known post-collection preparative and storage techniques (e. g. fixation, storage, freezing, lysis, homogenization, DNA or RNA extraction, ultrafiltration, concentration, evaporation, centrifugation, etc.) prior to determining the level of expression in the sample.

Expression of a gene according to the invention may be assessed by any of a wide variety of well known methods for detecting expression of a protein or transcribed molecule. Nonlimiting examples suitable determination steps include immunological methods for detection of secreted, cell-surface, cytoplasmic, or nuclear proteins, protein purification methods, protein function or activity assays, nucleic acid hybridization methods, nucleic acid reverse transcription methods, and nucleic acid amplification methods. Such methods may also include physical methods such as liquid and gas chromatography, mass spectroscopy, nuclear magnetic resonance and other imaging technologies.

In a preferred embodiment, the step of determination of the level of expression is performed using microarray, preferably DNA-microarray, more preferably low-density DNA-spotted microarray. Suitable probes for said microarray are identified hereunder.

In particular, a mixture of transcribed polynucleotides obtained from the sample is contacted with a substrate having fixed thereto a polynucleotide complementary to or homologous with at least a portion (e: g. at least 7, 10, 1 5, 20, 25, 30, 40, 50, 100, 250, 296, or more nucleotide residue) of a RNA transcript encoded by a gene for use in the invention. If polynucleotides complementary to or homologous with a RNA transcript encoded by the gene for use in the invention are differentially detectable on the substrate (e. g. detectable using radioactivity, different chromophores or fluorophores), are fixed to different selected positions, then the levels of expression of a plurality of genes can be assessed simultaneously using a single substrate.

When the assay has an internal control, which can be, for example, a known quantity of a nucleic acid derived from a gene for which the expression level is either known or can be accurately determined, unknown expression levels of other genes can be compared to the known internal control. More specifically, when the assay involves hybridizing labeled total RNA to a solid support comprising a known amount of nucleic acid derived from reference genes, an appropriate internal control could be a housekeeping gene (e. g. glucose-6-phosphate dehydrogenase or elongation factor-1), a housekeeping gene being defined as a gene for which the expression level in all cell types and under all conditions is substantially the same. Use of such an internal control allows a discrete expression level for a gene to be determined (e. g. relative to the expression of the housekeeping gene) both for the nucleic acids present on the solid support and also between different experiments using the same solid support. This discrete expression level can then be normalized to a value relative to the expression level of the control gene (for example, a housekeeping gene). As used herein, the term "normalized", and grammatical derivatives thereof, refers to a manipulation of discrete expression level data wherein the expression level of a reference gene is expressed relative to the expression level of a control gene. For example, the expression level of the control gene can be set at 1, and the expression levels of all reference genes can be expressed in units relative to the expression of the control gene.

In one embodiment, nucleic acids isolated from a biological sample are hybridized to a microarray, wherein the microarray comprises nucleic acids corresponding to those genes to be tested as well as internal control genes. The genes are immobilized on a solid support, such that each position on the support identifies a particular gene. Solid supports include, but are not limited to nitrocellulose and nylon membranes. Solid supports can also be glass or silicon-based (i.e. gene "chips"). Any solid support can be used in the methods of the presently claimed subject matter, so long as the support provides a substrate for the localization of a known amount of a nucleic acid in a specific position that can be identified subsequent to the hybridization and detection steps.

A microarray can be assembled using any suitable method known to one of skill in the art, and any one microarray configuration or method of construction is not considered to be a limitation of the disclosures

The present embodiments also encompasses a method for the determination and the classification of rheumatic conditions, said method comprising:
- first obtaining a polynucleotide sample from a biological sample, preferably a synovial sample, and
- then reacting the sample polynucleotide obtained in the first step with probes immobilized on a solid support having polynucleotide sequences corresponding to all or part of at least 100 genes selected from the genes or fragment thereof as listed in Table 1, preferably to all the genes or fragments thereof of Table 1 or to all the genes or fragments thereof of Table 2, or to all the genes or fragments thereof of Table 3, or to all the genes or fragments thereof of Table 4, preferably corresponding to all or part of at least 100 genes (more preferably all the genes) selected from PLAC8, IRF4, SAMD3, HLA-DOB, EOMES, PDK1, BG548679, AK025048, AU146285, P2RY8, AI225238, JAK3, LAX, PTPN7, RP26, TRIM, SLAMF1, PTPRCAP, LCK, PTP4A3, AI825068, BCL11B, CD79A, IL7R, GPR18, STRBP, C20orf103, AA732944, ZAP70, SLC38A1, RUNX3, TOSO, BCL11A, NELL2, ICAM3, LTB, TCF7, TRD@; ANK3, CREB5, FAH, FKBP7, AF009316, KLF4, ANKH, NTN4, THBS4, SCARA3, CYP3A5, MGP, AMN, CMAH, FN1, GABRA4, ABCC6, BE503823, SLC24A6; CXCL11, LOC113730, cig5, STAT1, GM2A, G1P3, IFIT4, EIF5A, EPSTI1, MX1, IFI44L, IFI27, LOC129607, G1P2, IFIT1, FLJ39885, OAS2, FLJ20637, OAS1, MDA5, OAS3, LILRA2, TGFBRAP1, BST2, OASL, CEB1, HLA-DOA, KIS, GNB4, CLECSF12, AW262311, CALR, FPRL2, MAP3K2, FLJ20668, CYBB, SNX10, GRB2, GPR43, FLJ20035, C1QG, ARF6, IFI35, FLJ21069, BE674143; ZNF607, X07868, AU157716, CCDC3, CPSF1, AW029203, AK022838, CCNL2, C7orf10, SEC24D, AFG3L1, TLE2, PCSK5, AA706701, C18orf18, OSBPL6, BC042472, AUTS2, SOX4, PTPRD, AA572675, COL18A1, COL16A1, GPM6B, SCG2, TNC, TP53I11, COL12A1, AL832806, AA912540, MYST4, STEAP, ARNT2, AA854843, KIAA0484, AU147442, PKN3, SYNE1, DSPG3, AW903934, FBXO26, LOC200772, AF116709; SCRG1, DNAJC12, FNDC4, AK024204, NBL1, BF591996, DIO2 SGCD, T90703, SPOCK, CKLFSF4, AW162210, CDC42BPA, KIAA1171, FKSG17, N73742, ZNF515, PTPRS, CTDSPL, NRP2, EXTL2, CCND1, CALU, PVR, ATBF1, AFURS1, SYNPO, MYO7A, KIAA1450, SLC35B4; BCL2L11, MBNL2, TXNIP, RNASET2, DHX9, RUNX2; MSI2, FZD8, AW265065, DBC-1, ANGPTL2, FBI4, C14orf131, BF057799, SRPR, TTC3, COPA, PCDHGA11; CXCL13, AI823917, AA789123, CD209, IAN4L1, NOD3, KIAA1268, HRMT1L1, AI821404, COPG, FLJ33814, H963, TAP1, CUL5, AW504569, AA809449, CCL8, ZC3HDC1, SYNCOILIN, KIAA0090, GGA2, NAP1L, ETV6, AL037998, AK024712, AW612461, BM873997, AK000795, FLJ00133, FOXC1, MGC43690, BF590303, AI090764, BF221547, ID4, AW296081, PGF, RGS5, W80359, AF086069, FLJ32949, RAMP3, T87730, AI742685, GPR4, GRID1, FAM20A, FRMD4, FLJ13089, TBX2, RAPGEF2, BM353142; SLC1A4, LIG3, EMILIN2, ALDH1A1, TNFSF8, PB1, AA365670, YWHAZ, ZNF581, BAG4, CWF19L1, SLC15A2, PTPRC, STRN3, TCL1A, PKHD1L1, CTLA4, and,
- detecting the reaction product and comparing with a reference reaction product.

Also provided are kits for use in practicing the subject methods. The term "kit" as used herein refers to any combination of reagents or apparatus that can be used to perform a method of the invention.

The present invention also provides kits useful for diagnosing, treating, and monitoring the disease state in subjects affected by a rheumatic condition. In one embodiment, the invention provides a kit for the determination and the classification of rheumatic conditions, the kit comprising a low density microarray comprising probes suitable for hybridizing with at least 20, preferably at least 100 genes or fragments thereof, selected from the genes listed in Tables 1, 2, 3 or 4. In one embodiment, said probes selectively hybridizes to a sequence at least 95% identical to a sequence of a gene as shown in Tables 1, 2, 3 or 4.

In an embodiment, said microarray comprises probes suitable for hybridizing with at least 100 genes up to 2059 genes selected from the group listed in Table 1. Preferably said 100 genes are those listed in Table 3.

In a preferred embodiment, said microarray comprises probes suitable for hybridizing with at least 264 genes selected from the group comprising PLAC8, IRF4, SAMD3, HLA-DOB, EOMES, PDK1, BG548679, AK025048, AU146285, P2RY8, AI225238, JAK3, LAX, PTPN7, RP26, TRIM, SLAMF1, PTPRCAP, LCK, PTP4A3, AI825068, BCL11B, CD79A, IL7R, GPR18, STRBP, C20orf103, AA732944, ZAP70, SLC38A1, RUNX3, TOSO, BCL11A, NELL2, ICAM3, LTB, TCF7, TRD@; ANK3, CREB5, FAH, FKBP7, AF009316, KLF4, ANKH, NTN4, THBS4, SCARA3, CYP3A5, MGP, AMN, CMAH, FN1, GABRA4, ABCC6, BE503823, SLC24A6; CXCL11, LOC113730, cig5, STAT1, GM2A, G1 P3, IFIT4, EIF5A, EPSTI1, MX1, IFI44L, IFI27, LOC129607, G1P2, IFIT1, FLJ39885, OAS2, FLJ20637, OAS1, MDA5, OAS3, LILRA2, TGFBRAP1, BST2, OASL, CEB1, HLA-DOA, KIS, GNB4, CLECSF12, AW262311, CALR, FPRL2, MAP3K2, FLJ20668, CYBB, SNX10, GRB2, GPR43, FLJ20035, C1QG, ARF6, IFI35, FLJ21069, BE674143; ZNF607, X07868, AU157716, CCDC3, CPSF1, AW029203, AK022838, CCNL2, C7orf10, SEC24D, AFG3L1, TLE2, PCSK5, AA706701, .C18orf18, OSBPL6, BC042472, AUTS2, SOX4, PTPRD, AA572675, COL18A1, COL16A1, GPM6B, SCG2, TNC, TP53I11, COL12A1, AL832806, AA912540, MYST4, STEAP, ARNT2, AA854843, KIAA0484, AU147442, PKN3, SYNE1, DSPG3, AW903934, FBXO26, LOC200772, AF116709; SCRG1, DNAJC12, FNDC4, AK024204, NBL1, BF591996, DIO2 SGCD, T90703, SPOCK, CKLFSF4, AW162210, CDC42BPA, KIAA1171, FKSG17, N73742, ZNF515, PTPRS, CTDSPL, NRP2, EXTL2, CCND1, CALU, PVR, ATBF1, AFURS1, SYNPO, MYO7A, KIAA1450, SLC35B4; BCL2L11, MBNL2, TXNIP, RNASET2, DHX9, RUNX2; MSI2, FZD8, AW265065, DBC-1, ANGPTL2, FBI4, C14orf131, BF057799, SRPR, TTC3, COPA, PCDHGA11; CXCL13, AI823917, AA789123, CD209, IAN4L1, NOD3, KIAA1268, HRMT1L1, AI821404, COPG, FLJ33814, H963, TAP1, CUL5, AW504569, AA809449, CCL8, ZC3HDC1, SYNCOILIN, KIAA0090, GGA2, NAP1L, ETV6, AL037998, AK024712, AW612461, BM873997, AK000795, FLJ00133, FOXC1, MGC43690, BF590303, AI090764, BF221547, ID4, AW296081, PGF, RGS5, W80359, AF086069, FLJ32949, RAMP3, T87730, AI742685, GPR4, GRID1, FAM20A, FRMD4, FLJ13089, TBX2, RAPGEF2, BM353142; SLC1A4, LIG3, EMILIN2, ALDH1A1, TNFSF8, PB1, AA365670, YWHAZ, ZNF581, BAG4, CWF19L1, SLC15A2, PTPRC, STRN3, TCL1A, PKHD1L1, and CTLA4.

In a preferred embodiment, said microarray comprises probes suitable for hybridizing with at least 100 genes selected from the group comprising AI923633; ARPC4; ASH1L; AW008502; AW296081; AW612461; BAG2; BCL11B; BE676335; BG231773; BSG; BTBD1; C12orf30/FLJ13089; C14orf131; CALU; CCL5; CCND1; CD79A; CDC42BPA; CDC42SE1/SPEC1; CHD1; CHD9/FLJ12178; CPSF1; CST7; CTDSPL; DUT; EIF3EIP/EIF3S6IP; ETV6; EXTL2; FLJ21395 fis; FN1; FRMD4A; G3BP1; GPR4; GRID1; GYG1; HMGB1; IFI27; IFI6/G1P3; IFIT3/IFIT4; IL7R; JAK3; JOSD3/MGC5306; KIAA0090; KIAA1128; KIAA1377; KIAA1450; LCK; LOC129607; MYEOV2/LOC150678; NBL1; NELL2; OAS1; PARP12/ZC3HDC1; PDE5A; PGF; PHF21A/BHC80; PIK3C2A; PTBP1; PTEN; PTPN7; QKI; RAB8A; RALGPS2; RAP2A; RAPGEF2; RASGRP1; RBBP6; RGS5; RPL4; RSAD2/cig5; SFRS2B/SRP46; SFRS6; SIPA1L3; SLC15A2; SPARCL1; SUPT16H; SYNCOILIN; TARP /// TRGC2 /// TRGV9; TBC1D20/C20orf140; TBC1D24/KIAA1171; THRAP3; TI-227H /// TUG1; TLE2; TMEM43/MGC3222; TNFSF8; TOX; TRBC1 /// TRBV19; TSPAN3/TM4SF8; UCKL1/URKL1; WDR90/LOC197336; ZFHX3/ATBF1; T87730.

In a preferred embodiment, said microarray comprises probes suitable for hybridizing with at least 20 genes selected from the group comprising FLJ21395 fis, TTC3, NELL2, PTPN7, HLA-DOA, GPR171, COPA, KIAA0484, TRAT1, FNDC4, BCL11B, C14orf131, FKBP7, TBC1D24, AL037998, AI225238, LOC113730, AA789123, KIAA1377, AW504569.

The kit may comprise a plurality of reagents, each of which is capable of binding specifically with a nucleic acid or polypeptide corresponding to a gene for use in the invention. Suitable probe for binding with a nucleic acid (e. g. a genomic DNA, an mRNA, a spliced mRNA, a cDNA, or the like) include complementary nucleic acids. For example, the nucleic acid reagents may include oligonucleotides (labeled or non-labeled) fixed to a substrate, labeled oligonucleotides not bound with a substrate, pairs of PCR primers, molecular beacon probes, and the like.

In an embodiment, the kit comprises a nucleic acid probe that binds specifically with a gene nucleic acid or a fragment of the nucleic acid.

The kit may further comprise means for performing PCR reactions. The kit may further comprise media and solution suitable for taking a sample and for extracting RNA from said blood sample.

The kit can further comprise additional components for carrying out the method of the invention, such as RNA extraction solutions, purification column and buffers and the like. The kit of the invention can further include any additional reagents, reporter molecules, buffers, excipients, containers and/or devices as required described herein or known in the art, to practice a method of the invention.

The various components of the kit may be present in separate containers or certain compatible components may be pre-combined into a single container, as desired. In addition to the above components, the kits may further include instructions for practicing the present invention. These instructions may be present in the kits in a variety of forms, one or more of which may be present in the kit.

One form in which these instructions may be present is as printed information on a suitable medium or substrate, e. g., a piece or pieces of paper on which the information is printed, in the packaging of the kit, in a package insert, etc. Yet another means would be a computer readable medium, e. g., diskette, CD, etc., on which the information has been recorded. The invention also provides a computer-readable medium comprising one or more digitally encoded expression profiles, where each profile has one or more values representing the expression of said at least 100 genes that are differentially-expressed in a SLE, OA, RA, SA, or MIC disease. In an embodiment, said digitally encoded expression profiles are profiles of SLE, OA, RA, SA, or MIC reference samples. In some embodiments, the digitally-encoded expression profiles are comprised in a database. The kits according to the invention may comprise a microarray as defined above and a computer readable medium as described above. The array comprises a substrate having addresses, where each address has a probe that can specifically bind a nucleic acid molecule (by using an oligonucleotide array) or a peptide (by using a peptide array) that is differentially-expressed in at least one SLE, OA, RA, SA, or MIC class. The results are converted into a computer-readable medium that has digitally-encoded expression profiles containing values representing the expression level of a nucleic acid molecule detected by the array. Any other convenient means may be present in the kits.

The invention also provides for the storage and retrieval of a collection of data relating to SLE, OA, RA, SA, or MIC specific gene expression data of the present invention, including sequences and expression levels in a computer data storage apparatus.

The present invention discloses at least 20, at least 50, at least 100, at least 120, at least 150, at least 180, at least 200, at least 220, at least 240, at least 250, at least 260, or at least 264 genes (preferably up to 2059, more preferably up to 2050, more preferably up to 1500 genes, more preferably up to 1000 genes, yet more preferably up to 500 genes, yet more preferably up to 300 genes, yet more preferably up to 264, 260, 250, 240, 230, 220, 210; 200, 190, 180, 170, 160, 150, 140, 130, 120, 110, 100, 50, up to 20 genes or fragment thereof) described herein that are differentially-expressed in SLE, OA, RA, SA, and MIC classes. Accordingly, these genes and their gene products are potential therapeutic targets that are useful in methods of screening test compounds to identify therapeutic compounds for the treatment of rheumatic conditions. The differentially-expressed genes of the invention may be used in cell-based screening assays involving recombinant host cells expressing the differentially-expressed gene product. The recombinant host cells are then screened to identify compounds that can activate the product of the differentially-expressed gene (i.e. agonists) or inactivate the product of the differentially-expressed gene (i.e. antagonists).

The following Tables and examples are intended to illustrate and to substantiate the present invention.

Table 1 list about 2059 genes or fragments thereof used for classifying a rheumatic condition into defined clusters. These are suitable genes the expression profile of which can differentiate between SLE, MIC, SA, OA or RA. Accordingly, one can select at least 20, at least 100, at least 120, at least 150, at least 180, at least 200, at least 220, at least 240, at least 250, at least 260, or at least 264 or up to 2059 genes from this group to correctly classify the rheumatic condition in an individual as SLE, MIC, SA, OA or RA based on the expression profiles of the genes. The genes differentially expressed between the five conditions (n = 2059) were found by ANOVA. Several sets of genes out of this list can be used to establish the correct diagnosis of biological samples. The fact that several sets of genes can be used means that the differential gene expression between the disorders is strong. The classification cannot be made by looking at absolute values of the expression of some genes. The classification uses algorithms that look at the expression of all the genes in the list in order to attribute one of the diagnoses to the biological sample.

**Table 1**

| **probe set id** | **Gene name** | **GenBank** | **probe set id** | **Gene name** | **GenBank** |
|---|---|---|---|---|---|
| 219363_s_at | CGI-12 | NM_015942 | 202760_s_at | AKAP2 | NM_007203 |
| 201068_s_at | PSMC2 | NM_002803 | 225197_at | PRO0149 | W58461 |
| 222372_at | | AW971248 | 226341_at | | AI535737 |
| 228843_at | | AI824171 | 201804_x_at | CKAP1 | NM_001281 |
| 200774_at | C9orf10 | BE963765 | 212543_at | AIM1 | U83115 |
| 209292_at | ID4 | AL022726 | 231130_at | FKBP7 | AA683602 |
| 200953_s_3t | CCND2 | NM_001759 | 226366_at | SHPRH | AI828221 |
| 226702_at | LOC129607 | AI742057 | 209300_s_at | DKFZP566B183 | DKFZP566B183 protein |
| 226797_at | | AL133577 | 205992_s_at | IL15 | NM_000585 |
| 219848_s_at | ZNF432 | NM_014650 | 208956_x_at | DUT | U62891 |
| 225450_at | AMOTL1 | AI433831 | 35685_at | RING1 | AL031228 |
| 240120_at | | H72914 | 213398_s_at | C14orf124 | AI347090 |
| 210011_s_at | EWSR1 | BC000527 | 237444_at | | AI801902 |
| 212658_at | LHFPL2 | N66633 | 238081_at | FBI4 | AI694300 |
| 218310_at | RABGEF1 | NM_014504 | 226673_at | SH2D3C | AW665063 |
| 203335_at | PHYH | NM_006214 | 201829_at | NET1 | AW263232 |
| 209905_at | HOXA9 | AI246769 | 212463_at | CD59 | BE379006 |
| 58780_s_at | FLJ10357 | R42449 | 224060_s_at | CGI-30 | AF157319 |
| 1556385_at | | BQ028191 | 213031_s_at | FLJ14888 | AF161382 |
| 221569_at | AHI1 | AL136797 | 236974_at | CCNI | AA808018 |
| 217795_s_at | MGC3222 | W74580 | 222088_s_at | SLC2A14 | AA778684 |
| 225433_at | GTF2A1 | AU144104 | 212904_at | KIAA1185 | AB033011 |
| 235032_at | LOC134218 | BG112118 | 227239_at | DRCTNNB1A | AV734839 |
| 212567_s_at | MAP4 | AL523310 | 203854_at | IF | NM_000204 |
| 221767_x_at | HDLBP | AA515560 | 235577_at | KIAA0924 | AL036451 |
| 203153_at | IFIT1 | NM_001548 | 223494_at | MGEA5 | AF307332 |
| 206133_at | HSXIAPAF1 | NM_017523 | 224502_s_at | KIAA1191 | BC006316 |
| 201056_at | | N53479 | 227802_at | RIPX | AI075999 |
| 211275_s_at | GYG | AF087942 | 212418_at | ELF1 | M82882 |
| 200905_x_at | HLA-E | NM_005516 | 205055_at | ITGAE | NM_002208 |
| 239866_at | | AA705933 | 222400_s_at | SIPL | BC001467 |
| 202882_x_at | NOL7 | NM_016167 | 205579_at | HRH1 | NM_000861 |
| 212547_at | | N34842 | 202862_at | FAH | NM_000137 |
| 225108_at | AGPS | BF111719 | 213455_at | LOC92689 | W87466 |
| 221509_at | DENR | AB014731 | 244487_at | | BE620513 |
| 202967_at | GSTA4 | NM_001512 | 215418_at | PARVA | AK022316 |
| 218613_at | EFA6R | NM_018422 | 205283_at | FCMD | NM_006731 |
| 201658_at | ARL1 | AU151560 | 208270_s_at | RNPEP | NM_020216 |
| 201123_s_at | EIF5A | NM_001970 | 205542_at | STEAP | NM_012449 |
| 227034_at | Sep-10 | BE669553 | 238478_at | BNC2 | H97386 |
| 220925_at | MAK10 | NM_021929 | 230178_s_at | STATIP1 | BE672676 |
| 214988_s_at | SON | X63071 | 205112_at | PLCE1 | NM_016341 |
| 201101_s_at | BCLAF1 | BE963370 | 212718_at | PAPOLA | BF797555 |
| 214702_at | FN1 | AJ276395 | 226039_at | MGAT4A | AW006441 |
| 224776_at | DKFZp586M: 1819 | putative lysophosphatidic acid acyltransferase | 212599_at | AUTS2 | AK025298 |
| 208722_s_at | ANAPC5 | BC001081 | 202742_s_at | PRKACB | NM_002731 |
| 212802_s_at | DKFZP434C212 | DKFZP434C212 protein | 225095_at | SPTLC2 | W81119 |
| 216005_at | TNC | BF434846 | 227051_at | | AU157716 |
| 229253_at | CTMP | AI184512 | 228248_at | MGC39830 | W49629 |
| 205528_s_at | CBFA2T1 | X79990 | 200845_s_at | PRDX6 | NM_004905 |
| 210571_s_at | CMAH | AF074480 | 212211_at | ANKRD17 | AI986295 |
| 227531_at | | AI816849 | 201321_s_at | SMARCC2 | NM_003075 |
| 200602_at | APP | NM_000484 | 225383_at | ZNF275 | BF793625 |
| 1558015_s_at | ACTR2 | BU175810 | 200837_at | BCAP31 | NM_005745 |
| 220147_s_at | C12orf14 | NM_021238 | 202363_at | SPOCK | AF231124 |
| 225078_at | EMP2 | AV686514 | 1558143_a_at | BCL2L11 | AK027160 |
| 225479_at | LOC116064 | AL524175 | 203339_at | SLC25A12 | AI887457 |
| 225205_at | KIF3B | AI819734 | 208839_s_at | TIP120A | AL136810 |
| 231199_at | | AA701676 | 230387_at | | AL038450 |
| 228304_at | | BE674118 | 223716_s_at | ZNF265 | AF065391 |
| 219352_at | FLJ20637 | NM_017912 | 220079_s_at | USP48 | NM_018391 |
| 218671_s_at | ATPIF1 | NM_016311 | 214773_x_at | MGC3794 | AI983505 |
| 201272_at | AKR1B1 | NM_001628 | 218269_at | RNASE3L | NM_013235 |
| 204774_at | EVI2A | NM_014210 | 221731_x_at | CSPG2 | BF218922 |
| 227444_at | MGC40053 | AW519141 | 244153_at | | AA521438 |
| 202317_s_at | UBE4B | NM_006048 | 40569_at | ZNF42 | M58297 |
| 218019_s_at | C21orf97 | NM_021941 | 205226_at | PDGFRL | NM_006207 |
| 219988_s_at | FLJ10597 | NM_018150 | 213940_s_at | FNBP1 | AU145053 |
| 219514_at | ANGPTL2 | NM_012098 | 202200_s_at | SRPK1 | NM_003137 |
| 218424_s_at | TSAP6 | NM_018234 | 203923_s_at | CYBB | NM_000397 |
| 1554062_at | XG | AF380356 | 205412_at | ACAT1 | NM_000019 |
| 212169_at | FKBP9 | AL050187 | 220966_x_at | ARPC5L | NM_030978 |
| 209656_s_at | TM4SF10 | AL136550 | 217842_at | LUC7L2 | NM_016019 |
| 1555579_s_at | PTPRM | BC029442 | 202220_at | KIAA0907 | NM_014949 |
| 220512_at | DLC1 | NM_024767 | 232914_s_at | SYTL2 | AB046817 |
| 202270_at | GBP1 | NM_002053 | 225665_at | ZAK | AI129320 |
| 1558675_s_at | SDCCAG1 | AV724508 | 230438_at | TBX15 | AI039005 |
| 226653_at | MARK1 | AB040910 | 200662_s_at | TOMM20 | NM_014765 |
| 203566_s_at | AGL | NM_000645 | 201212_at | LGMN | D55696 |
| 201011_at | RPN1 | NM_002950 | 224824_at | FAM36A | AV694386 |
| 210840_s_at | IQGAP1 | D29640 | 225870_s_at | TRAPPC5 | BF569208 |
| 201416_at | SOX4 | BG528420 | 225115_at | HIPK2 | BF529628 |
| 243462_s_at | | Transcribed sequences | 226821_at | Rif1 | R41296 |
| 204258_at | CHD1 | NM_001270 | 211615_s_at | LRPPRC | M92439 |
| 202251_at | PRPF3 | NM_004698 | 213296_at | RER1 | BF339133 |
| 214870_x_at | LOC339047 | AC002045 | 213097_s_at | ZRF1 | AI338837 |
| 201449_at | TIA1 | AL567227 | 209199_s_at | MEF2C | N22468 |
| 206385_s_at | ANK3 | NM_020987 | 201217_x_at | RPL3 | NM_000967 |
| 235079_at | | AW265065 | 201299_s_at | C2orf6 | NM_018221 |
| 1556427_s_at | LOC221091 | AL834319 | 223144_s_at | C6orf166 | BC000764 |
| 212673_at | METAP1 | D42084 | 202119_s_at | CPNE3 | NM_003909 |
| 218404_at | SNX10 | NM_013322 | 226406_at | C18orf25 | AI823360 |
| 225677_at | BCAP29 | AW152589 | 213213_at | DATF1 | AL035669 |
| 224959_at | SLC26A2 | AI718385 | 224606_at | | BG250721 |
| 202596_at | ENSA | BC000436 | 210879_s_at | GAF1 | AF334812 |
| 227646_at | EBF | BG435302 | 212294_at | GNG12 | BG111761 |
| 218047_at | OSBPL9 | NM_024586 | 229018_at | FLJ22789 | AI310001 |
| 212865_s_at | COL14A1 | BF449063 | 224695_at | C2orf29 | AK024221 |
| 222679_s_at | RP42 | AW468880 | 223014_at | UBE2R2 | BC004862 |
| 243016_at | TYMS | AW271958 | 212987_at | FBXO9 | AL031178 |
| 235443_at | | BG284827 | 36994_at | ATP6V0C | M62762 |
| 217922_at | MAN1A2 | AL157902 | 222418_s_at | MGC3222 | AA115485 |
| 224761_at | GNA13 | AI928136 | 205707_at | IL17R | NM_014339 |
| 226981 _at | MLL | AW002079 | 205618_at | PRRG1 | NM_000950 |
| 229120_s_at | SPEC1 | BG150636 | 226050_at | C13orf11 | AL576117 |
| 208662_s_at | TTC3 | AI885338 | 200004_at | EIF4G2 | NM_001418 |
| 213675_at | | W61005 | 228822_s_at | USP16 | AI435036 |
| 202432_at | PPP3CB | NM_021132 | 217893_s_at | FLJ12666 | NM_024595 |
| 225272_at | SAT2 | AA128261 | 209167_at | GPM6B | AI419030 |
| 205831_at | CD2 | NM_001767 | 213218_at | ZNF187 | AV705032 |
| 226604_at | SMILE | AA418403 | 213493_at | FLJ00133 | BF509657 |
| 228617_at | HSXIAPAF1 | AA142842 | 209417_s_at | IFI35 | BC001356 |
| 225147_at | PSCD3 | AL521959 | 201010_s_at | TXNIP | NM_006472 |
| 201894_s_at | DCN | NM_001920 | 224694_at | ANTXR1 | AF279145 |
| 202043_s_at | SMS | NM_004595 | 63009_at | FLJ10539 | AI188402 |
| 212848_s_at | C9orf3 | BG036668 | 209224_s_at | NDUFA2 | BC003674 |
| 203216_s_at | MYO6 | NM_004999 | 213700_s_at | | H.sapiens (xs157) mRNA, 315bp |
| 223501_at | TNFSF13B | AW151360 | 202776_at | ERBP | NM_014597 |
| 1553185_at | RAB45 | NM_152573 | 203894_at | TUBG2 | NM_016437 |
| 228153_at | IBRDC2 | AI953847 | 200971_s_at | SERP1 | NM_014445 |
| 206373_at | ZIC1 | NM_003412 | 203508_at | TNFRSF1B | NMI_001066 |
| 209702_at | FTO | U79260 | 227471_at | HACE1 | AB037741 |
| 202086_at | MX1 | NMI_002462 | 225381_at | | AW162210 |
| 226420_at | EVI1 | BG261252 | 226633_at | RAB8B | AI807023 |
| 210473_s_at | GPR125 | M37712 | 225125_at | LOC93380 | BF978280 |
| 1558236_at | | BC014318 | 225352_at | TLOC1 | AI763287 |
| 221695_s_at | MAP3K2 | AF239798 | 213287_s_at | KRT10 | X14487 |
| 212880_at | WDR7 | AB011113 | 224852_at | TTC17 | BE964325 |
| 230379_x_at | PRO1853 | BF439153 | 226581_at | ZFYVE20 | AA524034 |
| 205407_at | RECK | NM_021111 | 221729_at | COL5A2 | AL575735 |
| 222662_at | LOC286044 | W60806 | 218901_at | PLSCR4 | NM_020353 |
| 207738_s_at | NCKAP1 | NM_013436 | 213543_at | SGCD | AA570453 |
| 204538_x_at | NPIP | NM_006985 | 230435_at | FLJ30851 | BF108666 |
| 228084_at | | AI767751 | 227526_at | | AU151222 |
| 210231_x_at | SET | D45198 | 205728_at | SH2D1A | AL022718 |
| 218024_at | BRP44L | NM_016098 | 213564_x_at | LDHB | BE042354 |
| 204205_at | APOBEC3G | NM_021822 | 202644_s_at | TNFAIP3 | NM_006290 |
| 202594_at | LEPROTL1 | NM_015344 | 218157_x_at | SPEC1 | NM_020239 |
| 219549_s_at | RTN3 | NM_006054 | 232882_at | | AA079839 |
| 212532_s_at | FLJ30656 | AW873564 | 226326_at | RNF159 | AI798098 |
| 210097_s_at | NOL7 | AF130102 | 201008_s_at | TXNIP | AA812232 |
| 216960_s_at | ZNF133 | AL049646 | 219929_s_at | ZFYVE21 | NM_024071 |
| 225318_at | DDHD2 | AW292751 | 225929_s_at | KIAA1554 | AA233374 |
| 228255_at | ALS2CR4 | AU150140 | 233341_s_at | POLR1B | AK025574 |
| 226876_at | MGC45871 | AI961778 | 212398_at | RDX | AI057093 |
| 209537_at | EXTL2 | AF000416 | 212057_at | KIAA0182 | AA206161 |
| 205475_at | SCRG1 | NM_007281 | 235201_at | | AW167727 |
| 227093_at | USP36 | AU152298 | 235561_at | | T16544 |
| 239071_at | | AI972451 | 217966_s_at | C1orf24 | NM_022083 |
| 218515_at | C21orf66 | NM_016631 | 208703_s_at | APLP2 | BG427393 |
| 204064_at | THOC1 | NM_005131 | 218389_s_at | APH-1A | NM_016022 |
| 221989_at | RPL10 | AW057781 | 235444_at | FOXP1 | AI417897 |
| 205583_s_at | FLJ23018 | NM_024810 | 203887_s_at | THBD | NM_000361 |
| 207023_x_at | KRT10 | NM_000421 | 221677_s_at | DONSON | AF232674 |
| 202106_at | GOLGA3 | NM_005895 | 238020_at | PSMC2 | BG166796 |
| 205525_at | CALD1 | NM_018495 | 232156_at | CMYA5 | AK024921 |
| 203552_at | MAP4K5 | AW298170 | 218757_s_at | UPF3B | NM_023010 |
| 201124_at | ITGB5 | AL048423 | 203079_s_at | CUL2 | NM_003591 |
| 232541_at | | AK000106 | 201020_at | YWHAH | NM_003405 |
| 219248_at | C2orf8 | NM_025264 | 208778_s_at | TCP1 | BC000665 |
| 229194_at | RNF159 | AL045882 | 223093_at | ANKH | T99215 |
| 225419_at | C7orf11 | AI869704 | 223894_s_at | FTS | BC001134 |
| 223287_s_at | FOXP1 | AF146696 | 212382_at | TCF4 | BF433429 |
| 202971_s_at | DYRK2 | NM_006482 | 200715_x_at | RPL13A | BC000514 |
| 203285_s_at | HS2ST1 | NM_012262 | 224773_at | NAV1 | N57538 |
| 212837_at | KIAA0157 | D63877 | 232235_at | NCAG1 | AK021539 |
| 207480_s_at | MEIS2 | NM_020149 | 200941_at | HSBP1 | AK026575 |
| 203408_s_at | SATB1 | NM_002971 | 1557543_at | | AL832672 |
| 204949_at | ICAM3 | NM_002162 | 200685_at | SFRS11 | AU146237 |
| 226811_at | FLJ20202 | AL046017 | 212423_at | C10orf56 | AK024784 |
| 202930_s_at | SUCLA2 | NM_003850 | 212364_at | MYO1B | BF432550 |
| 218456_at | C1QDC1 | NM_023925 | 233647_s_at | CDADC1 | AL138875 |
| 218511_s_at | PNPO | NM_018129 | 221725_at | WASF2 | AI962978 |
| 200630_x_at | SET | AV702810 | 227547_at | | AA824321 |
| 212387_at | TCF4 | BG495771 | 224673_at | LENG8 | AI613244 |
| 225315_at | MRPL21 | BF344406 | 228661_s_at | | CDNA clone IMAGE:4821863 , partial cds |
| 222791_at | FLJ11220 | AK022166 | 211445_x_at | FKSG17 | AF315951 |
| 230466_s_at | | Mesenchymal stem cell protein DSC96 mRNA, partial cds | 201061_s_at | STOM | M81635 |
| 205483_s_at | G1P2 | NMI_005101 | 244871_s_at | USP32 | AW268357 |
| 238653_at | LRIG2 | BF967997 | 208680_at | PRDX1 | L19184 |
| 225610_at | UHRF2 | BF511410 | 224586_x_at | PC4 | BE784583 |
| 223322_at | RASSF5 | BC004270 | 218289_s_at | UBE1DC1 | NMI_024818 |
| 211352_s_at | NCOA3 | U80737 | 213413_at | SBLF | BG434174 |
| 223160_s_at | KIAA2010 | AK024297 | 219892_at | TM6SF1 | NM_023003 |
| 218499_at | MST4 | NM_016542 | 224690_at | C20orf108 | BG432350 |
| 206283_s_at | TAL1 | NM_003189 | 208445_s_at | BAZ1B | NM_023005 |
| 224579_at | SLC38A1 | BF247552 | 218986_s_at | FLJ20035 | NM_017631 |
| 225685_at | | AI801777 | 226525_at | | N51102 |
| 203028_s_at | CYBA | NM_000101 | 226677_at | ZNF521 | AF141339 |
| 217722_s_at | NEUGRIN | NM_016645 | 202269_x_at | GBP1 | BC002666 |
| 208920_at | SRI | AV752215 | 228334_x_at | KIAA1712 | AI633734 |
| 214730_s_at | GLG1 | AK025457 | 243966_at | | AA778095 |
| 212587_s_at | PTPRC | AI809341 | 224591_at | HP1-BP74 | AK023129 |
| 239300_at | | AI632214 | 214434_at | HSPA12A | AB007877 |
| 210371_s_at | RBBP4 | BC003092 | 227880_s_at | FAM11A | AW300965 |
| 222151_s_at | FLJ13386 | AK023738 | 208405_s_at | CD164 | NM_006016 |
| 219317_at | POLI | NM_007195 | 240539_at | | AI684551 |
| 202897_at | PTPNS1 | AB023430 | 235759_at | EFCBP1 | A1095542 |
| 226037_s_at | TAF9L | AL049589 | 227256_at | USP31 | BG289456 |
| 200943_at | HMGN1 | NM_004965 | 223315_at | NTN4 | AF278532 |
| 213362_at | PTPRD | N73931 | 202564_x_at | ARL2 | NM_001667 |
| 220939_s_at | DPP8 | NM_017743 | 226656_at | | AW024741 |
| 218733_at | FLJ10546 | NM_018133 | 202833_s_at | SERPINA1 | NM_000295 |
| 202409_at | | X07868 | 211672_s_at | ARPC4 | AF019888 |
| 226098_at | KIAA1374 | AB037795 | 202404_s_at | COL1A2 | NM_000089 |
| 214323_s_at | UPF3A | N36842 | 224564_s_at | RTN3 | BE544689 |
| 220044_x_at | LUC7A | NM_016424 | 1569472_s_at | TTC3 | BC026260 |
| 212615_at | FLJ12178 | AI742305 | 243046_at | UBE2D3 | BF679700 |
| 212692_s_at | LRBA | W60686 | 229078_s_at | KIAA1704 | AI073486 |
| 211458_s_at | GABARAPL1 | AF180519 | 234982_at | KIAA2024 | BF577193 |
| 228287_at | ING5 | BG054893 | 201934_at | PRO2730 | N92524 |
| 203301_s_at | DMTF1 | NM_021145 | 224617_at | ROD1 | AI735576 |
| 224676_at | HNLF | AI472339 | 213001_at | ANGPTL2 | AF007150 |
| 229390_at | | AV734646 | 213331_s_at | NEK1 | AV700007 |
| 211073_x_at | RPL3 | BC006483 | 222434_at | ENAH | AI963713 |
| 225923_at | VAPB | AW291083 | 224451_x_at | ARHGAP9 | BC006107 |
| 205255_x_at | TCF7 | NM_003202 | 203882_at | ISGF3G | NM_006084 |
| 225339_at | SPAG9 | BG290577 | 223058_at | C10orf45 | AL136885 |
| 209314_s_at | HBS1L | AK024258 | 214394_x_at | EEF1D | AI613383 |
| 224945_at | BTBD7 | AI935657 | 221841_s_at | KLF4 | BF514079 |
| 211036_x_at | ANAPC5 | BC006301 | 1560526_at | PR47 | 8Q880034 |
| 203063_at | PPM1F | NM_014634 | 222422_s_at | NDFIP1 | AW167859 |
| 215023_s_at | PEX1 | AC000064 | 228071_at | hlAN7 | AA858297 |
| 232852_at | | AK024979 | 229366_at | LOC51185 | BG149765 |
| 209356_x_at | EFEMP2 | AB030655 | 204429_s_at | SLC2A5 | BE560461 |
| 225186_at | RAPH1 | BF114947 | 229391_s_at | | chromosome 6 open reading frame 187 |
| 212504_at | KIAA0934 | N31807 | 232617_at | CTSS | AK024855 |
| 200957_s_at | SSRP1 | NM_003146 | 205247_at | NOTCH4 | AI743713 |
| 223502_s_at | TNFSF13B | AF134715 | 206548_at | FLJ23556 | NM_024880 |
| 235529_x_at | FLJ46365 | BF437747 | 51200_at | FLJ20850 | AI744084 |
| 234111_at | | AK026716 | 202838_at | FUCA1 | NM_000147 |
| 203787_at | SSBP2 | NM_012446 | 232323_s_at | TTC17 | AK026217 |
| 215209_at | SEC24D | AU143984 | 216100_s_at | LAP1B | BG289527 |
| 229228_at | | AI819043 | 207941_s_at | RNPC2 | NM_004902 |
| 224325_at | FZD8 | AB043703 | 211761_s_at | SIP | BC005975 |
| 214132_at | ATP5C1 | BG232034 | 214663_at | DustyPK | AB007941 |
| 217781_s_at | ZFP106 | NM_022473 | 224748_at | HAN11 | AK025925 |
| 209744_x_at | ITCH | AB056663 | 203781_at | MRPL33 | NM_004891 |
| 209520_s_at | NCBP1 | BC001450 | 205807_s_at | TUFT1 | NM_020127 |
| 200782_at | ANXA5 | NMI_001154 | 224786_at | SCOC | AL133580 |
| 224616_at | DNCLI2 | BG110975 | 211429_s_at | | PRO2275 mRNA, complete cds |
| 204035_at | SCG2 | NM_003469 | 228040_at | MGC21881 | AW294192 |
| 205100_at | GFPT2 | NM_005110 | 220155_s_at | BRD9 | NM_023924 |
| 212399_s_at | KIAA0121 | D50911 | 237006_at | | AA703523 |
| 211528_x_at | HLA-C | M90685 | 32042_at | COVA1 | S72904 |
| 217837_s_at | VPS24 | NM_016079 | 215978_x_at | LOC152719 | AK021514 |
| 212838_at | DNMBP | AB023227 | 208869_s_at | GABARAPL1 | AF087847 |
| 216526_x_at | HLA-C | AK024836 | 223298_s_at | NT5C3 | AF312735 |
| 225056_at | SIPA1L2 | AB037810 | 227810_at | ZNF558 | AW119060 |
| 219802_at | FLJ22028 | NM_024854 | 227322_s_at | BCCIP | BE464077 |
| 213846_at | COX7C | AA382702 | 221819_at | RAB35 | BF791960 |
| 200955_at | IMMT | NM_006839 | 200670_at | XBP1 | NM_005080 |
| 213588_x_at | RPL14 | AA838274 | 202743_at | PIK3R3 | BE622627 |
| 1553960_at | C20orf161 | CA447177 | 212992_at | C14orf78 | AI935123 |
| 205089_at | ZNF7 | NM_003416 | 213603_s_at | RAC2 | BE138888 |
| 227069_at | | AA806989 | 232511_at | | AK022838 |
| 203044_at | CHSY1 | NM_014918 | 1555872_a_at | MGC21881 | BC019880 |
| 201458_s_at | BUB3 | NM_004725 | 35776_at | ITSN1 | AF064243 |
| 212638_s_at | WWP1 | BF131791 | 227594_at | ZNF258 | AI743551 |
| 200937_s_at | RPL5 | NM_000969 | 225441_x_at | MGC14151 | AA828224 |
| 201125_s_at | ITGB5 | NM_002213 | 202020_s_at | LANCL1 | NM_006055 |
| 203775_at | SLC25A13 | NM_014251 | 219737_s_at | PCDH9 | AI524125 |
| 204747_at | IFIT4 | NM_001549 | 224936_at | EIF2S3 | BE252813 |
| 200900_s_at | M6PR | AI583537 | 203255_at | FBXO11 | NM_018693 |
| 222430_s_at | HGRG8 | BC002559 | 224701_at | KIAA1268 | AA056548 |
| 226452_at | PDK1 | AU146532 | 226542_at | RNF24 | AI300571 |
| 200899_s_at | MGEA5 | NM_012215 | 225489_at | TMEM18 | AI720705 |
| 211676_s_at | IFNGR1 | AF056979 | 222182_s_at | CNOT2 | BG105204 |
| 226686_at | | AI188518 | 213397_x_at | RNASE4 | AI761728 |
| 214007_s_at | PTK9 | AW665024 | 210137_s_at | DCTD | BC001286 |
| 235078_at | | AI393725 | 201314_at | STK25 | NM_006374 |
| 203865_s_at | ADARB1 | NM_015833 | 217526_at | FLJ14639 | AI478300 |
| 200048_s_at | JTB | NM_006694 | 221529_s_at | PLVAP | AF326591 |
| 243184_at | | AW173166 | 208625_s_at | EIF4G1 | AF104913 |
| 244356_at | | AL079909 | 233437_at | GABRA4 | AF238869 |
| 221740_x_at | KIAA0563 | AI140364 | 227693_at | WDR20 | AI092930 |
| 208857_s_at | PCMT1 | M93008 | 218517_at | PHF17 | NM_024900 |
| 228751_at | CLK4 | AW975057 | 218277_s_at | DHX40 | NM_024612 |
| 1555832_s_at | | MRNA; cDNA DKFZp564C206 3 (from clone DKFZp564C206 3) | 227905_s_at | AZ2 | BF000175 |
| 209550_at | NDN | U35139 | 227454_at | KIAA1361 | AB037782 |
| 224764_at | ARHGAP21 | AB037845 | 229111_at | MASP2 | AA033699 |
| 225120_at | PURB | N25931 | 202813_at | TARBP1 | NM_005646 |
| 228567_at | | BG109230 | 203766_s_at | LMOD1 | NM_012134 |
| 202745_at | USP8 | NM_005154 | 235390_at | FLJ36754 | AA398321 |
| 233449_at | | AU143940 | 204312_x_at | CREB1 | AI655737 |
| 203804_s_at | OA48-18 | NM_006107 | 233480_at | | AK026869 |
| 217506_at | | H49382 | 207467_x_at | CAST | NM_001750 |
| 202491_s_at | IKBKAP | NM_003640 | 203450_at | C22orf2 | NM_015373 |
| 226327_at | ZNF507 | N64593 | 226626_at | THOC2 | AL133117 |
| 229544_at | | AI690169 | 201546_at | TRIP12 | NM_004238 |
| 218285_s_at | DHRS6 | NM_020139 | 218025_s_at | PECI | NM_006117 |
| 210568_s_at | RECQL | BC001052 | 225276_at | GSPT1 | AA143579 |
| 232113_at | | N90870 | 226364_at | HIP1 | AU145049 |
| 227978_s_at | MGC45594 | BF591611 | 208615_s_at | PTP4A2 | BF795101 |
| 212409_s_at | LAP1B | AK021613 | 222548_s_at | MAP4K4 | AL561281 |
| 203478_at | NDUFC1 | NM_002494 | 227607_at | AMSH-LP | AI638611 |
| 204279_at | PSMB9 | NM_002800 | 242500_at | | T87730 |
| 223203_at | PRO0659 | BC000867 | 201608_s_at | PWP1 | NM_007062 |
| 208770_s_at | EIF4EBP2 | BC005057 | 48580_at | CXXC1 | U55777 |
| 236229_at | | AW014345 | 244015_at | | AA704163 |
| 235509_at | MGC40214 | AV662196 | 242358_at | | AW024656 |
| 214801_at | | W88821 | 201785_at | RNASE1 | NM_002933 |
| 229017_s_at | DustyPK | N31717 | 208653_s_at | CD164 | AF263279 |
| 201070_x_at | SF3B1 | AI739389 | 227822_at | ZNF605 | AI341321 |
| 201837_s_at | STAF65(gamma ) | SPTF-associated factor 65 gamma | 218543_s_at | ZC3HDC1 | NM_022750 |
| 229303_at | SF3B1 | AI018793 | 214259_s_at | AKR7A2 | AI144075 |
| 239979_at | | BE645480 | 213341_at | FEM1C | AI862658 |
| 203637_s_at | MID1 | NM_000381 | 208996_s_at | POLR2C | BC000409 |
| 217028_at | CXCR4 | AJ224869 | 212286_at | ANKRD12 | AW572909 |
| 201415_at | GSS | NM_000178 | 204524_at | PDPK1 | NM_002613 |
| 226527_at | KIAA0460 | AI569785 | 202795_x_at | HRIHFB2122 | NM_007032 |
| 212826_s_at | SLC25A6 | AI961224 | 228455_at | | AI092824 |
| 41644_at | SASH1 | AB018333 | 217933_s_at | LAP3 | NM_015907 |
| 209762_x_at | SP110 | AF280094 | 224639_at | SPPL3 | AI928466 |
| 225931_s_at | KIAA1554 | AI954660 | 243570_at | KIAA0102 | AA921960 |
| 240188_at | | AW268884 | 201310_s_at | C5orf13 | NM_004772 |
| 235410_at | NPHP3 | BG034966 | 204671_s_at | ANKRD6 | BE677131 |
| 201544_x_at | PABPN1 | BF675004 | 217790_s_at | SSR3 | NM_007107 |
| 210589_s_at | GBA | D13287 | 226910_at | COMMD2 | AW008502 |
| 225160_x_at | MGC5370 | AI952357 | 213939_s_at | RIPX | AI871641 |
| 223843_at | SCARA3 | AB007830 | 232843_s_at | DOCK8 | AL161725 |
| 242482_at | PRKAR1A | AI682905 | 220027_s_at | RASIP1 | NM_017805 |
| 215452_x_at | SUMO2 | AL031133 | 219485_s_at | PSMD10 | NM_002814 |
| 211921_x_at | PTMA | AF348514 | 1567214_a_at | PNN | U59479 |
| 217797_at | Ufc1 | NM_016406 | 203336_s_at | ITGB1BP1 | AL548363 |
| 211376_s_at | C10orf86 | BC005212 | 223996_s_at | MRPL30 | AF151083 |
| 209944_at | ZNF410 | BC000330 | 213049_at | GARNL1 | BG436400 |
| 203367_at | DUSP14 | NM_007026 | 235635_at | | N50119 |
| 222787_s_at | FLJ11273 | AV705186 | 213179_at | RQCD1 | BG289914 |
| 243521_at | | AW590862 | 201901_s_at | YY1 | Z14077 |
| 228841_at | LOC90624 | AW299250 | 216037_x_at | TCF7L2 | AA664011 |
| 226247_at | PLEKHA1 | AI346026 | 208896_at | DDX18 | X98743 |
| 201558_at | RAE1 | NM_003610 | 209578_s_at | C21orf80 | BC000626 |
| 208866_at | CSNK1A1 | BF510713 | 212454_x_at | HNRPDL | AI762552 |
| 202466_at | POLS | NM_006999 | 221776_s_at | BRD7 | AI885109 |
| 201998_at | SIAT1 | AI743792 | 1554241_at | COCH | BC007230 |
| 220038_at | SGKL | NM_013257 | 202273_at | PDGFRB | NM_002609 |
| 202552_s_at | CRIM1 | NM_016441 | 214356_s_at | KIAA0368 | AI272899 |
| 202962_at | KIF13B | NM_015254 | 218494_s_at | SLC2A4RG | NM_020062 |
| 212042_x_at | RPL7 | BG389744 | 239404_at | | BF840360 |
| 224777_s_at | PAFAH1B2 | BG386322 | 221986_s_at | DRE1 | AW006750 |
| 218694 at | ARMCX1 | NM_016608 | 223604_at | GARNL3 | AL136573 |
| 218510_x_at | FLJ20152 | AI816291 | 217817_at | ARPC4 | BE891920 |
| 244533_at | | BE617483 | 208885_at | LCP1 | J02923 |
| 225202_at | RHOBTB3 | BE620739 | 212234_at | ASXL1 | AL034550 |
| 213156_at | | BG251521 | 224674_at | TTYH3 | AI934753 |
| 222789_at | FLJ11220 | R45958 | 232750_at | AU158570 | AU158570 |
| 209031_at | IGSF4 | AL519710 | 201962_s_at | RNF41 | NM_005785 |
| 202599_s_at | NRIP1 | NM_003489 | 225839_at | LOC155435 | AW290882 |
| 200757_s_at | CALU | NM_001219 | 1555878_at | RPS24 | AK094613 |
| 204972_at | OAS2 | NM_016817 | 216941_s_at | TAF1B | AK026521 |
| 226635_at | | BG170478 | 235003_at | KIS | AI249980 |
| 221830_at | RAP2A | AI302106 | 211085_s_at | STK4 | Z25430 |
| 224871_at | LOC127262 | AK025464 | 235458_at | HAVCR2 | AW025572 |
| 215936_s_at | KIAA1033 | AK001657 | 204923_at | CXorf9 | AL023653 |
| 225017_at | FLJ12892 | AK022954 | 227344_at | ZNFN1A1 | AI439886 |
| 218016_s_at | POLR3E | NM_018119 | 239798_at | | AI825068 |
| 242366_at | | AI921844 | 227293_at | LNX | AI264003 |
| 1552486_s_at | LACTB | NM_171846 | 209256_s_at | KIAA0265 | AF277177 |
| 225363_at | PTEN | AK024986 | 226474_at | NOD27 | AA005023 |
| 201987_at | THRAP1 | AI984051 | 203833_s_at | TGOLN2 | BF061845 |
| 221842_s_at | ZNF131 | BE972394 | 209606_at | PSCDBP | L06633 |
| 228062_at | NAP1L5 | AW025330 | 235341_at | DNAJC3 | AL119957 |
| 229504_at | RAB23 | AI810826 | 208072_s_at | DGKD | NM_003648 |
| 201967_at | RBM6 | NM_005777 | 1559052_s_at | PAK2 | U25975 |
| 225116_at | HIPK2 | AW300045 | 241395_at | NIT1 | AL572553 |
| 239231_at | | BE464819 | 209195_s_at | ADCY6 | AF250226 |
| 208296_x_at | TNFAIP8 | NM_014350 | 224913_s_at | TIMM50 | AA877820 |
| 202794_at | INPP1 | NM_002194 | 205560_at | PCSK5 | NM_006200 |
| 202637_s_at | ICAM1 | AI608725 | 205552_s_at | OAS1 | NM_002534 |
| 224836_at | C20orf110 | AL109824 | 238787_at | | AA988769 |
| 1569594_a_at | SDCCAG1 | BC006001 | 201545_s_at | PABPN1 | NM_004643 |
| 201030_x_at | LDHB | NM_002300 | 232746_at | CMKOR1 | BE552368 |
| 203629_s_at | COG5 | AU152134 | 205147_x_at | NCF4 | NM_000631 |
| 225128_at | MGC33424 | AL548941 | 238341_at | | BF677084 |
| 228716_at | THRB | BG494007 | 225353_s_at | C1QG | AI184968 |
| 202944_at | NAGA | NM_000262 | 202723_s_at | FOXO1A | AW117498 |
| 228760_at | SRP46 | AV725947 | 222868_s_at | IL18BP | AI521549 |
| 226895_at | NFIC | AW134798 | 202643_s_at | TNFAIP3 | AI738896 |
| 218240_at | NKIRAS2 | NM_017595 | 224631_at | ZFP91 | AA758013 |
| 200098_s_at | ANAPC5 | T33068 | 206991_s_at | CCR5 | NM_000579 |
| 225035_x_at | FLJ25222 | BG258971 | 215253_s_at | DSCR1 | AL049369 |
| 219078_at | GPATC2 | NM_018040 | 224804_s_at | C15orf17 | AU152410 |
| 209289_at | NFIB | AI700518 | 214995_s_at | APOBEC3G | BF508948 |
| 229004_at | | AI970797 | 211796_s_at | | Homo sapiens T cell receptor beta chain (TCRBV13S1-TCRBJ2S1) mRNA, complete cds. |
| 200638_s_at | YWHAZ | BC003623 | 214181_x_at | LST1 | AI735692 |
| 215794_x_at | GLUD1 | AC006144 | 204139_x_at | ZNF42 | NM_003422 |
| 201290_at | SPC18 | NM_014300 | 214833_at | KIAA0792 | AB007958 |
| 213348_at | CDKN1C | N33167 | 1558699_a_at | FLJ22313 | BG249246 |
| 235276_at | EPSTI1 | AA781795 | 207655_s_at | BLNK | NM_013314 |
| 213116_at | NEK3 | AI191920 | 219593_at | SLC15A3 | NM_016582 |
| 1555201_a_at | C6orf96 | BC012081 | 37425_g_at | C6orf18 | AB029343 |
| 212256_at | GALNT10 | BE906572 | 225750_at | ERO1L | BE966748 |
| 214016_s_at | SFPQ | AL558875 | 205522_at | HOXD4 | NM_014621 |
| 223245_at | STRBP | AK024285 | 223640_at | HCST | AF285447 |
| 213080_x_at | RPL5 | BF214492 | 222581_at | XPR1 | AF089744 |
| 243591_at | | AI887749 | 244650_at | | AA581439 |
| 225589_at | SH3MD2 | AB040927 | 235902_at | | AI090764 |
| 212781_at | RBBP6 | AK026954 | 235349_at | FLJ32954 | AI261321 |
| 212493_s_at | HYPB | AI761110 | 215633_x_at | LST1 | AV713720 |
| 213624_at | SMPDL3A | AA873600 | 219511_s_at | SNCAIP | NM_005460 |
| 1554703_at | ARHGEF10 | BC040474 | 206805_at | SEMA3A | NM_006080 |
| 217904_s_at | BACE1 | NM_012104 | 224828_at | CPEB4 | AV704132 |
| 232935_at | | AA569225 | 219627_at | FLJ12700 | NM_024910 |
| 232794_at | LOC153682 | AL137383 | 227908_at | KIAA1171 | BG236006 |
| 242323_at | | AV714268 | 227039_at | AKAP13 | AI674926 |
| 227063_at | MGC40107 | BF975929 | 207629_s_at | ARHGEF2 | NM_004723 |
| 201585_s_at | SFPQ | BG035151 | 202825_at | SLC25A4 | NM_001151 |
| 219130_at | FLJ10287 | NM_019083 | 215743_at | NMT2 | AL134489 |
| 226742_at | | AI890133 | 210629_x_at | LST1 | AF000425 |
| 226385_s_at | C7orf30 | BG397444 | 215248_at | GRB10 | AU145003 |
| 215698_at | JARID1A | AF007135 | 204436_at | pp9099 | NMI_025201 |
| 213113_s_at | SLC43A3 | AI630178 | 209879_at | SELPLG | AI741056 |
| 214670_at | ZNF36 | AA653300 | 220266_s_at | KLF4 | NM_004235 |
| 242837_at | | AI435248 | 221965_at | MPHOSPH9 | AI990326 |
| 232683_s_at | LOC56965 | AL122091 | 219681_s_at | RCP | NMI_025151 |
| 235849_at | MGC45780 | BE787752 | 227148_at | KIAA2028 | AI913749 |
| 209290_s_at | NFIB | BC001283 | 227458_at | PDCD1LG1 | AI608902 |
| 219561_at | COPZ2 | NM_016429 | 222820_at | KIAA1582 | AW005818 |
| 209121_x_at | NR2F2 | M64497 | 220577_at | FLJ13373 | NM_025006 |
| 203762_s_at | D2LIC | NM_016008 | 205371_s_at | DBT | M27093 |
| 213844_at | HOXA5 | NM_019102 | 235440_at | FLJ39441 | BE780878 |
| 201334_s_at | ARHGEF12 | AB002380 | 230533_at | PRKCBP1 | AF144233 |
| 225662_at | ZAK | H28667 | 226767_s_at | DKFZP566J204 6 | hypothetical protein DKFZp566J204 6 |
| 200679_x_at | HMGB1 | BE311760 | 204192_at | CD37 | NM_001774 |
| 213488_at | FLJ00133 | N73970 | 34726_at | CACNB3 | U07139 |
| 204224_s_at | GCH1 | NM_000161 | 238613_at | ZAK | AI475164 |
| 222745_s_at | C6orf103 | AI924685 | 221919_at | HNRPA1 | AW450929 |
| 242738_s_at | ATBF1 | BG402859 | 220576_at | PGAP1 | NM_024989 |
| 241613_at | | AW296081 | 231174_s_at | EPB41 L2 | H92979 |
| 213119_at | SLC36A1 | AW058600 | 1552691_at | ARL11 | NM_138450 |
| 222850_s_at | FLJ14281 | BF590675 | 223454_at | CXCL16 | AF275260 |
| 1558996_at | FOXP1 | AA654769 | 225140_at | KLF3 | BF438116 |
| 224814_at | DPP7 | NM_013379 | 217749_at | COPG | NM_016128 |
| 207700_s_at | NCOA3 | NM_006534 | 207277_at | CD209 | AF290886 |
| 225594_at | ZF | AL038866 | 215250_at | LOC55831 | AU147317 |
| 218974_at | FLJ10159 | NM_018013 | 1558467_a_at | | Clone IMAGE:125405, mRNA sequence |
| 231577_s_at | GBP1 | AW014593 | 212061_at | SR140 | AB002330 |
| 204570_at | COX7A1 | NM_001864 | 236072_at | ATAD1 | N64578 |
| 224610_at | RNU22 | AL530869 | 1553906_s_at | FGD2 | NM_173558 |
| 224685_at | MLLT4 | AI675354 | 203700_s_at | DIO2 | NM_013989 |
| 236259_at | STK4 | BF433725 | 237464_at | IMAA | AI241501 |
| 212254_s_at | BPAG1 | AI798790 | 226123_at | KIAA1416 | AI870918 |
| 230713_at | | BF115786 | 209733_at | LOC286440 | AL034399 |
| 219192_at | UBAP2 | NM_018449 | 201794_s_at | C1orf16 | NM_014837 |
| 205584_at | FLJ23018 | NM_024810 | 215296_at | CDC42BPA | AK027000 |
| 202307_s_at | TAP1 | NM_000593 | 235780_at | PRKACB | BE622723 |
| 229287_at | | BE326214 | 204533_at | CXCL10 | NM_001565 |
| 204439_at | C1orf29 | NM_006820 | 207339_s_at | LTB | NM_002341 |
| 203794_at | CDC42BPA | NM_014826 | 1561973_at | SMARCC2 | AL833124 |
| 202646_s_at | D1S155E | AA167775 | 215314_at | ANK3 | AU146646 |
| 219743_at | HEY2 | NM_012259 | 90265_at | CENTA1 | AW050627 |
| 222056_s_at | CGI-105 | AA723370 | 227346_at | ZNFN1A1 | AI741188 |
| 223295_s_at | LUC7L | BE049621 | 221794_at | DOCK6 | AI198543 |
| 229088_at | | BF591996 | 227805_at | MAP1D | AA779679 |
| 218146_at | AD-017 | NM_018446 | 1559584_a_at | FLJ35681 | BC025741 |
| 225932_s_at | HNRPA2B1 | AI375753 | 226090_x_at | RABL3 | AK025772 |
| 230532_at | MGC39350 | BF001685 | 210152_at | LILRB4 | U82979 |
| 201276_at | RAB5B | AF267863 | 209604_s_at | GATA3 | BC003070 |
| 1554029_a_at | KIAA0372 | BC030966 | 214917_at | PRKAA1 | AK024252 |
| 201906_s_at | CTDSPL | NM_005808 | 215768_at | | AL049337 |
| 225221_at | | AA195485 | 230748_at | SLC16A6 | AI873273 |
| 218353_at | RGS5 | NM_025226 | 232473_at | PRPF18 | AU144329 |
| 212036_s_at | PNN | AW152664 | 210042_s_at | CTSZ | AF073890 |
| 225782_at | LOC253827 | AW027333 | 231546_at | | AI638151 |
| 202042_at | HARS | NM_002109 | 229948_at | | BF511763 |
| 203504_s_at | ABCA1 | NM_005502 | 214059_at | IFI44 | BE049439 |
| 212754_s_at | KIAA1040 | AI760249 | 203760_s_at | SLA | U44403 |
| 236816_at | FLJ13089 | BF110370 | 218400_at | OAS3 | NM_006187 |
| 225949_at | LOC340371 | N21030 | 221073_s_at | CARD4 | NM_006092 |
| 208677_s_at | BSG | AL550657 | 202531_at | IRF1 | NM_002198 |
| 231853_at | TUBD1 | AK022771 | 1559882_at | SAMHD1 | AF147427 |
| 205788_s_at | KIAA0663 | NM_014827 | 231776_at | EOMES | NM_005442 |
| 212635_at | TNPO1 | AW161626 | 229035_s_at | DKFZp434G052 2 | junctophilin 3 |
| 222728_s_at | MGC5306 | AF275800 | 206978_at | CCR2 | NM_000647 |
| 225871_at | STEAP2 | BF680588 | 229686_at | P2RY8 | AI436587 |
| 226711_at | HTLF | BF590117 | 211581_x_at | LST1 | AF000426 |
| 218463_s_at | MUS81 | NM_025128 | 203988_s_at | FUT8 | NM_004480 |
| 211038_s_at | MGC12760 | BC006312 | 202901_x_at | CTSS | BC002642 |
| 225745_at | LRP6 | AV725248 | 219551_at | EAF2 | NM_018456 |
| 203860_at | PCCA | NMI_000282 | 204784_s_at | MLF1 | NM_022443 |
| 223096_at | NOP5/NOP58 | nucleolar protein NOP5/NOP58 | 204055_s_at | MGEA6 | NM_005930 |
| 222569_at | UGCGL1 | AU153746 | 219326_s_at | B3GNT1 | NM_006577 |
| 227979_at | MGC10871 | AU 152162 | 222018_at | NACA | AI992187 |
| 226030_at | ACADSB | BE897866 | 215946_x_at | LOC91316 | AL022324 |
| 208882_s_at | DD5 | U69567 | 214036_at | | BE464799 |
| 200947_s_at | GLUD1 | NM_005271 | 228711_at | ZNF37A | BF059259 |
| 211339_s_at | ITK | D13720 | 228433_at | FLJ11236 | AU157605 |
| 212985_at | FLJ14001 | BF115739 | 1566144_at | | AK098337 |
| 225986_x_at | CPSF2 | AB037788 | 214033_at | ABCC6 | AI084637 |
| 221579_s_at | NUDT3 | AF062530 | 203052_at | C2 | NM_000063 |
| 208073_x_at | TTC3 | NM_003316 | 1557539_at | | BC008052 |
| 203459_s_at | VPS16 | NM_022575 | 235385_at | FLJ20668 | AI935334 |
| 212724_at | ARHE | BG054844 | 1557155_a_at | | Clone IMAGE:5301781 , mRNA |
| 219289_at | FLJ20718 | NM_017939 | 205068_s_at | ARHGAP26 | BE671084 |
| 229741_at | | AI885294 | 227045_at | ZNF614 | AI087872 |
| 212634_at | KIAA0776 | AW298092 | 220252_x_at | FLJ11577 | NM_025159 |
| 214743_at | CUTL1 | BE046521 | 213550_s_at | CD14 | AA993683 |
| 219024_at | PLEKHA1 | NM_021622 | 214764_at | KIAA0507 | AW029169 |
| 227609_at | EPSTI1 | AA633203 | 214574_x_at | LST1 | NM_007161 |
| 204415_at | G1P3 | NM_022873 | 205116_at | LAMA2 | NM_000426 |
| 228045_at | SUGT1 | BF438106 | 211795_s_at | FYB | AF198052 |
| 227955_s_at | | CDNA: FLJ22256 fis, clone HRC02860 | 203230_at | DVL1 | AF006011 |
| 200878_at | EPAS1 | AF052094 | 201335_s_at | ARHGEF12 | NM_015313 |
| 202974_at | MPP1 | NMI_002436 | 1405_i_at | CCL5 | M21121 |
| 218263_s_at | LOC58486 | NM_021211 | 209653_at | KPNA4 | U93240 |
| 203038_at | PTPRK | NM_002844 | 219505_at | CECR1 | NM_017424 |
| 213703_at | LOC150759 | W95043 | 235536_at | | AI640483 |
| 232090_at | | AI761578 | 204197_s_at | RUNX3 | NM_004350 |
| 212419_at | C10orf56 | AA131324 | 209257_s_at | CSPG6 | BF795297 |
| 225256_at | | AI457965 | 209269_s_at | SYK | AW450910 |
| 214453_s_at | IFI44 | NM_006417 | 207734_at | LAX | NM_017773 |
| 225364_at | STK4 | BE222274 | 220987_s_at | SNARK | NM_030952 |
| 202117_at | ARHGAP1 | BG468434 | 232484_at | | AL137616 |
| 1552426_a_at | BLP2 | NM_078474 | 225775_at | MGC50844 | AK000208 |
| 212521_s_at | PDE8A | BE568219 | 238418_at | SLC35B4 | AI590926 |
| 204201_s_at | PTPN13 | NM_006264 | 212394_at | KIAA0090 | D42044 |
| 240259_at | | AI188161 | 205291_at | IL2RB | NM_000878 |
| 225629_s_at | ZBTB4 | AI669498 | 222859_s_at | DAPP1 | AA150186 |
| 229422_at | NRD1 | AA448346 | 242916_at | CEP1 | AA642477 |
| 1560339_s_at | NAP1 L4 | AK095320 | 1555759_a_at | CCL5 | AF043341 |
| 206792_x_at | PDE4C | NM_000923 | 213505_s_at | SFRS14 | BG252853 |
| 201926_s_at | DAF | BC001288 | 1558624_at | | BC033250 |
| 201655_s_at | HSPG2 | M85289 | 59631_at | TXNRD3 | AI247566 |
| 208655_at | CCNI | BG530368 | 1565689_at | | BG400570 |
| 201393_s_at | IGF2R | NM_000876 | 202954_at | UBE2C | NM_007019 |
| 220739_s_at | CNNM3 | NM_017623 | 232144_at | | AV710542 |
| 221775_x_at | RPL22 | BG152979 | 230109_at | PDE7B | AI638433 |
| 1552931_a_at | PDE8A | NM_002605 | 206914_at | CRTAM | NM_019604 |
| 200730_s_at | PTP4A1 | BF576710 | 221002_s_at | DC-TM4F2 | NM_030927 |
| 201503_at | G3BP | BG500067 | 242564_at | | AI703142 |
| 222409_at | CORO1C | AL162070 | 242973_at | | F11066 |
| 203915_at | CXCL9 | NM_002416 | 216873_s_at | ATP8B2 | AL137537 |
| 219922_s_at | LTBP3 | NMI_021070 | 206134_at | ADAMDEC1 | NM_014479 |
| 208819_at | RAB8A | BC002977 | 223316_at | CCDC3 | AL136562 |
| 201154_x_at | RPL4 | NM_000968 | 204294_at | AMT | NM_000481 |
| 212232_at | FNBP4 | AB023231 | 33646_g_at | GM2A | X61094 |
| 214239_x_at | RNF110 | AI560455 | 208997_s_at | UCP2 | U82819 |
| 202440_s_at | ST5 | NM_005418 | 243660_at | FLJ12178 | AW971892 |
| 201326_at | CCT6A | BE737030 | 219103_at | UPLC1 | NM_017707 |
| 202227_s_at | BRD8 | NM_006696 | 205488_at | GZMA | NM_006144 |
| 209984_at | JMJD2C | AB037901 | 1562189_at | | W73730 |
| 211710_x_at | RPL4 | BC005817 | 214002_at | MYL6 | AA419227 |
| 224763_at | | BF724210 | 208914_at | GGA2 | BE646414 |
| 217813_s_at | SPIN | NM_006717 | 232521_at | PCSK7 | AK027156 |
| 202411_at | IFI27 | NM_005532 | 213310_at | EIF2C2 | AI613483 |
| 219626_at | FLJ12649 | NM_024597 | AFFX-HUMISGF3A/M9 7935_MA_at | STAT1 | AFFX-HUMISGF3A/M9 7935_MA |
| 242233_at | | AI739332 | 224868_at | ZDHHC5 | BE961925 |
| 226571_s_at | PTPRS | N38920 | 242616_at | | W80359 |
| 223251_s_at | ANKRD10 | BC001727 | 1558761_a_at | C9orf10OS | AK093641 |
| 204400_at | EFS | NM_005864 | 205997_at | ADAM28 | NM_021778 |
| 211345_x_at | EEF1G | AF119850 | 1555154_a_at | QKI | AF142421 |
| 217815_at | SUPT16H | NM_007192 | 235331_x_at | RNF159 | AI341142 |
| 212074_at | UNC84A | BE972774 | 206929_s_at | NFIC | NM_005597 |
| 200089_s_at | RPL4 | AI953886 | 205798_at | IL7R | NM_002185 |
| 202465_at | PCOLCE | NM_002593 | 205238_at | FLJ12687 | NM_024917 |
| 228141_at | | AA173223 | 37226_at | BNIP1 | U15172 |
| 205590_at | RASGRP1 | NM_005739 | 212291_at | HIPK1 | AI393355 |
| 222667_s_at | ASH1L | AI806500 | 209670_at | | M12959 |
| 227088_at | | BF221547 | 210915_x_at | | T-cell receptor precursor; Human T-cell receptor rearranged beta-chain V-region (V-D-J) mRNA, complete cds. |
| 227567_at | | AL524467 | 1553275_s_at | | |
| 228347_at | SIX1 | N79004 | 238807_at | LOC157567 | AW973964 |
| 212725_s_at | TI-227H | N37081 | 226565_at | MGC21518 | AW054855 |
| 208804_s_at | SFRS6 | AL031681 | 1554444_s_at | C2orf18 | BC028081 |
| 215016_x_at | BPAG1 | BC004912 | 240118_at | | A1401105 |
| 227391_x_at | | 7d75g01.x1 NCl_CGAP_Lu2 4 Homo sapiens cDNA clone IMAGE:3278832 3' similar to contains element MER1 repetitive element , mRNA sequence. | 207038_at | SLC16A6 | NM_004694 |
| 226203_at | | AA868896 | 1567706_at | | AF009316 |
| 201395_at | RBM5 | NM_005778 | 235764_at | | AA029888 |
| 227208_at | DLNB14 | BF446390 | 206513_at | AIM2 | NM_004833 |
| 216620_s_at | ARHGEF10 | AF009205 | 206636_at | RASA2 | NM_006506 |
| 235419_at | | AW612461 | 220744_s_at | WDR10 | NM_018262 |
| 208768_x_at | RPL22 | D17652 | 241790_at | | T57946 |
| 217301_x_at | RBBP4 | X71810 | 201689_s_at | TPD52 | BE974098 |
| 208877_at | PAK2 | W74494 | 211599_x_at | MET | U19348 |
| 224792_at | TNKS1BP1 | AL566438 | 232303_at | ZNF608 | AB033107 |
| 212783_at | RBBP6 | Al538172 | 223740_at | C6orf59 | AL136708 |
| 209379_s_at | KIAA1128 | AF241785 | 238513_at | TMG4 | BF905445 |
| 224822_at | DLC1 | AA524250 | 211919_s_at | CXCR4 | AF348491 |
| 221860_at | HNRPL | AL044078 | 219386_s_at | SLAMF8 | NM_020125 |
| 209737_at | AIP1 | AB014605 | 235924_at | | N73742 |
| 212269_s_at | MCM3AP | AJ010089 | 230213_at | MGC2803 | BE220399 |
| 1552455_at | C9orf65 | NM_138818 | 204556_s_at | DZIP1 | AL568422 |
| 210999_s_at | GRB10 | U66065 | 204116_at | IL2RG | NM_000206 |
| 213193_x_at | | T cell receptor beta chain BV20S1 BJ1-5 BC1 mRNA, complete cds | 214560_at | FPRL2 | NM_002030 |
| 215412_x_at | PMS2L2 | AB017007 | 206181_at | SLAMF1 | NM_003037 |
| 200887_s_at | STAT1 | NM_007315 | 223389_s_at | ZNF581 | AF151023 |
| 205353_s_at | PBP | NM_002567 | 209712_at | SLC35D1 | AI769637 |
| 210794_s_at | MEG3 | AF119863 | 228031_at | LOC149705 | AW444778 |
| 219648_at | FLJ10116 | NM_018000 | 218748_s_at | SEC10L1 | NM_006544 |
| 218533_s_at | URKL1 | NM_017859 | 243606_at | | BE883167 |
| 213446_s_at | IQGAP1 | AI679073 | 232538_at | | AK027226 |
| AFFX-HUMISGF3A/M9 7935_3_at | STAT1 | AFFX-HUMISGF3A/M9 7935 3 | 202834_at | AGT | NM_000029 |
| 215780_s_at | SET | Z95126 | 232668_at | | AW903934 |
| 213262_at | SACS | AI932370 | 217629_at | | AA365670 |
| 225176_at | | AA156754 | 211645_x_at | IGKC | M85256 |
| 228131_at | ERCC1 | BG111047 | 221973_at | | AI983904 |
| 216306_x_at | PTBP1 | X62006 | 1552584 art | IL12RB1 | NM_153701 |
| 214043_at | PTPRD | BF062299 | 216894_x_at | CDKN1C | D64137 |
| 202893_at | UNC13B | NM_006377 | 240721_at | DBC-1 | BE672858 |
| 203448_s_at | TERF1 | AI347136 | 214836_x_at | | Clone 2-12 immunoglobulin light chain mRNA, partial cds |
| 204594_s_at | FLJ20232 | NM_013298 | 219045_at | RHOF | NM_019034 |
| 227630_at | PPP2R5E | AW274445 | 206500_s_at | C14orf106 | NM_018353 |
| 214336_s_at | COPA | AI621079 | 1563473_at | | AL833255 |
| 220692_at | HSPC047 | NM_014147 | 222895_s_at | BCL11B | AA918317 |
| 218652_s_at | FLJ20265 | NM_017733 | 228882_at | TUB | AL042088 |
| 208730_x_at | RAB2 | AA535244 | 215992_s_at | RAPGEF2 | AL117397 |
| 201876_at | PON2 | NM_000305 | 1557578_at | PHLDB2 | BQ722176 |
| 1552287_s_at | AFG3L1 | NM_001132 | 218564_at | FLJ10520 | BC002574 |
| 200689_x_at | EEF1G | NM_001404 | 236915_at | | AA625683 |
| 217719_at | EIF3S6IP | NM_016091 | 235355_at | | AL037998 |
| 201831_s_at | VDP | BE875592 | 208932_at | PPP4C | BC001416 |
| 202397_at | NUTF2 | NM_005796 | 215578_at | | AU145365 |
| 204963_at | SSPN | AL136756 | 212311_at | KIAA0746 | AA522514 |
| 229310_at | KBTBD9 | BE465475 | 213539_at | CD3D | NM_000732 |
| 226845_s_at | LOC150678 | AL036350 | 223254_s_at | KIAA1333 | AA887053 |
| 225808_at | LOC124512 | AA883486 | 229407_at | SDK1 | AF131799 |
| 225764_at | ETV6 | AI762695 | 213358_at | KIAA0802 | AB018345 |
| 203651_at | ZFYVE16 | NM_014733 | 229435_at | ZNF515 | AW025602 |
| 232080_at | NEDL2 | AL390186 | 243366_s_at | RP26 | AI936034 |
| 1554690_a_at | TACC1 | BC041391 | 55583_at | DOCK6 | AI198543 |
| 212522_at | PDE8A | W73272 | 209201_x_at | CXCR4 | L01639 |
| 228564_at | LOC375295 | AI569804 | 239725_at | | T90703 |
| 202946_s_at | BTBD3 | NM_014962 | 22H991_s_at | SYTL4 | AI167292 |
| 206481_s_at | LDB2 | NM_001290 | 210969_at | PRKCL2 | AF118089 |
| 213670_x_at | WBSCR20C | AI768378 | 204714_s_at | F5 | NM_000130 |
| 200691_s_at | HSPA9B | BC000478 | 204345_at | COL16A1 | NM_001856 |
| 235405_at | GSTA4 | N79662 | 222830_at | TFCP2L2 | BE566136 |
| 227931_at | | AI823917 | 238142_at | | AW029203 |
| 207283_at | DKFZp5471014 | spectrin, beta, non-erythrocytic 1 | 236295_s_at | NOD3 | AA694067 |
| 225664_at | COL12A1 | AA788946 | 214038_at | CCL8 | AI984980 |
| 238458_at | LOC286097 | AI868167 | 52837_at | KIAA1644 | AL047020 |
| 217945_at | BTBD1 | NM_025238 | 243675_at | | BF512500 |
| 201559_s_at | CLIC4 | AF109196 | 206682_at | CLECSF14 | NM_006344 |
| 225060_at | LRP11 | BF696304 | 1558385_at | | AL832806 |
| 201174_s_at | TERF2IP | NM_018975 | 227817_at | PRKCB1 | R51324 |
| 231213_at | PDE1A | AU146305 | 243634_at | | BF028225 |
| 1554154_at | GDAP2 | BC013132 | 218337_at | RAI16 | NM_022749 |
| 202373_s_at | RAB3-GAP150 | rab3 GTPase-activating protein, non-catalytic subunit (150kD) | 244646_at | | AW972881 |
| 221501_x_at | KIAA0220 | AF229069 | 227533_at | | AA732944 |
| 227854_at | FANCL | BE620258 | 217480_x_at | IGKC | M20812 |
| 229450_at | IFIT4 | AI075407 | 205467_at | CASP10 | NM_001230 |
| 218181_s_at | MAP4K4 | NM_017792 | 228171_s_at | DKFZP434I216 | DKFZP4341216 protein |
| 1554089_s_at | SBDS | BC010183 | 223539_s_at | SERF1A | AF073518 |
| 226272_at | | N25986 | 202062_s_at | SEL1L | NM_005065 |
| 224711_at | YY1 | AI670903 | 225208_s_at | C6orf119 | AW575350 |
| 1555929_s_at | | Iaa10f11.x1 8 5 week embryo anterior tongue 8 5 EAT Homo sapiens cDNA 3', mRNA sequence. | 227192_at | LOC112476 | BF060707 |
| 212314_at | KIAA0746 | AB018289 | 207336_at | SOX5 | NM_006940 |
| 244050_at | LOC401494 | AI804932 | 238694_at | | AI589594 |
| 227476_at | | AW576195 | 230407_at | | AW440490 |
| 212096_s_at | MTUS1 | AL096842 | 227224_at | RALGPS2 | AW003297 |
| 219038_at | ZCWCC2 | NM_024657 | 217371_s_at | IL15 | Y09908 |
| 1569201_a_at | | Clone IMAGE:3454421 mRNA | 219014_at | PLAC8 | NM_016619 |
| 202336_s_at | PAM | NM_000919 | 215891_s_at | GM2A | X61094 |
| 90610_at | LRCH4 | AI654857 | 202921_s_at | ANK2 | NM_001148 |
| 210645_s_at | TTC3 | D83077 | 222890_at | HSPC065 | BG054922 |
| 242625_at | cig5 | AW189843 | 233152_x_at | | MRNA; cDNA DKFZp564C142 (from clone DKFZp564C142 ) |
| 224955_at | TEAD1 | AI590088 | 227025_at | PPHLN1 | BG284497 |
| 46665_at | SEMA4C | A1949392 | 240921_at | | A1027296 |
| 201084_s_at | BCLAF1 | NM_014739 | 213924_at | MPPE1 | BF476502 |
| 212911_at | KIAA0962 | AB023179 | 223680_at | ZNF607 | BC005085 |
| 226409_at | C20orf140 | BE349614 | 214617_at | PRF1 | AI445650 |
| 228750_at | | AI693516 | 205018_s_at | MBNL2 | NM_005757 |
| 222440_s_at | THRAP3 | AL576205 | 222891_s_at | BCL11A | AI912275 |
| 225990_at | BOC | W72626 | 239896_at | | AW190479 |
| 202271_at | KIAA0483 | AB007952 | 236521_at | | BF196060 |
| 218607_s_at | SDAD1 | NM_018115 | 239414_at | | BF942260 |
| 239476_at | PIK3R1 | AW152166 | 233302_at | | AU146285 |
| 1566482_at | | AL833114 | 212444_at | RAI3 | AA156240 |
| 225332_at | | BF674064 | 210972_x_at | | CDNA clone MGC:71411 IMAGE:4853814 , complete cds |
| 1558080_s_at | LOC144871 | BG913589 | 213842_x_at | WBSCR20C | AK021688 |
| 208739_x_at | SUMO2 | L76416 | 1554240_a_at | ITGAL | BC008777 |
| 209652_s_at | PGF | BC001422 | 232262_at | PIGL | AU155941 |
| 221253_s_at | TXNDC5 | NM-030810 | 209782_s_at | DBP | U79283 |
| 204502_at | SAMHD1 | NM_015474 | 40837_at | TLE2 | M99436 |
| 225002_s_at | SUMF2 | BE349022 | 226911_at | FLJ39155 | BF114725 |
| 200680_x_at | HMGB1 | NM_002128 | 206236_at | GPR4 | NM_005282 |
| 225573_at | FLJ12592 | AL518293 | 216207_x_at | IGKC | AW408194 |
| 227740_at | KIS | AW173222 | 210715_s_at | SPINT2 | AF027205 |
| 230063_at | ZNF264 | BF063192 | 1552497_a_at | SLAMF6 | NM_052931 |
| 204655_at | CCL5 | NM_002985 | 230605_at | KCNAB1 | BF433830 |
| 209932_s_at | DUT | U90223 | 204562_at | IRF4 | NM_002460 |
| 200717_x_at | RPL7 | NM_000971 | 236285_at | LOC113730 | AI631846 |
| 212805_at | KIAA0367 | AB002365 | 206144_at | BAIAP1 | NM_004742 |
| 214092_x_at | SFRS14 | AI928127 | 206666_at | GZMK | NM_002104 |
| 218138_at | MKKS | NM_018848 | 219684_at | IFRG28 | NM_022147 |
| 224829_at | CPEB4 | AA772278 | 229176_at | ANKH | AI672354 |
| 224894_at | YAP1 | BF247906 | 205267_at | POU2AF1 | NM_006235 |
| 204211_x_at | PRKR | NM_002759 | 204103_at | CCL4 | NM_002984 |
| 228718_at | | AI379070 | 228977_at | IL17D | AI669535 |
| 225916_at | ZNF131 | AA789302 | 210279_at | GPR18 | AF261135 |
| 212542_s_at | PHIP | BF224151 | 204118_at | CD48 | NM_001778 |
| 208798_x_at | GOLGIN-67 | AF204231 | 214032_at | ZAP70 | AI817942 |
| 221763_at | JMJD1C | AI694023 | 225617_at | ODF2 | AL138382 |
| 204076_at | LYSAL1 | AB002390 | 1554608_at | TGOLN2 | BC028219 |
| 226696_at | RBBP9 | AI761595 | 242156_at | | AA765841 |
| 229218_at | COL1A2 | AA628535 | 227213_at | DEADC1 | AA706895 |
| 221884_at | EVI1 | BE466525 | 236010_at | | AI373107 |
| 204776_at | THBS4 | NM_003248 | 204891_s_at | LCK | NM_005356 |
| 212503_s_at | KIAA0934 | N22859 | 217148_x_at | IGLJ3 | AJ249377 |
| 218793_s_at | SCML1 | NM_006746 | 209361_s_at | PCBP4 | BC004153 |
| 212297_at | AFURS1 | BF218804 | 222062_at | IL27RA | AI983115 |
| 238736_at | REV3L | AA805939 | 201378_s_at | NICE-4 | NM_014847 |
| 203662_s_at | TMOD1 | NM_003275 | 210995_s_at | ARFD1 | AF230399 |
| 205668_at | LY75 | NM_002349 | 242162_at | FLJ25955 | AA904430 |
| 1553186_x_at | RAB45 | NM 152573 | 215084_s_at | MGC8974 | AL031427 |
| 221483_s_at | ARPP-19 | AF084555 | 205188_s_at | SMAD5 | NM_005903 |
| 212276_at | LPIN1 | D80010 | 215214_at | | H53689 |
| 228046_at | LOC152485 | AA741243 | 218816_at | LRRC1 | NM_018214 |
| 1558801_at | | AK055769 | 1568597_at | | CA309468 |
| 227517_s_at | GAS5 | AI056992 | 219528_s_at | BCL11B | NM_022898 |
| 207785_s_at | RBPSUH | NM_015874 | 204117_at | PREP | NM_002726 |
| 226126_at | MGC16169 | AA702160 | 214157_at | GNAS | AA401492 |
| 208313_s_at | SF1 | NM_004630 | 216401_x_at | IGKC | AJ408433 |
| 209210_s_at | PLEKHC1 | Z24725 | 1562953_s_at | FBI4 | BC019264 |
| 219957_at | RUFY2 | NM_017987 | 228962_at | PDE4D | BF507941 |
| 202780_at | OXCT1 | NM_000436 | 204862_s_at | NME3 | NM_002513 |
| 200091_s_at | RPS25 | AA888388 | 217281_x_at | IGHG1 | AJ239383 |
| 203370_s_at | PDLIM7 | NM_005451 | 231895_at | DKFZp761A078 | ne67f08.s1 NCI_CGAP_Alv 1 Homo sapiens cDNA clone IMAGE:909351, mRNA sequence. |
| 225756_at | CSNK1E | AV762065 | 205890_s_at | UBD | NM_006398 |
| 200972_at | TM4SF8 | BC000704 | 206439_at | DSPG3 | NM_004950 |
| 1556325_at | FILIP1 | AL832009 | 234764_x_at | IGL@ | U96394 |
| 229342_at | | AI708256 | 240159_at | SLC15A2 | AA836116 |
| 1559332_at | CRIM1 | BC016339 | 212094_at | PEG10 | AL582836 |
| 1566324_a_at | MAF | AA442149 | 206574_s_at | PTP4A3 | NM_007079 |
| 204797_s_at | EML1 | NM_004434 | 235643_at | FLJ39885 | BE886225 |
| 226575_at | ZNF462 | T89120 | 219239_s_at | FLJ10997 | NM_018293 |
| 220890_s_at | DDX47 | NM_016355 | 210164_at | GZMB | J03189 |
| 219243_at | HIMAP4 | NM_018326 | 214768_x_at | | Clone 2-12 immunoglobulin light chain mRNA, partial cds |
| 244872_at | SYNCOILIN | BE514107 | 236293_at | | BE676335 |
| 208808_s_at | HMGB2 | BC000903 | 33132_at | CPSF1 | U37012 |
| 228971_at | | AI357655 | 237753_at | | AW504569 |
| 224319_s_at | FLJ20232 | AL136768 | 226664_at | C20orf140 | AL121747 |
| 226381_at | | AW450329 | 240877_x_at | | EST386882 MAGE resequences, MAGN Homo sapiens cDNA, mRNA sequence. |
| 224984_at | NFAT5 | W61007 | 212975_at | KIAA0870 | AB020677 |
| 222753_s_at | FLJ22649 | AL136660 | 1559814_at | | AK024712 |
| 218950_at | ARAP3 | NM_022481 | 236782_at | SAMD3 | AI129628 |
| 226137_at | ATBF1 | AI288759 | 226457_at | | BG527339 |
| 224928_at | SET7 | AK024846 | 216557_x_at | IGHG1 | U92706 |
| 223281_s_at | COX15 | AF026850 | 206150_at | TNFRSF7 | NM_001242 |
| 224716_at | SLC35B2 | BG163267 | 226754_at | ZNF251 | W93231 |
| 203803_at | PCYOX1 | N45309 | 205692_s_at | CD38 | NM_001775 |
| 226117_at | T2BP | AA195074 | 244476_at | | R39769 |
| 1552329_art | RBBP6 | BC029352 | 222838_at | SLAMF7 | AL121985 |
| 200795_at | SPARCL1 | NM_004684 | 217960_s_at | TOMM22 | NM_020243 |
| 231968_at | | AK025416 | 212105_s_at | DHX9 | BF313832 |
| 202202_s_at | LAMA4 | NM_002290 | 202638_s_at | ICAM1 | NM_000201 |
| 223264_at | MESDC1 | BC001373 | 1565705_x_at | | CDNA: FLJ21395 fis, clone COL03557 |
| 201681_s_at | DLG5 | AB011155 | 244752_at | LOC220929 | AI563915 |
| 211935_at | ARL6IP | D31885 | 216510_x_at | IGHG1 | AB035175 |
| 203906_at | KIAA0763 | AI652645 | 205456_at | CD3E | NM_000733 |
| 222669_s_at | SBDS | AK001779 | 1555565_s_at | TAPBP | AF314222 |
| 201621_at | NBL1 | NM_005380 | 222858_s_at | DAPP1 | AI632216 |
| 207405_s_at | RAD17 | NM_002873 | 207216_at | TNFSF8 | NM_001244 |
| 201622_at | SND1 | NM_014390 | 1553107_s_at | FLJ37562 | BF436799 |
| 209442_x_at | ANK3 | AL136710 | 209374_s_at | IGHM | BC001872 |
| 213161_at | C9orf97 | AI583393 | 1557689_at | | BU683708 |
| 213677_s_at | PMS1 | BG434893 | 205294_at | BAIAP2 | NM_017450 |
| 227677_at | JAK3 | BF512748 | 237176_at | | AW205969 |
| 214701_s_at | FN1 | AJ276395 | 211798_x_at | IGLJ3 | AB001733 |
| 212001_at | SFRS14 | AV738039 | 234563_at | | AK000795 |
| 201237_at | CAPZA2 | AV685920 | 203895_at | PLCB4 | AL535113 |
| 211048_s_at | ERP70 | BC006344 | 203047_at | STK10 | NM_005990 |
| 215758_x_at | ZNF505 | AC007204 | 219403_s_at | HPSE | NM_006665 |
| 201948_at | HUMAUANTIG | nucleolar GTPase | 229055_at | | AI805006 |
| 217850_at | NS | NM_014366 | 1554676_at | PRG1 | BC022313 |
| 204849_at | TCFL5 | NM_006602 | 215994_x_at | KIAA0676 | AK001196 |
| 210985_s_at | sup100 | AF056322 | 211902_x_at | | putative; Homo sapiens T-cell receptor alpha chain (TCRA) mRNA (HLA-A1, 24; B7, 8; DR 1, 3), complete cds. |
| 202146_at | IFRD1 | AA747426 | 1554153_a_at | BHC80 | BC015714 |
| 216210_x_at | HRIHFB2122 | AA046650 | 209691_s_at | DOK4 | BC003541 |
| 228662_at | SOCS7 | AI492369 | 206553_at | OAS2 | NM_002535 |
| 213500_at | | AI307760 | 229629_at | | AI923633 |
| 226159_at | LOC285636 | N31982 | 222784_at | SMOC1 | AJ249900 |
| 224718_at | YY1 | AK025731 | 213747_at | OAZIN | AA047234 |
| 227405_s_at | FZD8 | AW340311 | 1566551_at | | AL137307 |
| 64064_at | IAN4L1 | AI435089 | 229672_at | | AA707125 |
| 227954_at | LOC162073 | AI458417 | 229958_at | | W93695 |
| 209406_at | BAG2 | AF095192 | 217235_x_at | | Immunoglobulin light chain lambda variable region [Homo sapiens], mRNA sequence |
| 213018_at | ODAG | AI337901 | 1561615_s_at | SLC8A1 | Y12885 |
| 210825_s_at | STOM | AF130103 | 203413_at | NELL2 | NM_006159 |
| 213334_x_at | TREX2 | BE676218 | 209813_x_at | TRGV9 | M16768 |
| 202600_s_at | NRIP1 | AI824012 | 236280_at | | AI225238 |
| 233036_at | | AU146418 | 227245_at | FLJ13089 | AW511198 |
| 208712_at | CCND1 | M73554 | 217227_x_at | IGL@ | X93006 |
| 203355_s_at | EFA6R | NM_015310 | 211634_x_at | IGHG1 | M24669 |
| 221472_at | C20orf121 | Z97053 | 204529_s_at | TOX | AI961231 |
| 222101_s_at | PCDH16 | BF222893 | 224304_x_at | NIN | AF223939 |
| 224724_at | SULF2 | AL133001 | 227894_at | LOC197336 | AL043021 |
| 230728_at | | BF516305 | 214916_x_at | IGHM | BG340548 |
| 203875_at | SMARCA1 | NM_003069 | 207968_s_at | MEF2C | NM_002397 |
| 213063_at | FLJ11806 | BF970253 | 219385_at | SLAMF8 | NM_020125 |
| 226334_s_at | AHSA2 | AW117717 | 237625_s_at | | Immunoglobulin kappa light chain mRNA, partial cds |
| 201641_at | BST2 | NM_004335 | 234366_x_at | | Clone H3 anti-mucin1 light chain variable region mRNA, partial cds |
| 223842_s_at | SCARA3 | AB007830 | 219532_at | ELOVL4 | NM_022726 |
| 224755_at | | BE621524 | 217179_x_at | IGLJ3 | X79782 |
| 202082_s_at | SEC14L1 | AV748469 | 1554762_a_at | BOMB | BC017957 |
| 232174_at | | AA480392 | 216984_x_at | | immunoglobulin lambda joining 3 |
| 224817_at | NEURL | W93554 | 224342_x_at | H.sapiens | (T1.1) mRNA for IG lambda light chain |
| 236325_at | | BF057799 | 214176_s_at | PBXIP1 | AI348545 |
| 213664_at | SLC1A1 | AW235061 | 232311_at | B2M | AU147899 |
| 217643_x_at | | Transcribed sequence with .weak similarity to protein ref:NP_060265. 1 (H.sapiens) hypothetical protein FLJ20378 [Homo sapiens] | 224853_at | KIAA1458 | AI979301 |
| 219221_at | FLJ35036 | NM_024724 | 222909_s_at | BAG4 | AF111116 |
| 228667_at | | AI733330 | 214777_at | | BG482805 |
| 202129_s_at | RIOK3 | AW006290 | 220219_s_at | KIAA0563 | NM_018001 |
| 213019_at | RANBP6 | AI123233 | 1554411_at | CTNNB1 | AB062292 |
| AFFX-HUMISGF3A/M9 7935_MB_at | STAT1 | AFFX-HUMISGF3A/M9 7935_MB | 226082_s_at | SFRS15 | AW513629 |
| 232458_at | COL3A1 | AU146808 | 1570166_a_at | | Similar to hypothetical protein FLJ13381, clone IMAGE:4719554 , mRNA |
| 242920_at | | AW590838 | 207704_s_at | GAS7 | NM_003644 |
| 212039_x_at | RPL3 | BG339228 | 223765_s_at | KBTBD4 | AF151086 |
| 228959_at | AI676241 | AI676241 | 207651_at | H963 | NM_013308 |
| 221260_s_at | C12orf22 | NM_030809 | 212800_at | STX6 | AI740832 |
| 229480_at | | AI341053 | 207509_s_at | LAIR2 | NM_002288 |
| 203802_x_at | WBSCR20C | NM_018044 | 1558775_s_at | NSMAF | AU142380 |
| 201024_x_at | EIF5B | BG261322 | 221964_at | TULP3 | AI591305 |
| 224709_s_at | SPEC2 | AF131831 | 243365_s_at | AUTS2 | AI417756 |
| 214494_s_at | SPG7 | NM_005200 | 220306_at | FLJ20202 | NM_017709 |
| 212148_at | PBX1 | AL049381 | 230966_at | IL4I1 | AI859620 |
| 205134_s_at | NUFIP1 | AW593143 | 232298-at | | AK026494 |
| 220076_at | ANKH | NM_019847 | 1555464_at | MDA5 | BC046208 |
| 225044_at | MGC20781 | AL526783 | 224998_at | CKLFSF4 | AK000855 |
| 221204_s_at | CRTAC1 | NM_018058 | 219251_s_at | FLJ10300 | NM_018051 |
| 234873_x_at | RPL7A | AJ224080 | 231647_s_at | IRTA2 | AW241983 |
| 212958_x_at | PAM | AI022882 | 215806_x_at | TRGV9 | M13231 |
| 1553613_s_at | FOXC1 | NM_001453 | 237491_at | | AA700633 |
| 225289_at | STAT3 | AI139252 | 211635_x_at | IGHG1 | M24670 |
| 235952_at | | AA521504 | 238784_at | FLJ32949 | AI039361 |
| 234723_x_at | | CDNA: FLJ21228 fis, clone COL00739 | 217147_s_at | TRIM | AJ240085 |
| 226465_s_at | SON | BF676840 | 1564203_at | LOC147004 | AK057317 |
| 201690_s_at | TPD52 | AA524023 | 1554489 a at | BITE | BC016050 |
| 209082_s_at | COL18A1 | AF018081 | 228439_at | MGC20410 | AW083820 |
| 209969_s_at | STAT1 | BC002704 | 242995_at | | AW976347 |
| 225922_at | KIAA1450 | BE501838 | 211643_x_at | | Clone P2-32 anti-oxidized LDL immunoglobulin light chain Fab mRNA, partial cds |
| 223279_s_at | UACA | AF322916 | 205993_s_at | TBX2 | NM_005994 |
| 201398_s_at | TRAM1 | BC000687 | 211810_s_at | GALC | D25284 |
| 213923_at | RAP2B | AW005535 | 206641_at | TNFRSF17 | NM_001192 |
| 216250_s_at | LPXN | X77598 | 211641_x_at | IGHG1 | L06101 |
| 200935_at | CALR | NM_004343 | 211881_x_at | IGLJ3 | AB014341 |
| 225847_at | KIAA1363 | AB037784 | 211868_x_at | IGHG1 | AJ225092 |
| 212134_at | PHLDB1 | AB014538 | 241401_at | FBI4 | BG496631 |
| 226218_at | IL7R | BE217880 | 243092_at | | AI140189 |
| 226250_at | | AU144961 | 236719_at | | AI042187 |
| 223092_at | ANKH | AA854943 | 203311_s_at | ARF6 | M57763 |
| 1564139_at | LOC144571 | AK056852 | 229625_at | GBP5 | BG545653 |
| 201417_at | SOX4 | AL136179 | 1552472_a_at | CENTB2 | NM_012287 |
| 226519_s_at | MGC15875 | AL561859 | 36564_at | IBRDC3 | W27419 |
| 217868_s_at | DREV1 | NM_016025 | 205569_at | LAMP3 | NM_014398 |
| 205571_at | LIPT1 | NM_015929 | 221601_s_at | TOSO | AI084226 |
| 223555_at | FLJ20203 | AL136565 | 227409_at | PPP1 R3E | AA167748 |
| 201009_s_at | TXNIP | AI439556 | 229829_at | C18orf18 | AA429735 |
| 211666_x_at | RPL3 | L22453 | 229178_at | LOC145786 | AV699825 |
| 212798_s_at | ANKMY2 | AK001389 | 1569961_at | | BC033124 |
| 202241_at | TRIB1 | NM_025195 | 204852_s_at | PTPN7 | NM_002832 |
| 209579_s_at | MBD4 | AL556619 | 225566_at | NRP2 | AI819729 |
| 221844_x_at | | Transcribed sequence with moderate similarity to protein sp:P39195 (H.sapiens) ALU8_HUMAN Alu subfamily SX sequence contamination warning entry | 223565_at | PACAP | AF151024 |
| 209225_x_at | TNPO1 | AI653355 | 239237_at | | AI798822 |
| 229891_x_at | KIAA1704 | AI630799 | 37831_at | SIPA1L3 | AB011117 |
| 210260_s_at | TNFAIP8 | BC005352 | 205242_at | CXCL13 | NM_006419 |
| 230266_at | MGC9726 | AI127991 | 228111_s_at | XLHSRF-1 | AI004779 |
| 200883_at | UQCRC2 | NM_003366 | 204698_at | ISG20 | NM_002201 |
| 240458_at | | AI242023 | 216920_s_at | TRGV9 | M27331 |
| 210211_s_at | HSPCA | AF028832 | 240070_at | FLJ39873 | AW512550 |
| 222824_at | SEC61A2 | AW237290 | 238581_at | GBP5 | BG271923 |
| 225502_at | DOCK8 | AL161725 | 234753_x_at | | Homo sapiens cDNA: FLJ22781 fis, clone KAIA1958. |
| 204072_s_at | 13CDNA73 | NM_023037 | 243065_at | | AA809449 |
| 242945_at | FAM20A | AI860568 | 220091_at | SLC2A6 | NM_017585 |
| 208664_s_at | TTC3 | AU131711 | 221286_s_at | PACAP | NM_016459 |
| 213988_s_at | SAT | BE971383 | 242288_s_at | EMILIN2 | AL552384 |
| 201976_s_at | MYO10 | NM_012334 | 205671_s_at | HLA-DOB | NM_002120 |
| 214328_s_at | HSPCA | R01140 | 217258_x_at | | Immunoglobulin lambda light chain variable and constant region mRNA, partial cds |
| 1557813_at | | BF724621 | 204346_s_at | RASSF1 | NM_007182 |
| 222999_s_at | CCNL2 | AF251294 | 237496_at | | AI821404 |
| 239811_at | | BF954306 | 221813_at | KIAA1332 | AI129395 |
| 241905_at | | AA579047 | 210163_at | CXCL11 | AF030514 |
| 225924_at | KIAA1450 | AI478634 | 1558041_a_at | LOC283849 | AL834156 |
| 218017 s at | FLJ32731 | NM_025070 | 219944_at | FLJ21069 | NM_024692 |
| 233276_at | | AU146390 | 235353_at | KIAA0746 | AI887866 |
| 212884_x_at | APOE | AI358867 | 1558972_s_at | C6orf190 | BC043608 |
| 218418_s_at | ANKRD25 | NM_015493 | 210140_at | CST7 | AF031824 |
| 227840_at | LOC130355 | AA738440 | 236777_at | | AA854843 |
| 226267_at | JDP2 | AA716425 | 216689_x_at | ARHGAP1 | U62794 |
| 228605_at | | AW449230 | 216412_x_at | | Immunoglobulin lambda light chain variable and constant region mRNA, partial cds |
| 217820_s_at | ENAH | NM_018212 | 229721_x_at | FLJ43842 | AI655697 |
| 209024_s_at | SYNCRIP | AI472757 | 211649_x_at | | Partial mRNA for immunoglobulin heavy chain variable region (IGHV32-D-JH-Calpha2 gene), clone ET74 |
| 226968_at | KIF1B | AK023184 | 227048_at | LAMA1 | AI990816 |
| 203211_s_at | MTMR2 | AK027038 | 236261_at | OSBPL6 | AI949389 |
| 227993_at | METAP2 | AW003997 | 1569830 at | PTPRC | BC031525 |
| 226340_x_at | DKFZp434K132 3 | tu87d04.x1 NCI_CGAP_Gas 4 Homo sapiens cDNA clone IMAGE:2258023 3', mRNA sequence. | 229038_at | CWF19L1 | BF939646 |
| 209700_x_at | PDE4DIP | AB042555 | 1557540_at | | BQ006233 |
| 215146_s_at | KIAA1043 | AB028966 | 211650_x_at | | Partial mRNA for IgM immunoglobulin heavy chain variable region (IGHV gene), clone LIBPM376 |
| 238974_at | FLJ38973 | N47077 | 210169_at | KIAA0420 | AB007880 |
| 204198_s_at | RUNX3 | AA541630 | 225619_at | FLJ30046 | AV730849 |
| 226463_at | ATP6V1C1 | AW241758 | 224404_s_at | IRTA2 | AF343662 |
| 202796_at | SYNPO | NM_007286 | 210797_s_at | OASL | AF063612 |
| 212231_at | FBXO21 | AB020682 | 215949_x_at | IGHG1 | BF002659 |
| 228590_at | FLJ20758 | AA045257 | 235229_at | OR2I6 | AI694413 |
| 230416_at | SLC18A2 | AI709335 | 230011_at | MGC40042 | AW195720 |
| 201715_s_at | ACIN1 | NM_014977 | 1563792_at | AMN | AK092824 |
| 224996_at | MGC34646 | N30209 | 200917_s_at | SRPR | BG474541 |
| 203006_at | INPP5A | NM_005539 | 213915_at | NKG7 | NM_005601 |
| 239442_at | KIAA0582 | BF589179 | 1553856_s_at | P2RY10 | NM_014499 |
| 225644_at | FLJ33814 | BF060776 | 1569052_at | | BC010121 |
| 213429_at | | AW025579 | 219159_s_at | SLAMF7 | NM_021181 |
| 223584_s_at | KBTBD2 | BF000166 | 239122_at | IL24 | AI638155 |
| 221087_s_at | APOL3 | NM_014349 | 204269_at | PIM2 | NM_006875 |
| 202907_s_at | NBS1 | NM_002485 | 236191_at | | T81422 |
| 219582_at | OGFRL1 | NM_024576 | 1555214_a_at | CLECSF12 | AF400602 |
| 222770_s_at | FLJ13220 | AK025248 | 207348_s_at | LIG3 | NM_002311 |
| 222420_s_at | UBE2H | Z29331 | 211908_x_at | IGHG1 | M87268 |
| 1555847_a_at | LOC284454 | BU617052 | 1553132_a_at | MTAC2D1 | NM_152332 |
| 221502_at | KPNA3 | AL120704 | 214784_x_at | XPO6 | BE966299 |
| 200766_at | CTSD | NM_001909 | 213437_at | RIPX | AA861784 |
| 1552733_at | KLHDC1 | NM_172193 | 223667_at | FKBP7 | AF092137 |
| 224893_at | LOC283241 | AA775408 | 205758_at | CD8A | AW006735 |
| 225033_at | LOC286167 | AV721528 | 231628_s_at | | xq64a07.x1 NCI_CGAP_HN 9 Homo sapiens cDNA clone IMAGE:2755380 3', mRNA sequence. |
| 224569_s_at | IRF2BP2 | AW242432 | 222280_at | | BG491393 |
| 214035_x_at | LOC339047 | AA308853 | 216430_x_at | | immunoglobulin lambda joining 3 |
| 1556989_at | | AF086069 | 216829_at | | X72475 |
| 203241_at | UVRAG | NM_003369 | 213797_at | cig5 | AI337069 |
| 225293_at | COL27A1 | AK021957 | 226569_s_at | CHTF18 | AK024476 |
| 238121_at | | AI473796 | 205049_s_at | CD79A | NM_001783 |
| 212629_s_at | PRKCL2 | AI633689 | 211122_s_at | CXCL11 | AF002985 |
| 241344_at | | AI821780 | 211640_x_at | IGHG1 | L23519 |
| 218989_x_at | SLC30A5 | NM_022902 | 216573_at | | X84340 |
| 219220_x_at | MRPS22 | NM_020191 | 226299_at | PKN3 | NM_013355 |
| 208946_s_at | BECN1 | AF139131 | 33304_at | ISG20 | U88964 |
| 212822_at | HEG | AA121502 | 213958_at | CD6 | AW134823 |
| 209077_at | TXN2 | AL022313 | 234306_s_at | SLAMF7 | AJ271869 |
| 209047_at | AQP1 | AL518391 | 203828_s_at | NK4 | NMI_004221 |
| 219700_at | PLXDC1 | NM_020405 | 213785_at | IPO9 | AW269792 |
| 211651_s_at | LAMB1 | M20206 | 1552504_a_at | KIAA1811 | NM_032430 |
| 203322_at | KIAA0863 | AU145934 | 230894_s_at | MSI2 | BE672557 |
| 207943_x_at | PLAGL1 | NM_006718 | 209611_s_at | SLC1A4 | AB026689 |
| 200655_s_at | CALM1 | NM_006888 | 210031_at | CD3Z | J04132 |
| 206315_at | CRLF1 | NM_004750 | 210321_at | GZMH | M36118 |
| 218532_s_at | FLJ20152 | NMI_019000 | 219971_at | IL21R | NM_021798 |
| 1552735_at | PCDHGA4 | AL832028 | 212831_at | EGFL5 | BF110421 |
| 230791_at | | AU146924 | 229921_at | KIF5A | BF196255 |
| 55872_at | KIAA1196 | AI493119 | 204960_at | PTPRCAP | NM_005608 |
| 205931_s_at | CREB5 | NM_004904 | 1562236_at | MYST4 | AL832065 |
| 1564796_at | EMP1 | BC017854 | 241525_at | LOC200772 | AV700191 |
| 232347_x_at | | CDNA FLJ11379 fis, clone HEMBA1000469 | 234381_at | | D87024 |
| 209955_s_at | FAP | U76833 | 216517_at | IGKV1D-8 | Z00008 |
| 221954_at | C20orf111 | AA160474 | 236117_at | | AA706701 |
| 200736_s_at | GPX1 | NM_000581 | 203532_x_at | CUL5 | AF017061 |
| 211956_s_at | SUI1 | BF246436 | 228326_at | MGC43690 | AI016894 |
| 200094_s_at | EEF2 | AI004246 | 234419_x_at | IGHG1 | AJ275401 |
| 1556339_a_at | | CDNA FLJ39845 fis, clone SPLEN2014452 | 1554208_at | MGC40042 | BC032248 |
| 212765_at | KIAA1078 | AB029001 | 211876_x_at | PCDHGA11 | AF152504 |
| 209522_s_at | CRAT | BC000723 | 215565_at | DTNB | AK022277 |
| 201883_s_at | B4GALT1 | D29805 | 218843_at | FNDC4 | NM_022823 |
| 212136_at | ATP2B4 | AW517686 | 211059_s_at | GOLGA2 | BC006381 |
| 218111_s_at | CMAS | NM_018686 | 219497_s_at | BCL11A | NM_022893 |
| 217808_s_at | MAPKAP1 | NM_024117 | 231249_at | HT036 | BE676062 |
| 213656_s_at | KNS2 | BF593594 | 234848_at | | AE000659 |
| 216215_s_at | RBM9 | AL049748 | 236301_at | | AA789123 |
| 211938_at | EIF4B | BF247371 | 204999_s_at | ATF5 | BC005174 |
| 209242_at | PEG3 | AL042588 | 230015_at | | AV729651 |
| 208827_at | PSMB6 | BC000835 | 219541_at | FLJ20406 | NM_017806 |
| 222140_s_at | SH120 | AK021758 | 232442_at | | AU147442 |
| 201592_at | EIF3S3 | NM_003756 | 211102_s_at | LILRA2 | U82277 |
| 212721_at | SFRS12 | AI810380 | 213830_at | TRD@ | AW007751 |
| 1560763_at | | BC033548 | 221212_x_at | PB1 | NM_018313 |
| 213229_at | DICER1 | BF590131 | 232858_at | | AK021989 |
| 214298_x_at | Sep-06 | AL568374 | 201514_s_at | G3BP | NM_005754 |
| 207469_s_at | PIR | NM_003662 | 230128_at | | AK025231 |
| 212180_at | CRKL | AK000311 | 204958_at | PLK3 | NM_004073 |
| 208908_s_at | CAST | AF327443 | 222727_s_at | SLC24A6 | AI339568 |
| 203319_s_at | ZNF148 | L04282 | 209727_at | GM2A | M76477 |
| 206414_s_at | DDEF2 | NM_003887 | 39318_at | TCL1A | X82240 |
| 225138_at | ZRANB1 | N52625 | 217084_at | IGHG1 | AF015124 |
| 212671_s_at | HLA-DQA1 | BG397856 | 242749_at | | AI022173 |
| 214696_at | MGC14376 | AF070569 | 242020_s_at | ZBP1 | AI925506 |
| 202125_s_at | ALS2CR3 | NM_015049 | 214567_s_at | XCL1 | NM_003175 |
| 219863_at | CEB1 | NM_016323 | 225301_s_at | MYO5B | AI991160 |
| 226985_at | FGD5 | AW269340 | 217799_x_at | UBE2H | NM_003344 |
| 204715_at | PANX1 | NM_015368 | 227030_at | | BG231773 |
| 213260_at | FOXC1 | AU145890 | 243375_at | | AI742685 |
| 223796_at | CNTNAP3 | AF333769 | 244144_at | SYNE1 | N52270 |
| 222619_at | ZNF281 | AU150752 | 220233_at | FBXO26 | NM_024907 |
| 1562062_at | | BM041211 | 216000_at | KIAA0484 | AA732995 |
| 215626_at | | AU144887 | 221903_s_at | CYLD | BE046443 |
| 222395_s_at | FLJ13855 | BE544096 | 1552656_s_at | KIS | NM_144624 |
| 212591_at | KIAA0117 | AA887480 | 207287_at | FLJ14107 | NM_025026 |
| 200768_s_at | MAT2A | BC001686 | 206313_at | HLA-DOA | NM_002119 |
| 226850_at | SUMF1 | AA683501 | 1555613_a_at | ZAP70 | AB083211 |
| 243933_at | | AI096634 | 230209_at | | AW194655 |
| 238773_at | FLJ33979 | AA251906 | 1569362_at | ALCAM | BC041127 |
| 223094_s_at | ANKH | AF274753 | 236341_at | CTLA4 | AI733018 |
| 232094_at | FLJ22557 | AU144048 | 205901_at | PNOC | NM_006228 |
| 218135_at | PTX1 | NM_016570 | 232286_at | | AA572675 |
| 230669_at | RASA2 | W38444 | 205495_s_at | GNLY | NM_006433 |
| 218250_s_at | CNOT7 | NM_013354 | 210029_at | INDO | M34455 |
| 213069_at | HEG | AI148659 | 207061_at | ERN1 | NM_001433 |
| 205326_at | RAMP3 | NM_005856 | 218848_at | MGC2655 | NM_024339 |
| 202053_s_at | ALDH3A2 | L47162 | 209834_at | CHST3 | AB017915 |
| 205530_at | ETFDH | NM_004453 | 208189_s_at | MYO7A | NM_000260 |
| 207543_s_at | P4HA1 | NM_000917 | 221658_s_at | IL21R | AF269133 |
| 210465_s_at | SNAPC3 | U71300 | 218382_s_at | U2AF2 | NM_007279 |
| 242903_at | IFNGR1 | AI458949 | 221283_at | RUNX2 | NM_004348 |
| 208012_x_at | SP110 | NM_004509 | 220423_at | PLA2G2D | NM_012400 |
| 203175_at | RHOG | NM_001665 | 230673_at | PKHD1L1 | AV706971 |
| 1565868_at | | W96225 | 243754_at | | AA149736 |
| 201568_at | QP-C | NM_014402 | 210384_at | HRMT1L1 | U79286 |
| 223240_at | FBXO8 | AF201932 | 1565641_at | | BE503823 |
| 228278_at | NFIX | AI817698 | 230499_at | BIRC3 | AA805622 |
| 228115_at | | AW299905 | 221345_at | GPR43 | NM_005306 |
| 242287_at | RSN | AI090487 | 205210_at | TGFBRAP1 | NM_004257 |
| 201241_at | DDX1 | NM_004939 | 221709_s_at | C14orf131 | BC006222 |
| 227176_at | | AL565362 | 1558176_at | | AJ388663 |
| 203964_at | NMI | NM_004688 | 215505_s_at | STRN3 | AF243424 |
| 204821_at | BTN3A3 | NM_006994 | 205765_at | CYP3A5 | NM_000777 |
| 1553677_a_at | MGC3794 | NM_152902 | 224348_s_at | | predicted protein of HQ2605; Homo sapiens PRO2605 mRNA, complete cds. |
| 222530_s_at | MKKS | AF275813 | 212662_at | PVR | BE615277 |
| 238481_at | MGP | AW512787 | 227216_at | LOC146206 | AI560765 |
| 208475_at | FRMD4 | NM_018027 | 213217_at | ADCY2 | AU149572 |
| 212224_at | ALDH1A1 | NM_000689 | 202986_at | ARNT2 | NM_014862 |
| 1568815_a_at | DDX50 | AA903184 | 228056_s_at | NAP1L | AI763426 |
| 240798_at | | BE467916 | 1555268_a_at | GRID1 | BC039263 |
| 228310_at | ENAH | BF223300 | 233498_at | | AK024204 |
| 217984_at | RNASET2 | NM_003730 | 1562398_at | | AA912540 |
| 217106_x_at | HSA9761 | AF091078 | 203421_at | TP53I11 | NM_006034 |
| 209761_s_at | SP110 | AA969194 | 223488_s_at | GNB4 | BC000873 |
| 215075_s_at | GRB2 | L29511 | 219655_at | C7orf10 | NM_024728 |
| 225378_at | FLJ32642 | AI866426 | 223721_s_at | DNAJC12 | AF176013 |
| 203903_s_at | HEPH | NM_014799 | 233568_x_at | CWF19L1 | AK023984 |
| 219373_at | DPM3 | NM_018973 | 220096_at | RNASET2 | NM_017795 |
| 232098_at | BPAG1 | AK025142 | 1556272_a_at | | Clone IMAGE:4830283 mRNA |
| 1552611_a_at | JAK1 | AL555086 | 219463_at | C20orf103 | NM_012261 |
| 218076_s_at | ARHGAP17 | NM_018054 | 225978_at | KIAA1238 | AW409794 |
| 210754_s_at | LYN | M79321 | | | |
| 218422_s_at | C13orf10 | NM_022118 | | | |
| 1558254_s_at | SRPK2 | BU 155802 | | | |

Table 2 provides a list of preferred genes the expression profile of which can differentiate between SLE, MIC, SA, OA or RA. The genes are selected from the 2059 genes differentially expressed between the five conditions (Table 1). Accordingly, one can select all these listed genes to correctly classify the rheumatic condition in an individual as SLE, MIC, SA, OA or RA based on the expression profiles of the genes.

**Table 2**

| **probe set id** | **Gene name** | **GenBank** | **Gene description** |
|---|---|---|---|
| 209292_at | ID4 | AL022726 | |
| 226702_at | LOC129607 | AI742057 | hypothetical protein LOC129607 |
| 203153_at | IFIT1 | NM_001548 | interferon-induced protein with tetratricopeptide repeats 1 |
| 201123_s_at | EIF5A | NM_001970 | eukaryotic translation initiation factor 5A |
| 214702_at | FN1 | AJ276395 | fibronectin 1 |
| 216005_at | TNC | BF434846 | tenascin C (hexabrachion) |
| 210571_s_at | CMAH | AF074480 | MRNA for CMP-N-acetylneuraminic acid hydroxylase, complete cds. |
| 219352_at | FLJ20637 | NM_017912 | |
| 219514_at | ANGPTL2 | NM_012098 | angiopoietin-like 2 |
| 201416_at | SOX4 | BG528420 | SRY (sex determining reqion Y)-box 4 |
| 243462_s_at | | BF590303 | Transcribed sequences |
| 206385_s_at | ANK3 | NM_020987 | ankyrin 3, node of Ranvier (ankyrin G) |
| 235079_at | | AW265065 | Clone IMAGE:3618365, mRNA |
| 218404_at | SNX10 | NM_013322 | sorting nexin 10 |
| 208662_s_at | TTC3 | AI885338 | tetratricopeptide repeat domain 3 |
| 202086_at | MX1 | NM_002462 | myxovirus (influenza virus) resistance 1, interferon-inducible protein p78 (mouse) |
| 221695_s_at | MAP3K2 | AF239798 | mitogen-activated protein kinase kinase kinase 2 |
| 209537_at | EXTL2 | AF000416 | exostoses (multiple)-like 2 |
| 205475_at | SCRG1 | NM_007281 | scrapie responsive protein 1 |
| 204949_at | ICAM3 | NM_002162 | intercellular adhesion molecule 3 |
| 205483_s_at | G1P2 | NM_005101 | interferon, alpha-inducible protein (clone IFI-15K) |
| 224579_at | SLC38A1 | BF247552 | solute carrier family 38, member 1 |
| 212587_s_at | PTPRC | AI809341 | protein tyrosine phosphatase, receptor type, C |
| 213362_at | PTPRD | N73931 | za74a07.s1 Soares_fetal_lung_NbHL19W Homo sapiens cDNA clone IMAGE:298260 3', mRNA sequence. |
| 202409_at | | X07868 | |
| 205255_x_at | TCF7 | NM_003202 | transcription factor 7 (T-cell specific, HMG-box) |
| 215209_at | SEC24D | AU143984 | SEC24 related gene family, member D (S. cerevisiae) |
| 224325_at | FZD8 | AB043703 | frizzled homolog 8 (Drosophila) |
| 204035_at | SCG2 | NM_003469 | secretogranin II (chromogranin C) |
| 204747_at | IFIT4 | NM_001549 | interferon-induced protein with tetratricopeptide |
| | | | repeats 4 |
| 226452_at | PDK1 | AU146532 | pyruvate dehydrogenase kinase, isoenzyme 1 |
| 223843_at | SCARA3 | AB007830 | scavenger receptor class A, member 3 |
| 200757_s_at | CALU | NM_001219 | calumenin |
| 204972_at | OAS2 | NM_016817 | 2'-5'-oligoadenylate synthetase 2, 69/71kDa |
| 200638_s_at | YWHAZ | BC003623 | tyrosine 3-monooxygenase/tryptophan 5-monooxygenase activation protein, zeta polypeptide |
| 235276_at | EPSTI1 | AA781795 | epithelial stromal interaction 1 (breast) |
| 223245_at | STRBP | AK024285 | spermatid perinuclear RNA binding protein |
| 213488_at | FLJ00133 | N73970 | FLJ00133 protein |
| 242738_s_at | ATBF1 | BG402859 | 602418552F1 NIH_MGC_93 Homo sapiens cDNA clone IMAGE:4525500 5', mRNA sequence. |
| 241613_at | | AW296081 | Transcribed sequence with moderate similarity to protein ref:NP_149105.1 (H.sapiens) MADP-1 protein [Homo sapiens] |
| 202307_s_at | TAP1 | NM_000593 | transporter 1, ATP-binding cassette, sub-family B (MDR/TAP) |
| 204439_at | C1orf29 (IFI44L) | NM_006820 | chromosome 1 open reading frame 29 |
| 203794_at | CDC42BPA | NM_014826 | CDC42 binding protein kinase alpha (DMPK-like) |
| 229088_at | | BF591996 | Transcribed sequence with weak similarity to protein ref:NP_060265.1 (H.sapiens) hypothetical protein FLJ20378 [Homo sapiens] |
| 201906_s_at | CTDSPL | NM_005808 | CTD (carboxy-terminal domain, RNA polymerase II, polypeptide A) small phosphatase-like |
| 218353_at | RGS5 | NM_025226 | |
| 236816_at | FLJ13089 | BF110370 | hypothetical protein FLJ13089 |
| 225871_at | STEAP2 | BF680588 | six transmembrane epithelial antigen of prostate 2 |
| 208073_x_at | TTC3 | NM_003316 | tetratricopeptide repeat domain 3 |
| 227609_at | EPSTI1 | AA633203 | epithelial stromal interaction 1 (breast) |
| 204415_at | G1P3 | NM_022873 | interferon, alpha-inducible protein (clone IFI-6-16) |
| 202411_at | IF127 | NM_005532 | interferon, alpha-inducible protein 27 |
| 226571_s_at | PTPRS | N38920 | protein tyrosine phosphatase, receptor type, S |
| 227088_at | | BF221547 | CDNA FLJ42757 fis, clone BRAWH3001712 |
| 227391_x_at | | BE674143 | 7d75g01.x1 NCI_CGAP_Lu24 Homo sapiens cDNA clone IMAGE:3278832 3' similar to contains element MER1 repetitive element, mRNA sequence. |
| 235419_at | | AW612461 | Transcribed sequences |
| 200887_s_at | STAT1 | NM_007315 | signal transducer and activator of transcription 1, 91 kDa |
| AFFX-HUMISGF3A/M9 7935_3_at | STAT1 | | AFFX-HUMISGF3A/M97935_3 |
| 214043_at | PTPRD | BF062299 | protein tyrosine phosphatase, receptor type, D |
| 214336_s_at | COPA | AI621079 | peroxisomal farnesylated protein |
| 1552287_s_at | AFG3L1 | NM_001132 | AFG3 ATPase family gene 3-like 1 (yeast) |
| 225764_at | ETV6 | AI762695 | ets variant gene 6 (TEL oncogene) |
| 227931_at | | AI823917 | Transcribed sequence with moderate similarity to protein pir:I60307 (E. coli) 160307 beta-galactosidase, alpha peptide - Escherichia coli |
| 225664_at | COL12A1 | AA788946 | collagen, type XII, alpha 1 |
| 229450_at | IFIT4 | AI075407 | interferon-induced protein with tetratricopeptide repeats 4 |
| 1555929_s_at | | BM873997 | laa10f11.x1 8 5 week embryo anterior tongue 8 5 EAT Homo sapiens cDNA 3', mRNA sequence. |
| 210645_s_at | TTC3 | D83077 | tetratricopeptide repeat domain 3 |
| 242625_at | cig5 | AW189843 | viperin |
| 209652_s_at | PGF | BC001422 | placental growth factor, vascular endothelial growth factor-related protein |
| 227740_at | KIS | AW173222 | kinase interacting with leukemia-associated gene (stathmin) |
| 204776_at | THBS4 | NM_003248 | thrombospondin 4 |
| 212297_at | AFURS1 | BF218804 | ATPase family homolog up-regulated in senescence cells |
| 244872_at | SYNCOILIN | BE514107 | 601315943F1 NIH_MGC_8 Homo sapiens cDNA clone IMAGE:3634547 5', mRNA sequence. |
| 226137_at | ATBF1 | AI288759 | AT-binding transcription factor 1 |
| 201621_at | NBL1 | NM_005380 | neuroblastoma, suppression of tumorigenicity 1 |
| 209442_x_at | ANK3 | AL136710 | ankyrin 3, node of Ranvier (ankyrin G) |
| 227677_at | JAK3 | BF512748 | Janus kinase 3 (a protein tyrosine kinase, leukocyte) |
| 214701_s_at | FN1 | AJ276395 | fibronectin 1 |
| 227405_s_at | FZD8 | AW340311 | frizzled homolog 8 (Drosophila) |
| 64064_at | IAN4L1 | AI435089 | th95b11.x1 Soares_NSF_F8_9W_OT_PA_P_S1 Homo sapiens cDNA clone IMAGE:2126397 3', mRNA sequence. |
| 208712_at | CCND1 | M73554 | cyclin D1 (PRAD1: parathyroid adenomatosis 1) |
| 201641_at | BST2 | NM_004335 | bone marrow stromal cell antigen 2 |
| 223842_s_at | SCARA3 | AB007830 | scavenger receptor class A, member 3 |
| 236325_at | | BF057799 | LOH11CR1P gene, loss of heterozygosity, 11, chromosomal region 1 gene P product |
| AFFX-HUMISGF3A/M9 7935_MB_at | STAT1 | | AFFX-HUMISGF3A/M97935_MB |
| 220076_at | ANKH | NM_019847 | |
| 1553613_s_at | FOXC1 | NM_001453 | forkhead box C1 |
| 209082_s_at | COL18A1 | AF018081 | collagen, type XVIII, alpha 1 |
| 209969_s_at | STAT1 | BC002704 | signal transducer and activator of transcription 1, 91 kDa |
| 225922_at | KIAA1450 | BE501838 | KIAA1450 protein |
| 200935_at | CALR | NM_004343 | calreticulin |
| 226218_at | IL7R | BE217880 | interleukin 7 receptor |
| 223092_at | ANKH | AA854943 | ankylosis, progressive homolog (mouse) |
| 201417_at | SOX4 | AL136179 | |
| 201009_s_at | TXNIP | AI439556 | thioredoxin interacting protein |
| 242945_at | FAM20A | AI860568 | CDNA FLJ27210 fis, clone SYN03494 |
| 208664_s_at | TTC3 | AU131711 | tetratricopeptide repeat domain 3 |
| 222999_s_at | CCNL2 | AF251294 | cyclin L2 |
| 225924_at | KIAA1450 | AI478634 | KlAA1450 protein |
| 204198_s_at | RUNX3 | AA541630 | runt-related transcription factor 3 |
| 202796_at | SYNPO | NM_007286 | synaptopodin |
| 225644_at | FLJ33814 | BF060776 | 7j60e11.x1 Soares_NSF_F8_9W_OT_PA_P_S1 Homo sapiens cDNA clone IMAGE:3390860 3', mRNA sequence. |
| 1556989_at | | AF086069 | Full length insert cDNA clone YZ35C05 |
| 205931_s_at | CREB5 | NM_004904 | cAMP responsive element binding protein 5 |
| 1556339_a_at | | BM353142 | CDNA FLJ39845 fis, clone SPLEN2014452 |
| 219863_at | CEB1 | NM_016323 | cyclin-E binding protein 1 |
| 213260_at | FOXC1 | AU145890 | forkhead box C1 |
| 223094_s_at | ANKH | AF274753 | ankylosis, progressive homolog (mouse) |
| 205326_at | RAMP3 | NM_005856 | receptor (calcitonin) activity modifying protein 3 |
| 238481_at | MGP | AW512787 | xt77g05.x1 NCI_CGAP_Ut1 Homo sapiens cDNA clone IMAGE:2792504 3' similar to gb:X53331 MATRIX GLA-PROTEIN PRECURSOR (HUMAN); mRNA sequence. |
| 208475_at | FRMD4 | NM_018027 | FERM domain containing 4 |
| 212224_at | ALDH1A1 | NM_000689 | aldehyde dehydrogenase 1 family, member A1 |
| 217984_at | RNASET2 | NM_003730 | ribonuclease T2 |
| 215075_s_at | GRB2 | L29511 | growth factor receptor-bound protein 2 |
| 231130_at | FKBP7 | AA683602 | FK506 binding protein 7 |
| 238081_at | FB14 | AI694300 | CDNA FLJ42672 fis, clone BRAMY2026533 |
| 202862_at | FAH | NM_000137 | fumarylacetoacetate hydrolase (fumarylacetoacetase) |
| 205542_at | STEAP | NM_012449 | six transmembrane epithelial antigen of the prostate |
| 212599_at | AUTS2 | AK025298 | autism susceptibility candidate 2 |
| 227051_at | | AU157716 | CDNA FLJ13585 fis, clone PLACE1009150 |
| 202363_at | SPOCK | AF231124 | sparclosteonectin, cwcv and kazal-like domains proteoglycan (testican) |
| 1558143_a_at | BCL2L11 | AK027160 | BCL2-like 11 (apoptosis facilitator) |
| 203923_s_at | CYBB | NM_000397 | cytochrome b-245, beta polypeptide (chronic granulomatous disease) |
| 209167_at | GPM6B | AI419030 | glycoprotein M6B |
| 213493_at | FLJ00133 | BF509657 | FLJ00133 protein |
| 209417_s_at | IFI35 | BC001356 | interferon-induced protein 35 |
| 201010_s_at | TXNIP | NM_006472 | thioredoxin interacting protein |
| 225381_at | | AW162210 | MRNA; cDNA DKFZp686J24156 (from clone DKFZp686J24156) |
| 213543_at | SGCD | AA570453 | sarcoglycan, delta (35kDa dystrophin-associated glycoprotein) |
| 201008_s_at | TXNIP | AA812232 | thioredoxin interacting protein |
| 223093_at | ANKH | T99215 | ye63a06.s1 Soares fetal liver spleen 1 NFLS Homo sapiens cDNA clone IMAGE:122386 3', mRNA sequence. |
| 211445_x_at | FKSG17 | AF315951 | FKSG17 |
| 218986_s_at | FLJ20035 | NM_017631 | hypothetical protein FLJ20035 |
| 223315_at | NTN4 | AF278532 | netrin 4 |
| 1569472_s_at | TTC3 | BC026260 | tetratricopeptide repeat domain 3 |
| 213001_at | ANGPTL2 | AF007150 | angiopoietin-like 2 |
| 221841_s_at | KLF4 | BF514079 | Kruppel-like factor 4 (gut) |
| 232511_at | | AK022838 | GRIP and coiled-coil domain containing 2 |
| 224701_at | KIAA1268 | AA056548 | KIAA1268 protein |
| 233437_at | GABRA4 | AF238869 | gamma-aminobutyric acid (GABA) A receptor, alpha 4 |
| 242500_at | | T87730 | Transcribed sequences |
| 218543_s_at | ZC3HDC1 | NM_022750 | zinc finger CCCH type domain containing 1 |
| 235003_at | KIS | Al249980 | kinase interacting with leukemia-associated gene (stathmin) |
| 239798_at | | Al825068 | Transcribed sequences |
| 205560_at | PCSK5 | NM_006200 | proprotein convertase subtilisin/kexin type 5 |
| 205552_s_at | OAS1 | NM_002534 | 2',5'-oligoadenylate synthetase 1, 40/46kDa |
| 225353_s_at | C1QG | Al184968 | qe51c05.x1 Soares_fetal_lung_NbHL19W Homo sapiens cDNA clone IMAGE:1742504 3' similar to SW:C1QC_HUMAN P02747 COMPLEMENT C1Q SUBCOMPONENT, C CHAIN PRECURSOR. ;, mRNA sequence. |
| 235902_at | | Al090764 | qa65f01.x1 Soares_fetal_heart_NbHH19W Homo sapiens cDNA clone IMAGE:1691641 3', mRNA sequence. |
| 227908_at | KIAA1171 | BG236006 | KIAA1171 protein |
| 220266_s_at | KLF4 | NM_004235 | Kruppel-like factor 4 (gut) |
| 217749_at | COPG | NM_016128 | coatomer protein complex, subunit gamma |
| 207277_at | CD209 | AF290886 | CD209 antigen |
| 203700_s_at | DIO2 | NM_013989 | deiodinase, iodothyronine, type II |
| 215296_at | CDC42BPA | AK027000 | CDC42 binding protein kinase alpha (DMPK-like) |
| 207339_s_at | LTB | NM_002341 | lymphotoxin beta (TNF superfamily, member 3) |
| 215314_at | ANK3 | AU146646 | AU146646 HEMBB1 Homo sapiens cDNA clone HEMBB1001096 3', mRNA sequence. |
| 218400_at | OAS3 | NM_006187 | 2'-5'-oligoadenylate synthetase 3,100kDa |
| 231776_at | EOMES | NM_005442 | eomesodermin homolog (Xenopus laevis) |
| 229686_at | P2RY8 | AI436587 | ti03d11.x1 NCI_CGAP_CLL1 Homo sapiens CDNA clone IMAGE:2129397 3' similar to contains Alu repetitive element; contains element MER22 repetitive element;, mRNA sequence. |
| 214033_at | ABCC6 | AI084637 | ATP-binding cassette, sub-family C (CFTR/MRP), member 6 |
| 235385_at | FLJ20668 | AI935334 | hypothetical protein FLJ20668 |
| 204197_s_at | RUNX3 | NM_004350 | runt-related transcription factor 3 |
| 207734_at | LAX | NM_017773 | hypothetical protein FLJ20340 |
| 238418_at | SLC35B4 | AI590926 | solute carrier family 35, member B4 |
| 212394_at | KIAA0090 | D42044 | KIAA0090 protein |
| 223316_at | CCDC3 | AL136562 | coiled-coil domain containing 3 |
| 33646_g_at | GM2A | X61094 | GM2 ganglioside activator protein |
| 208914_at | GGA2 | BE646414 | golgi associated, gamma adaptin ear containing, ARF binding protein 2 |
| AFFX-HUMISGF3A/M9 7935_MA_at | STAT1 | | AFFX-HUMISGF3A/M97935_MA |
| 242616_at | | W80359 | zh49a08.s1 Soares_fetal_liver_spleen_1NFLS_S1 Homo sapiens cDNA clone IMAGE:415382 3', mRNA sequence. |
| 1567706_at | | AF009316 | Homo sapiens clone TUB2 Cri-du-chat region mRNA. |
| 235924_at | | N73742 | CDNA FLJ42287 fis, clone TLIVE2005866 |
| 214560_at | FPRL2 | NM_002030 | formyl peptide receptor-like 2 |
| 206181_at | SLAMF1 | NM_003037 | signaling lymphocytic activation molecule family member 1 |
| 223389_s_at | ZNF581 | AF151023 | zinc finger protein 581 |
| 232668_at | | AW903934 | CDNA FLJ11528 fis, clone HEMBA1002621 |
| 217629_at | | AA365670 | Transcribed sequence with strong similarity to protein ref:NP_057175.1 (H.sapiens) HSPC025; eIEF associated protein HSPC021 [Homo sapiens] |
| 240721_at | DBC-1 | BE672858 | p30 DBC protein |
| 222895_s_at | BCL11 B | AA918317 | B-cell CLL/lymphoma 11 B (zinc finger protein) |
| 215992_s_at | RAPGEF2 | AL117397 | Rap guanine nucleotide exchange factor (GEF) 2 |
| 235355_at | | AL037998 | CDNA FLJ30740 fis, clone FEBRA2000319 |
| 229435_at | ZNF515 | AW025602 | zinc finger protein 515 |
| 243366_s_at | RP26 | AI936034 | wo47c10.x1 NCI_CGAP_Gas4 Homo sapiens CDNA clone IMAGE:2458482 3', mRNA sequence. |
| 239725_at | | T90703 | Transcribed sequences |
| 204345_at | COL16A1 | NM_001856 | collagen, type XVI, alpha 1 |
| 238142_at | | AW029203 | Transcribed sequence with moderate similarity to protein ref:NP_060312.1 (H.sapiens) hypothetical protein FLJ20489 [Homo sapiens] |
| 236295_s_at | NOD3 | AA694067 | NOD3 protein |
| 214038_at | CCL8 | AI984980 | wr88g11.x1 NCI_CGAP_Kid11 Homo sapiens CDNA clone IMAGE:2494820 3' similar to SW:MCP2_HUMAN P80075 MONOCYTE CHEMOTACTIC PROTEIN 2 PRECURSOR;, mRNA sequence. |
| 1558385_at | | AL832806 | MRNA; cDNA DKFZp667O1523 (from clone DKFZp667IO523) |
| 227533_at | | AA732944 | zg78d04.s1 Soares_fetal_heart_NbHH19W Homo sapiens cDNA clone IMAGE:399463 3', mRNA sequence. |
| 219014_at | PLAC8 | NM_016619 | placenta-specific 8 |
| 215891_s_at | GM2A | X61094 | GM2 ganglioside activator protein |
| 223680_at | ZNF607 | BC005085 | hypothetical protein MGC13071 |
| 205018_s_at | MBNL2 | NM_005757 | muscleblind-like 2 (Drosophila) |
| 222891_s_at | BCL11A | AI912275 | B-cell CLL/ymphoma 11A (zinc finger protein) |
| 233302_at | | AU146285 | CDNA FLJ10224 fis, clone HEMBB1000025 |
| 40837_at | TLE2 | M99436 | transducin-like enhancer of split 2 (E(sp1) homolog, Drosophila) |
| 206236_at | GPR4 | NM_005282 | G protein-coupled receptor 4 |
| 204562_at | IRF4 | NM_002460 | interferon regulatory factor 4 |
| 236285_at | LOC113730 | AI631846 | hypothetical protein BC009980 |
| 229176_at | ANKH | AI672354 | ankylosis, progressive homolog (mouse) |
| 210279_at | GPR18 | AF261135 | G protein-coupled receptor 18 |
| 214032_at | ZAP70 | AI817942 | zeta-chain (TCR) associated protein kinase 70kDa |
| 204891_s_at | LCK | NM_005356 | lymphocyte-specific protein tyrosine kinase |
| 1562953_s_at | FBI4 | BC019264 | CDNA FLJ42672 fis, clone BRAMY2026533 |
| 206439_at | DSPG3 | NM_004950 | dermatan sulfate proteoglycan 3 |
| 240159_at | SLC15A2 | AA836116 | solute carrier family 15 (H+/peptide transporter), member 2 |
| 206574_s_at | PTP4A3 | NM_007079 | protein tyrosine phosphatase type IVA, member 3 |
| 235643_at | FLJ39885 | BE886225 | hypothetical protein FLJ39885 |
| 33132_at | CPSF1 | U37012 | cleavage and polyadenylation specific factor 1, 160kDa |
| 237753_at | | AW504569 | Transcribed sequence with moderate similarity to protein ref:NP_113661.1 (H.sapiens) hypothetical protein MGC4027 [Homo sapiens] |
| 1559814_at | | AK024712 | CDNA: FLJ21059 fis, clone CAS00740 |
| 236782_at | SAMD3 | AI129628 | sterile alpha motif domain containinq 3 |
| 212105_s_at | DHX9 | BF313832 | DEAH (Asp-Glu-Ala-His) box polypeptide 9 |
| 1565705_x_at | | AK025048 | CDNA: FLJ21395 fis, clone COL03557 |
| 207216_at | TNFSF8 | NM_001244 | tumor necrosis factor (ligand) superfamily, member 8 |
| 234563_at | | AK000795 | Homo sapiens cDNA FLJ20788 fis, clone COL02074. |
| 206553_at | OAS2 | NM_002535 | 2'-5'-oligoadenylate synthetase 2, 69171 kDa |
| 203413_at | NELL2 | NM_006159 | NEL-like 2 (chicken) |
| 236280_at | | AI225238 | Transcribed sequences |
| 227245_at | FLJ13089 | AW511198 | hypothetical protein FLJ13089 |
| 237625_s_at | | BG548679 | Immunoglobulin kappa light chain mRNA, partial cds |
| 222909_s_at | BAG4 | AF111116 | BCL2-associated athanogene 4 |
| 207651_at | H963 | NM_013308 | platelet activating receptor homolog |
| 243365_s_at | AUTS2 | AI417756 | Transcribed sequence with strong similarity to protein ref:NP_056385.1 (H.sapiens) autism-related protein 1 [Homo sapiens] |
| 1555464_at | MDA5 | BC046208 | melanoma differentiation associated protein-5 |
| 224998_at | CKLFSF4 | AK000855 | chemokine-like factor super family 4 |
| 238784_at | FLJ32949 | AI039361 | hypothetical protein FLJ32949 |
| 217147_s_at | TRIM | AJ240085 | T-cell receptor interacting molecule |
| 205993_s_at | TBX2 | NM_005994 | T-box 2 |
| 241401_at | FBI4 | BG496631 | CDNA FLJ42672 fis, clone BRAMY2026533 |
| 203311_s_at | ARF6 | M57763 | ADP-ribosylation factor 6 |
| 221601_s_at | TOSO | AI084226 | regulator of Fas-induced apoptosis |
| 229829_at | C18orf18 | AA429735 | hypothetical protein MGC17515 |
| 204852_s_at | PTPN7 | NM_002832 | protein tyrosine phosphatase, non-receptor type 7 |
| 225566_at | NRP2 | AI819729 | neuropilin 2 |
| 205242_at | CXCL13 | NM_006419 | chemokine (C-X-C motif) ligand 13 (B-cell chemoattractant) |
| 243065_at | | AA809449 | Transcribed sequences |
| 242288_s_at | EMILIN2 | AL552384 | elastin microfibril interfacer 2 |
| 205671_s_at | HLA-DOB | NM_002120 | major histocompatibility complex, class II, DO beta |
| 237496_at | | AI821404 | Transcribed sequence with weak similarity to protein ref:NP_071431.1 (H.sapiens) cytokine receptor-like factor 2; cytokine receptor CRL2 precusor [Homo sapiens] |
| 210163_at | CXCL11 | AF030514 | chemokine (C-X-C motif) ligand 11 |
| 219944_at | FLJ21069 | NM_024692 | hypothetical protein FLJ21069 |
| 236777_at | | AA854843 | CDNA clone IMAGE:5287486, partial cds |
| 236261_at | OSBPL6 | AI949389 | oxysterol binding protein-like 6 |
| 1569830_at | PTPRC | BC031525 | protein tyrosine phosphatase, receptor type, C |
| 229038_at | CWF19L1 | BF939646 | nac82a07.x1 NCI_CGAP_Brn23 Homo sapiens cDNA clone IMAGE:3440557 3' similar to contains Alu repetitive element;, mRNA sequence. |
| 210797_s_at | OASL | AF063612 | interferon-induced protein; 30 kDa; alternatively spliced; Homo sapiens 2'-5'oligoadenylate synthetase-related protein p30 (OASL) mRNA, alternatively spliced, complete cds. |
| 1563792_at | AMN | AK092824 | amnionless homolog (mouse) |
| 200917_s_at | SRPR | BG474541 | signal recognition particle receptor ('docking protein') |
| 1555214_a_at | CLECSF12 | AF400602 | C-type (calcium dependent, carbohydrate-recognition domain) lectin, superfamily member 12 |
| 207348_s_at | LIG3 | NM_002311 | ligase III, DNA, ATP-dependent |
| 223667_at | FKBP7 | AF092137 | FK506 binding protein 7 |
| 231628_s_at | | AW262311 | xq64a07.x1 NCI_CGAP_HN9 Homo sapiens cDNA clone IMAGE:2755380 3', mRNA sequence. |
| 213797_at | cig5 | AI337069 | viperin |
| 205049_s_at | CD79A | NM_001783 | CD79A antigen (immunoglobulin-associated alpha) |
| 211122_s_at | CXCL11 | AF002985 | chemokine (C-X-C motif) ligand 11 |
| 226299_at | PKN3 | NM_013355 | protein kinase PKNbeta |
| 230894_s_at | MSI2 | BE672557 | musashi homolog 2 (Drosophila) |
| 209611_s_at | SLC1A4 | AB026689 | solute carrier family 1 (glutamate/neutral amino acid transporter), member 4 |
| 204960_at | PTPRCAP | NM_005608 | protein tyrosine phosphatase, receptor type, C-associated protein |
| 1562236_at | MYST4 | AL832065 | MYST histone acetyltransferase (monocytic leukemia) 4 |
| 241525_at | LOC200772 | AV700191 | Clone IMAGE:5745636, mRNA |
| 236117_at | | AA706701 | Transcribed sequences |
| 203532_x_at | CUL5 | AF017061 | cullin 5 |
| 228326_at | MGC43690 | AI016894 | hypothetical protein MGC43690 |
| 211876_x_at | PCDHGA11 | AF152504 | protocadherin gamma subfamily C, 3 |
| 218843_at | FNDC4 | NM_022823 | fibronectin type III domain containing 4 |
| 219497_s_at | BCL11A | NM_022893 | B-cell CLL/lymphoma 11A (zinc finger protein) |
| 236301_at | | AA789123 | CDNA FLJ46579 fis, clone THYMU3042758 |
| 232442_at | | AU147442 | CDNA FLJ45059 fis, clone BRAWH3023274 |
| 211102_s_at | LILRA2 | U82277 | leukocyte immunoglobulin-like receptor, subfamily B (with TM and ITIM domains), member 1 |
| 213830_at | TRD@ | AW007751 | T cell receptor delta locus |
| 221212_x_at | PB1 | NM_018313 | polybromo 1 |
| 222727_s_at | SLC24A6 | AI339568 | solute carrier family 24 (sodium/potassium/calcium exchanger), member 6 |
| 209727_at | GM2A | M76477 | GM2 ganglioside activator protein |
| 39318_at | TCL1A | X82240 | T-cell leukemia/lymphoma 1A |
| 243375_at | | AI742685 | Transcribed sequences |
| 244144_at | SYNE1 | N52270 | spectrin repeat containing, nuclear envelope 1 |
| 220233_at | FBXO26 | NM_024907 | F-box only protein 26 |
| 216000_at | KIAA0484 | AA732995 | CDNA FLJ39257 fis, clone OCBBF2009115 |
| 1552656_s_at | KIS | NM_144624 | kinase interacting with leukemia-associated gene (stathmin) |
| 206313_at | HLA-DOA | NM_002119 | major histocompatibility complex, class II, DO alpha |
| 236341_at | CTLA4 | AI733018 | cytotoxic T-lymphocyte-associated protein 4 |
| 232286_at | | AA572675 | CDNA FLJ12187 fis, clone MAMMA1000831 |
| 208189_s_at | MYO7A | NM_000260 | myosin VIIA (Usher syndrome 1 B (autosomal recessive, severe)) |
| 221283_at | RUNX2 | NM_004348 | runt-related transcription factor 2 |
| 230673_at | PKHD1L1 | AV706971 | polycystic kidney and hepatic disease 1 (autosomal recessive)-like 1 |
| 210384_at | HRMT1L1 | U79286 | HMT1 hnRNP methyltransferase-like 1 (S. cerevisiae) |
| 1565641_at | | BE503823 | hv83e07.x1 NCI_CGAP_Lu24 Homo sapiens CDNA clone IMAGE:3180036 3' similar to contains element LTR1 repetitive element ;, mRNA sequence. |
| 221345_at | GPR43 | NM_005306 | G protein-coupled receptor 43 |
| 205210_at | TGFBRAP1 | NM_004257 | transforming growth factor, beta receptor associated protein 1 |
| 221709_s_at | C14orf131 | BC006222 | chromosome 14 open reading frame 131 |
| 215505_s_at | STRN3 | AF243424 | striatin, calmodulin binding protein 3 |
| 205765_at | CYP3A5 | NM_000777 | cytochrome P450, family 3, subfamily A, polypeptide 5 |
| 224348_s_at | | AF116709 | predicted protein of HQ2605; Homo sapiens PRO2605 mRNA, complete cds. |
| 212662_at | PVR | BE615277 | poliovirus receptor |
| 202986_at | ARNT2 | NM_014862 | aryl-hydrocarbon receptor nuclear translocator 2 |
| 228056_s_at | NAP1L | AI763426 | napsin B pseudogene |
| 1555268_a_at | GRID1 | BC039263 | glutamate receptor, ionotropic, delta 1 |
| 233498_at | | AK024204 | CDNA FLJ14142 fis, clone MAMMA1002880 |
| 1562398_at | | AA912540 | ol36b01.s1 Soares_NFL_T_GBC_S1 Homo sapiens cDNA clone IMAGE:1525513 3' similar to contains MER2.b2 MER2 repetitive element ;, mRNA sequence. |
| 203421_at | TP53I11 | NM_006034 | tumor protein p53 inducible protein 11 |
| 223488_s_at | GNB4 | BC000873 | guanine nucleotide binding protein (G protein), beta polypeptide 4 |
| 219655_at | C7orf10 | NM_024728 | chromosome 7 open reading frame 10 |
| 223721_s_at | DNAJC12 | AF176013 | J domain containing protein 1 |
| 233568_x_at | CWF19L1 | AK023984 | CWF19-like 1, cell cycle control (S. pombe) |
| 220096_at | RNASET2 | NM_017795 | |
| 1556272_a_at | | BC042472 | Clone IMAGE:4830283, mRNA |
| 219463_at | C20orf103 | NM_012261 | chromosome 20 open reading frame 103 |

Table 3 provides a list of preferred genes the expression profile of which can discriminate between SLE, MIC, SA, OA or RA. Accordingly, one can select all these listed genes to correctly classify the rheumatic condition in an individual as SLE, MIC, SA, OA or RA based on the expression profiles of the genes.

**Table 3**

| **Probe Set ID** | **Gene name** | **GeneBank** | **Gene description** |
|---|---|---|---|
| 1405_i_at | CCL5 | M21121 | chemokine (C-C motif) ligand 5 |
| 1552329_at | RBBP6 | BC029352 | retinoblastoma binding protein 6 |
| 1554153_a_at. | PHF21A/BHC80 | BC015714 | PHD finger protein 21A |
| 1555154_a_at | QKI | AF142421 | quaking homolog, KH domain RNA binding (mouse) |
| 1555268_a_at | GRID1 | BC039263 | glutamate receptor, ionotropic, delta 1 |
| 1565705_x_at | FLJ21395fis | CDNA: FLJ21395 fis, clone COL03557 | CDNA: FLJ21395 fis, clone COL03557 |
| 200089_s_at | RPL4 | AI953886 | ribosomal protein L4 |
| 200680_x_at | HMGB1 | NM_002128 | high-mobility group box 1 |
| 200757_s_at | CALU | NM_001219 | calumenin |
| 200795_at | SPARCL1 | NM_004684 | SPARC-like 1 (mast9, hevin) |
| 200972_at | TSPAN3/TM4SF8 | BC000704 | tetraspanin 3 |
| 201154_x_at | RPL4 | NM_000968 | ribosomal protein L4 |
| 201503_at | G3BP1 | BG500067 | GTPase activating protein (SH3 domain) binding protein 1 |
| 201621_at | NBL1 | NM_005380 | neuroblastoma, suppression of tumorigenicity 1 |
| 201906_s_at | CTDSPL | NM_005808 | CTD (carboxy-terminal domain, RNA polymerase II, polypeptide A) small phosphatase-like |
| 202411_at | IFI27 | NM_005532 | interferon, alpha-inducible protein 27 |
| 203413_at | NELL2 | NM_006159 | NEL-like 2 (chicken) |
| 203794_at | CDC42BPA | NM_014826 | CDC42 binding protein kinase alpha (DMPK-like) |
| 204258_at | CHD1 | NM_001270 | chromodomain helicase DNA binding protein 1 |
| 204415_at | IFI6/G1P3 | NM_022873 | interferon, alpha-inducible protein 6 |
| 204529_s_at | TOX | AI961231 | thymocyte selection-associated high mobility group box |
| 204747 at | IFIT3/IFIT4 | NM_001549 | interferon-induced protein with tetratricopeptide repeats 3/4 |
| 204852_s_at | PTPN7 | NM_002832 | protein tyrosine phosphatase, non-receptor type 7 |
| 204891_s_at | LCK | NM_005356 | lymphocyte-specific protein tyrosine kinase |
| 205049 s at | CD79A | NM_001783 | CD79a molecule, immunoglobulin-associated alpha |
| 205552_s_at | OAS1 | NM_002534 | 2',5'-oligoadenylate synthetase 1, 40/46kDa |
| 205590_at | RASGRP1 | NM_005739 | RAS guanyl releasing protein 1 (calcium and DAG-regulated) |
| 205798_at | IL7R | NM_002185 | interleukin 7 receptor |
| 206236_at | GPR4 | NM_005282 | G protein-coupled receptor 4 |
| 207216 at | TNFSF8 | NM_001244 | tumor necrosis factor (ligand) superfamily, member 8 |
| 208475_at | FRMD4A | NM_018027 | FERM domain containinq 4A |
| 208677_s_at | BSG | AL550657 | basigin (Ok blood group) |
| 208712_at | CCND1 | M73554 | cyclin D1 |
| 208804_s_at | SFRS6 | AL031681 | splicing factor, arginine/serine-rich 6 |
| 208819_at | RAB8A | BC002977 | RAB8A, member RAS oncogene family |
| 209379_s_at | KIAA1128 | AF241785 | KIAA1128 |
| 209406_at | BAG2 | AF095192 | BCL2-associated athanogene 2 |
| 209537_at | EXTL2 | AF000416 | exostoses (multiple)-like 2 |
| 209652_s_at | PGF | BC001422 | placental growth factor, vascular endothelial growth factor-related protein |
| 209932_s_at | DUT | U90223 | dUTP pyrophosphatase |
| 210140_at | CST7 | AF031824 | cystatin F (leukocystatin) |
| 211275_s_at | GYG1 | AF087942 | glycogenin 1 |
| 211710_x_at | RPL4 | BC005817 | ribosomal protein L4 |
| 212394_at | KIAA0090 | D42044 | KIAA0090 |
| 212615_at | CHD9/FLJ12178 | AI742305 | chromodomain helicase DNA binding protein 9 |
| 212725_s_at | TI-227H/// TUG1 | N37081 | hypothetical protein TI-227H /// taurine upregulated gene 1 |
| 213193_x_at | TRBC1/ // TRBV19 | T cell receptor beta chain BV20S1 BJ1-5 BC1 mRNA, complete cds | T cell receptor beta variable 19 /// T cell receptor beta constant 1 |
| 213797_at | RSAD2/cig5 | AI337069 | radical S-adenosyl methionine domain containing 2 |
| 214701_s_at | FN1 | AJ276395 | fibronectin 1 |
| 215992_s_at | RAPGEF2 | AL117397 | Rap guanine nucleotide exchange factor (GEF) 2 |
| 216306_x_at | PTBP1 | X62006 | polypyrimidine tract binding protein 1 |
| 216920_s_at | TARP /// TRGC2 /// TRGV9 | M27331 | T cell receptor gamma constant 2 /// T cell receptor gamma variable 9 /// TCR gamma alternate reading frame protein |
| 217719_at | EIF3EIP/EIF3S6IP | NM_016091 | eukaryotic translation initiation factor 3, subunit E interacting protein |
| 217795_s_at | TMEM43/MGC3222 | W74580 | transmembrane protein 43 |
| 217815_at | SUPT16H | NM_007192 | suppressor of Ty 16 homolog (S. cerevisiae) |
| 217817 at | ARPC4 | BE891920 | actin related protein 2/3 complex, subunit 4, 20kDa |
| 217945_at | BTBD1 | NM_025238 | BTB (POZ) domain containing 1 |
| 218353_at | RGS5 | NM_025226 | regulator of G-protein signaling 5 |
| 218533_s_at | UCKL1/URKL1 | NM_017859 | uridine-cytidine kinase 1-like 1 |
| 218543_s_at | PARP12/ZC3HDC1 | NM_022750 | poly (ADP-ribose) polymerase family, member 12 |
| 219528_s_at | BCL11 B | NM_022898 | B-cell CLL/lymphoma 11 B (zinc finger protein) |
| 221709_s_at | C14orf131 | BC006222 | chromosome 14 open reading frame 131 |
| 221830_at | RAP2A | AI302106 | RAP2A, member of RAS oncogene family |
| 222440_s_at | THRAP3 | AL576205 | thyroid hormone receptor associated protein 3 |
| 222667_s_at | ASH1L | Al806500 | ash1 (absent, small, or homeotic)-like (Drosophila) |
| 222728_s_at | JOSD3/MGC5306 | AF275800 | Josephin domain containing 3 |
| 222895_s_at | BCL11 B | AA918317 | B-cell CLUlymphoma 11 B (zinc finger protein) |
| 225363_at | PTEN | AK024986 | phosphatase and tensin homolog (mutated in multiple advanced cancers 1) |
| 225764_at | ETV6 | Al762695 | ets variant gene 6 (TEL oncogene) |
| 225922_at | KIAA1450 | BE501838 | KIAA1450 protein |
| 226137_at | ZFHX3/ATBF1 | Al288759 | zinc finger homeobox 3/AT-binding transcription factor 1 |
| 226218_at | IL7R | BE217880 | interleukin 7 receptor |
| 226409_at | TBC1D20/C20orf140 | BE349614 | TBC1 domain family, member 20 |
| 226702_at | LOC129607 | Al742057 | hypothetical protein LOC129607 |
| 226845_s_at | MYEOV2/LOC150678 | AL036350 | myeloma overexpressed 2 |
| 226910_at | AW008502 | AW008502 | CDNA FLJ30661 fis, clone DFNES2000526 |
| 227030_at | BG231773 | BG231773 | Full length insert cDNA clone YY82H04 |
| 227088_at | PDE5A | BF221547 | phosphodiesterase 5A, cGMP-specific/CDNA FLJ42757 fis, clone BRAWH3001712 |
| 227224_at | RALGPS2 | AW003297 | Ral GEF with PH domain and SH3 binding motif 2 |
| 227677_at | JAK3 | BF512748 | Janus kinase 3 (a protein tyrosine kinase, leukocyte) |
| 227894_at | WDR90/LOC197336 | AL043021 | WD repeat domain 90 |
| 227908_ at | TBC1D24/KIAA1171 | BG236006 | TBC1 domain family, member 24/KIAA1171 protein |
| 228760_at | SFRS2B/SRP46 | AV725947 | splicing factor, arginine/serine-rich 2B |
| 229120_s_at | CDC42SE1/SPEC1 | BG150636 | CDC42 small effector 1 |
| 229450_at | IFIT3/IFIT4 | Al075407 | interferon-induced protein with tetratricopeptide repeats 3/interferon-induced protein with tetratricopeptide repeats 4 |
| 229629_at | Al923633 | Al923633 | Transcribed locus |
| 235419_at | AW612461 | AW612461 | Transcribed locus |
| 236293_at | BE676335 | BE676335 | Transcribed locus |
| 236325_at | KIAA1377 | BF057799 | KIAA1377 |
| 236816_at | C12orf301/FLJ13089 | BF110370 | chromosome 12 open reading frame 30/hypothetical protein FLJ13089 |
| 240159_at | SLC15A2 | AA836116 | solute carrier family 15 (H+/peptide transporter), member 2 |
| 241613_at | AW296081 | AW296081 | Transcribed locus, weakly similar to XP_001083900.1 similar to zinc finger CCHC-type and RNA binding motif 1 [Macaca mulatta] |
| 241905_at | PIK3C2A | AA579047 | Phosphoinositide-3-kinase, class 2, alpha-polypeptide |
| 242500_at | T87730 | T87730 | Transcribed locus |
| 242625_at | RSAD2/cig5 | AW189843 | radical S-adenosyl methionine domain containing 2/viperin |
| 242738_s_at | ZFHX3/ATBF1 | BG402859 | zinc finger homeobox 3/602418552F1 NIH_MGC_93 Homo sapiens cDNA clone IMAGE:4525500 5', mRNA sequence |
| 244872_at | SYNCOILIN | BE514107 | CDNA FLJ30297 fis, clone BRACE2003168/601315943F1 NIH_MGC_8 Homo sapiens cDNA clone IMAGE:3634547 5', mRNA sequence |
| 33132_at | CPSF1 | U37012 | cleavage and polyadenylation specific factor 1, 160kDa |
| 37831_at | SIPA1L3 | AB011117 | signal-induced proliferation-associated 1 like 3 |
| 40837_at | TLE2 | M99436 | transducin-like enhancer of split 2 (E(sp1) homolog, Drosophila) |

Table 4 provides a list of preferred genes the expression profile of which can differentiate between SLE, MIC, SA, OA or RA. Accordingly, one can select these 20 listed genes to correctly classify the rheumatic condition in an individual as SLE, MIC, SA, OA or RA based on the expression profiles of these genes.

**Table 4**

| **Probe Set ID** | **Gene name** | **GenBank** | **Gene description** |
|---|---|---|---|
| 1565705_x_at | FLJ21395 fis | | CDNA: FLJ21395 fis, clone COL03557 |
| 1569472_s_at | TTC3 | BC026260 | tetratricopeptide repeat domain 3 |
| 203413_at | NELL2 | NM_006159 | NEL-like 2 (chicken) |
| 204852_s_at | PTPN7 | NM_002832 | protein tyrosine phosphatase, non-receptor type 7 |
| 206313_at | HLA-DOA | NM_002119 | major histocompatibility complex, class II, DO alpha |
| 207651_at | GPR171/H963 | NM_013308 | G protein-coupled receptor 171/platelet activating receptor homolog |
| 214336_s_at | COPA | Al621079 | coatomer protein complex, subunit alpha |
| 216000_at | KIAA0484 | AA732995 | KIAA0484 protein |
| 217147_s_at | TRAT1/TRIM | AJ240085 | T cell receptor associated transmembrane adaptor 1/T-cell receptor interacting molecule |
| 218843_at | FNDC4 | NM_022823 | fibronectin type III domain containing 4 |
| 219528_s_at | BCL11B | NM_022898 | B-cell CLL/lymphoma 11 B (zinc finger protein) |
| 221709_s_at | C14orf131 | BC006222 | chromosome 14 open reading frame 131 |
| 223667_at | FKBP7 | AF092137 | FK506 binding protein 7 |
| 227908_at | TBC1D24//KIAA1171 | BG236006 | TBC1 domain family, member 24/KIAA1171 protein |
| 235355_at | AL037998 | 4L037998 | MRNA; cDNA DKFZp564E143 (from clone DKFZp564E143) |
| 236280_at | Al225238 | Al225238 | Transcribed locus |
| 236285_at | LOC113730 | Al631846 | Al631846 |
| 236301_at | AA789123 | AA789123 | Full length insert cDNA clone YY82H04 |
| 236325_at | KIAA1377 | BF057799 | KIAA1377 |
| 237753_at | AW504569 | AW504569 | Transcribed locus |

### Examples

### Example 1 Identification of distinct molecular signatures characterizing the five disorders: SLE, RA, OA, SA and MIC

### Patients and synovial biopsies:

Synovial biopsies were obtained by needle-arthroscopy from the knee of patients with SLE (n=4), RA (n=7), OA (n=5), MIC (n=5) and SA (n=4). For each patient, 4 to 8 synovial samples were snap frozen in liquid nitrogen and stored at -80° for later RNA extraction.

The same amount of tissue was also kept at -80° for future immunostaining experiments on frozen sections. The remaining material was stored in formaldehyde and paraffin embedded for conventional optical evaluation and immunostaining of selected cell markers. All SLE patients met the American College of Rheumatology (ACR) revised criteria for the diagnosis of systemic lupus; they all were females and were average 32.0 year-old (range 19-40 year). All of them had active articular disease at the time of synovial tissue sampling. None of the SLE patients was treated with immunosuppressive therapy; some of them were on non-steroidal anti-inflammatory drugs. All RA patients met the ACR criteria for the diagnosis of rheumatoid arthritis. They all had early (< 1 year duration) active disease at the time of tissue sampling. They were 2 females and 5 males. They were average 51 year-old (range 37-69 year). Average CRP level was 25 mg/l (range 9-96 mg/l) and average DAS28-CRP score was 5.08 (range 3.76-5.82). They were not treated except with non-steroidal anti-inflammatory drugs. OA individuals were 4 females and 1 male; their average age was 63.2 year (range 51-73 year). All of them had a swollen knee at the time of the needle-arthroscopic procedure. Similarly, MIC and SA individuals were untreated had the time of the needle-arthroscopic procedure and they had a swollen knee. The study was approved by the ethical committee of the Université catholique de Louvain, and informed consent was obtained from all patients.

### Microarray hybridization and statistical interpretation:

Total RNA was extracted from the synovial biopsies using the Nucleospin® RNA II extraction kit (Macherey-Nagel GmbH & Co, Düren, Germany), including DNase treatment of the samples. Labeling of RNA (cRNA synthesis) was performed according to a standard Affymetrix® procedure (One-Cycle Target Labeling kit, Affymetrix UK Ltd, High Wycombe, United Kingdom); briefly total RNA was first reverse transcribed into single-stranded cDNA using a T7-Oligo(dT) Promoter Primer and Superscript II reverse transcriptase. Next, RNase H was added together with E. Coli DNA polymerase I and E. Coli DNA ligase, followed by a short incubation with T4 DNA polymerase in order to achieve synthesis of the second-strand cDNA. The double-stranded cDNA was purified and served as a template for the overnight in vitro transcription reaction, carried out in the presence of T7 RNA polymerase and a biotinylated nucleotide analog/ribonucleotide mix. At the end of this procedure, the biotinylated complementary RNA (cRNA) was cleaned, and fragmented by a 35 minute incubation at 95°c.

GeneChip® Human genome U133 Plus 2.0 Arrays were hybridized overnight at 45°c in monoplicates with 10 µg cRNA. The slides were then washed and stained using the EukGE-WS2v5 Fluidics protocol on the Genechip® Fluidics Station (Affymetrix) before being scanned on a Genechip® Scanner 3000. Data were retrieved on GCOS software for the initial normalization and analysis steps. The number of positive genes was between 48 and 55% on each slide. After scaling on all probe set (to a value of 100), the amplification scale was reported between 1.1 and 2.5 for all the slides. The signals given by the poly-A RNA controls, hybridization controls and housekeeping/control genes (GAPDH 3'/5' ratio < 2) were indicative of the good quality of the amplification and hybridization procedures. Further statistical analyses were performed using the Genespring® software (Agilent Technologies Inc). For each slide, scaled data were normalized to the 50th percentile per chip and to the median per gene. The data were analyzed by ANOVA with or without Benjamini-Hochberg corrections for multiple comparisons, with a minimal fold change between RA or SLE versus OA set at two. Around 2059 genes displayed differences in expression patterns (Table 1) and can be used as synovial markers for the present invention that are useful for characterizing the five disorders.. At least 310 probe set for 264 genes that displayed significant differences in expression patterns among the samples were selected (Table 2). Supervised gene clustering studies were performed using these genes on Genesis® Software (Genesis 1.0, developed par Bioinformatics Grazand) and on TMEV 3.1 Software (TIGR Multiple Array Viewer) from The Institute for Genomic Research (TIGR). Complete linkage clustering was performed based on Pearson correlations between the selected genes. In another experiment, at least 100 probe sets that displayed significant differences in expression patterns among the samples were selected (Table 3).

GeneChip HGU133 Plus 2.0 is a high-density oligonucleotide spotted array covering the whole genome (about 50 000 genes). Its use for a diagnostic test is inappropriate because of the high cost of the procedure and the noise caused by the high number of genes. The invention relates to the development of a customized low-density array that can make a diagnosis based on the evaluation of the expression of a low number of selected genes (Table 1, 2, 3 and 4). The selection of the genes and the development of a customized array allow to improve sensitivity and specificity (as compared to a high density array) and to lower the cost, making possible its introduction into clinical practice.

### Results:

Supervised clustering of the samples first distributed the samples into two clusters made of SLE and RA versus OA, SA and MIC samples. Inside the two groups, the samples also correctly clustered according to the diagnosis thereby indicating that the five disorders are characterized by distinct molecular signatures. The clustering results are shown in Figure 1.

### Example 2

A 53-year-old female patient presented with arthritis of both knees. X-rays and MRI showed severe degenerative changes of the internal femoro-tibial compartment and a severe inflammatory thickening of the synovial tissue. Biological work-up identified the presence of anti-citrulline antibodies in the serum of the patient, a marker that is associated with rheumatoid arthritis. Her rheumatologist hesitated between a diagnosis of severe OA versus atypical RA. A synovial biopsy was performed at that time.

RNA was extracted, labeled according to the Affymerix procedure described above and hybridized on a GeneChip® Human genome U133 Plus 2.0 Array. Data were retrieved on GCOS software for the initial normalization and analysis steps. The normalized data from this sample were used for a supervised clustering study on TMEV, together with the 25 reference samples from example 1, using the specific selection of genes listed in Table 2 or 3.

The results of that experiment are shown in Figure 2. The present experiment indicated that the expression of the genes studied in this experiment allowed the synovial biopsies obtained from that patient to cluster with OA samples. The rheumatologist was unaware of these results and treated the patient with RA remission inducing drugs (methotrexate, then a combination of methotrexate and salazopyrine), without any success. The drugs were stopped; the patient was finally diagnosed with OA and underwent successful knee replacement surgery.

### Example 3

A 58-year-old male patient presented with chronic inflammatory knee arthritis. Synovial fluid examination had shown the presence of an inflammatory cell population (> 4,000 elements/mm3); X-ray studies were not contributive. Because of the presence of an atypical rash, his rheumatologist questioned the possibility that the patient suffered from seronegative (psoriatic) arthritis. Synovial biopsies were taken by needle-arthroscopy, RNA was extracted, labeled according to the Affymerix procedure described above and hybridized on a GeneChip® Human genome U133 Plus 2.0 Array. Data were retrieved on GCOS software for the initial normalization and analysis steps. The normalized data from this sample were used for a supervised clustering study on TMEV, together with the 25 reference samples from example 1, using the specific selection of genes listed in Table 2 or 3. The results of the clustering study indicated that his synovial tissue did not cluster with SA samples, but with RA synovial tissue (Figure 3). A dermatologist, who was unaware of these findings, subsequently excluded a diagnostic of cutaneous psoriasis.

### Example 4

A 55-year-old patient with a 4 months history of undifferentiated arthritis affecting both knees and one ankle was submitted to several tests (biological work-up, X-rays, synovial fluid examination) in order to establish a correct diagnosis of his condition. None of these tests provided his rheumatologist with a definite diagnosis; in particular blood tests indicated an elevated uric acid level (10 mg/dl) but were also positive for anti-citrulline antibodies (a specific marker of RA). For that reason, he underwent a needle-arthroscopic procedure allowing harvesting several knee synovial biopsies. RNA is extracted, labeled and hybridized on a diagnostic low-density array that is spotted with a small number of probes such as those listed in Tables 1, 2, 3 or 4 that define a specific gene signature for RA, OA, SLE, MIC or SA. Because of the small number of genes requested, the low-density array according to the invention is cheap and can be routinely used in clinical practice for that purpose. The gene signature found in the synovial biopsies was that of microcrystalline arthritis. The patient was treated with local joint injections, colchicine, NSAID's and allopurinol and went into remission

## Claims

1. Method for the determination and the classification of a rheumatic condition as being either one of systemic lupus erythematosus (SLE), osteoarthritis (OA), rheumatoid arthritis (RA), seronegative arthritis (SA), or microcrystalline arthritis (MIC), in at least one synovial sample of a subject afflicted with said rheumatic condition, comprising the steps of
- providing for said synovial sample the gene expression profile of at least 100 genes or fragments thereof as listed in table 3;
- providing reference profiles by establishing a gene expression profile for reference samples from reference subjects afflicted by osteoarthritis (OA), rheumatoid arthritis (RA), seronegative arthritis (SA), systemic lupus erythematosus (SLE), and microcrystalline arthritis (MIC),
- clustering the subject profile together with reference profiles,
- determining the clustered position of said subject profile among the reference profiles, and
- assigning to said rheumatic condition of said subject the class that corresponds to said clustered position in case said subject profile is within any cluster of reference profiles, or assigning to said rheumatic condition of said subject a new rheumatic condition class.

2. Method according to claim 1 wherein said synovial sample is a synovial tissue.

3. Method according to claim 1 or 2, wherein said synovial sample is a synovial fluid.

4. Method according to claim 3, wherein said synovial sample is cells from the synovial fluid.

5. Method according to any one of claims 1 to 4, wherein the step of determination of the level of expression is performed using DNA-microarray, preferably low-density DNA-spotted microarray.

6. Method according to any one of claims 1 to 5, wherein said clustering step is performed according to hierarchical clustering selected from complete linkage clustering; average linkage clustering and/or single linkage clustering; using at least one of the following metrics selected from Euclidean distance; Manhattan distance; Average dot product; Pearson correlation; Pearson uncentered; Pearson squared; Cosine correlation; Covariance value; Spearman Rank correlation; Kedall's Tau; or Mutual information.

7. Use of a kit comprising a low density microarray comprising probes suitable for hybridizing with at least 100 genes or fragments thereof as listed in table 3, for the determination and the classification of a rheumatic condition as being systemic lupus erythematosus (SLE), osteoarthritis (OA), rheumatoid arthritis (RA), seronegative arthritis (SA), or microcrystalline arthritis (MIC) in at least one synovial sample of a subject afflicted with said rheumatic condition, using the method according to any one of claims 1 to 6.

8. Use according to claim 7, wherein said kit further comprises a computer-readable medium comprising one or more digitally encoded expression profiles of SLE, OA, RA, SA, or MIC reference samples, where each profile has one or more values representing the expression of said genes.

## Patentansprüche

1. Verfahren für die Bestimmung und die Klassifizierung eines rheumatischen Zustandes als entweder systemischer Lupus erythematodes (SLE), Osteoarthritis (OA), rheumatoide Arthritis (RA), seronegative Arthritis (SA) oder mikrokristalline Arthritis (MIC) in mindestens einer Synovialprobe von einem Subjekt, das an dem rheumatischen Zustand leidet, umfassend die folgenden Schritte
- Bereitstellen des Genexpressionsprofils aus mindestens 100 Genen oder Fragmenten davon, wie in Tabelle 3 angeführt, für die Synovialprobe;
- Bereitstellen von Referenzprofilen durch Erstellen eines Genexpressionsprofils für Referenzproben von Referenzsubjekten, die an Osteoarthritis (OA), rheumatoider Arthritis (RA), seronegativer Arthritis (SA), systemischem Lupus erythematodes (SLE) und mikrokristalliner Arthritis (MIC) leiden,
- Cluster-Bildung der Subjektprofile gemeinsam mit Referenzprofilen;
- Bestimmen der Cluster-Position des Subjektprofils unter den Referenzprofilen, und
- Zuordnen der Klasse, die der Cluster-Position entspricht, dem rheumatischen Zustandes des Subjekts, falls sich das Subjektprofil innerhalb eines Clusters von Referenzprofilen befindet, oder Zuordnen einer neuen Klasse eines rheumatischen Zustandes dem rheumatischen Zustandes des Subjekts.

2. Verfahren nach Anspruch 1, wobei die Synovialprobe ein Synovialgewebe ist.

3. Verfahren nach Anspruch 1 oder 2, wobei die Synovialprobe eine Synovialflüssigkeit ist.

4. Verfahren nach Anspruch 3, wobei die Synovialprobe Zellen aus der Synovialflüssigkeit sind.

5. Verfahren nach einem der Ansprüche 1 bis 4, wobei der Schritt zum Bestimmen des Expressionswertes mittels DNA-Mikroarray, vorzugsweise DNA-Spotted Mikroarray geringer Dichte, ausgeführt wird.

6. Verfahren nach einem der Ansprüche 1 bis 5, wobei der Cluster-Bildungsschritt nach einem hierarchischen Clustering ausgeführt wird, ausgewählt aus Complete Linkage Clustering; Average Linkage Clustering und/oder Single Linkage Clustering; wobei mindestens eine der folgenden Metriken verwendet wird, ausgewählt aus Euklidische Distanz; Manhattan Distanz; Average Dot Product; Pearson-Korrelation; unzentrierte Pearson-Korrelation; quadratische Pearson-Korrelation; Kosinus-Korrelation; Kovarianzwert; Spearman-Rangkorrelation; Kendalls Tau; oder wechselseitiger Informationen.

7. Verwendung eines Kits, der ein Mikroarray geringer Dichte umfasst, das Sonden umfasst, die für eine Hybridisierung mit mindestens 100 Genen oder Fragmenten, wie in Tabelle 3 angeführt, geeignet sind, für die Bestimmung und die Klassifizierung eines rheumatischen Zustandes als entweder systemischer Lupus erythematodes (SLE), Osteoarthritis (OA), rheumatoide Arthritis (RA), seronegative Arthritis (SA) oder mikrokristalline Arthritis (MIC) in mindestens einer Synovialprobe von einem Subjekt, das an dem rheumatischen Zustand leidet, unter Verwendung des Verfahrens nach einem der Ansprüche 1 bis 6.

8. Verwendung nach Anspruch 7, wobei der Kit ferner ein computerlesbares Medium umfasst, das ein oder mehrere digital codierte Expressionsprofile von SLE-, OA-, RA-, SA- oder MIC-Referenzproben umfasst, wobei jedes Profil einen oder mehrere Werte hat, die die Expression der Gene darstellen.

## Revendications

1. Procédé de détermination et de classification d'une condition rhumatismale comme étant l'une du lupus érythémateux systémique (LES), de l'ostéo-arthrite (OA), de l'arthrite rhumatoïde (AR), de l'arthrite séronégative (AS), ou de l'arthrite microcristalline (MIC), dans au moins un échantillon synovial d'un sujet atteint de ladite condition rhumatismale, qui comprend les étapes consistant à :
- fournir le profil d'expression génique d'au moins 100 gènes ou fragments de ceux-ci tels que listés dans le tableau 3 pour ledit échantillon synovial ;
- fournir des profils de référence par établissement d'un profil d'expression génique pour des échantillons de référence de sujets de référence atteints d'ostéo-arthrite (OA), d'arthrite rhumatoïde (AR), d'arthrite séronégative (AS), de lupus érythémateux systémique (LES), et d'arthrite microcristalline (MIC),
- grouper le profil du sujet et les profils de référence,
- déterminer la position dans les groupes dudit profil du sujet parmi les profils de référence, et
- assigner à ladite condition rhumatismale dudit sujet la classe qui correspond à ladite position dans les groupes dans le cas où ledit profil du sujet se trouve dans un groupe de profils de référence quelconque, ou assigner à ladite condition rhumatismale dudit sujet une nouvelle classe de condition rhumatismale.

2. Procédé selon la revendication 1, dans lequel ledit échantillon synovial est un tissu synovial.

3. Procédé selon la revendication 1 ou 2, dans lequel ledit échantillon synovial est un fluide synovial.

4. Procédé selon la revendication 3, dans lequel ledit échantillon synovial est constitué de cellules du fluide synovial.

5. Procédé selon l'une quelconque des revendications 1 à 4, dans lequel l'étape de détermination du niveau d'expression est réalisée en utilisant une puce à ADN, de préférence une puce à dépôt d'ADN de faible densité.

6. Procédé selon l'une quelconque des revendications 1 à 5, dans lequel ladite étape de groupement est réalisée selon un groupement hiérarchique choisi parmi le groupement à lien complet, le groupement à liaison moyenne et/ou le groupement à lien simple ; en utilisant au moins l'une des mesures suivantes choisies parmi la distance euclidienne ; la distance de Manhattan ; le produit scalaire moyen ; la corrélation de Pearson ; la corrélation de Pearson non centrée ; la corrélation carrée de Pearson ; la corrélation cosinus ; la valeur de covariance ; la corrélation des rangs de Spearman ; le tau de Kedall ; ou les informations mutuelles.

7. Utilisation d'un kit comprenant une puce de faible densité comprenant des sondes adaptées à l'hybridation avec au moins 100 gènes ou fragments de ceux-ci tels que listés dans le tableau 3, pour la détermination et la classification d'une condition rhumatismale comme étant le lupus érythémateux systémique (LES), l'ostéo-arthrite (OA), l'arthrite rhumatoïde (AR), l'arthrite séronégative (AS), ou l'arthrite microcristalline (MIC) dans au moins un échantillon synovial d'un sujet atteint de ladite condition rhumatismale, en utilisant le procédé selon l'une quelconque des revendications 1 à 6.

8. Utilisation selon la revendication 7, dans laquelle ledit kit comprend en outre un support lisible par ordinateur comprenant un ou plusieurs profils d'expression codés numériquement d'échantillons de référence de LES, d'OA, de AR, d'AS ou de MIC, chaque profil ayant une ou plusieurs valeurs représentant l'expression desdits gènes.
